# EUROPEAN PATENT APPLICATION

(11) **EP 2 042 186 A1**
(43) Date of publication of application: **01.04.2009**
(21) Application number: 09000056.3
(22) Date of filing: 30.01.2006
(51) Int. Cl.: A61K 38/03, C07K 4/00, C07K 19/00, A61K 39/00

(54) **Novel polypeptide ligands for toll-like receptor 2 (TLR2)**

(30) Priority: 31.01.2005 US 648923 P
(62) Divisional of application: 06734080.2
(71) Applicant: Vaxinnate Corporation, Cranbury NJ 08512 (US)
(72) Inventor: Nakaar, Valerian, Hamden Connecticut 06514 (US); Huang, Yan, North Branford Connecticut 06471 (US); Powel, Thomas, J., Madison Connecticut 06443 (US)
(74) Representative: Tuxworth, Pamela M.

(57) **Abstract**

The present invention provides novel polypeptide ligands for Toll-like Receptor 2 (TLR2). Preferably, the novel polypeptide ligands modulate TLR2 signaling and thereby regulate the Innate Immune Response. The invention also provides vaccines comprising the novel polypeptide TLR2 ligands and an antigen. The invention further provides methods of modulating TLR2 signaling using the polypeptide ligands or vaccines of the invention.

SEQUENCE LISTING

<110> Vaxinnate Corporation
      Nakaar, Valerian
      Huang, Yan
      Powell, Thomas J.

<120> NOVEL POLYPETPTIDE LIGANDS FOR TOLL-LIKE RECEPTOR 2 (TLR2)          (TLR2)

<130> 04348/200N204-WO0

<150> 60/648,923
<151> 2005-01-31

<160> 81

<170> PatentIn version 3.3

<210> 1
<211> 2838
<212> DNA
<213> Mus musculus

<400> 1
ggcacgagag atggctagtg ggcacgggga gcggcggctg gaggactcct aggctccggg      60

caggcggtca ctggcaggag atgtgtccgc aatcatagtt tctgatggtg aaggttggac     120

ggcagtctct gcgacctaga agtggaaaag atgtcgttca aggaggtgcg gactgtttcc     180

ttctgaccag gatcttgttt ctgagtgtag gggcttcact tctctgcttt tcgttcatct     240

ctggagcatc cgaattgcat caccggtcag aaaacaactt accgaaacct cagacaaagc     300

gtcaaatctc agaggatgct acgagctctt tggctcttct ggatcttggt ggccataaca     360

gtcctcttca gcaaacgctg ttctgctcag gagtctctgt catgtgatgc ttctggggtg     420

tgtgatggcc gctccaggtc tttcacctct attccctccg gactcacagc agccatgaaa     480

agccttgacc tgtctttcaa caagatcacc tacattggcc atggtgacct ccgagcgtgt     540

gcgaacctcc aggttctgat tttgaagtcc agcagaatca atacaataga gggagacgcc     600

ttttattctc tgggcagtct tgaacatttg gatttgtctg ataatcacct atctagttta     660

tcttcctcct ggttcgggcc cctttcctct ttgaaatact taaacttaat gggaaatcct     720

taccagacac tgggggtaac atcgcttttt cccaatctca caaatttaca aaccctcagg     780

ataggaaatg tagagacttt cagtgagata aggagaatag attttgctgg gctgacttct     840

ctcaatgaac ttgaaattaa ggcattaagt ctccggaatt atcagtccca aagtctaaag     900

tcgatccgcg acatccatca cctgactctt cacttaagcg agtctgcttt cctgctggag     960

atttttgcag atattctgag ttctgtgaga tatttagaac taagagatac taacttggcc    1020

aggttccagt tttcaccact gcccgtagat gaagtcagct caccgatgaa gaagctggca    1080

ttccgaggct cggttctcac tgatgaaagc tttaacgagc tcctgaagct gttgcgttac    1140

atcttggaac tgtcggaggt agagttcgac gactgtaccc tcaatgggct cggcgatttc    1200

aacccctcgg agtcagacgt agtgagcgag ctgggtaaag tagaaacagt cactatccgg 1260
aggttgcata tcccccagtt ctatttgttt tatgacctga gtactgtcta ttccctcctg 1320
gagaaggtga agcgaatcac agtagagaac agcaaggtct tcctggttcc ctgctcgttc 1380
tcccagcatt taaaatcatt agaattctta gacctcagcg aaaatctgat ggttgaagaa 1440
tatttgaaga actcagcctg taagggagcc tggccttctc tacaaacctt agttttgagc 1500
cagaatcatt tgagatcaat gcaaaaaaca ggagagattt tgctgactct gaaaaacctg 1560
acctcccttg acatcagcag gaacactttt catccgatgc ccgacagctg tcagtggcca 1620
gaaaagatgc gcttcctgaa tttgtccagt acagggatcc gggtggtaaa aacgtgcatt 1680
cctcagacgc tggaggtgtt ggatgttagt aacaacaatc ttgactcatt ttctttgttc 1740
ttgcctcggc tgcaagagct ctatatttcc agaaataagc tgaaaacact cccagatgct 1800
tcgttgttcc ctgtgttgct ggtcatgaaa atcagagaga atgcagtaag tactttctct 1860
aaagaccaac ttggttcttt tcccaaactg gagactctgg aagcaggcga caaccacttt 1920
gtttgctcct gcgaactcct atcctttact atggagacgc cagctctggc tcaaatcctg 1980
gttgactggc cagacagcta cctgtgtgac tctccgcctc gcctgcacgg ccacaggctt 2040
caggatgccc ggccctccgt cttggaatgt caccaggctg cactggtgtc tggagtctgc 2100
tgtgcccttc tcctgttgat cttgctcgta ggtgccctgt gccaccattt ccacggactg 2160
tggtacctga gaatgatgtg ggcgtggctc caggccaaga ggaagcccaa gaaagctccc 2220
tgcagggacg tttgctatga tgcctttgtt tcctacagtg agcaggattc ccattgggtg 2280
gagaacctca tggtccagca gctggagaac tctgacccgc cctttaagct gtgtctccac 2340
aagcgggact tcgttccggg caaatggatc attgacaaca tcatcgattc catcgaaaag 2400
agccacaaaa ctgtgttcgt gctttctgag aacttcgtac ggagcgagtg gtgcaagtac 2460
gaactggact tctcccactt caggctcttt gacgagaaca acgacgcggc catccttgtt 2520
ttgctggagc ccattgagag gaaagccatt ccccagcgct tctgcaaact gcgcaagata 2580
atgaacacca agacctacct ggagtggccc ttggatgaag gccagcagga agtgttttgg 2640
gtaaatctga gaactgcaat aaagtcctag gttctccacc cagttcctga cttccttaac 2700
taaggtcttt gtgacacaaa ctgtaacaaa gtttataagt aacatagaat tgtattattg 2760
aggatattaa ctatgggttt tgtcttgaat actgttatat aaatatgtga catcaggaaa 2820
aaaaaaaaaa aaaaaaaa 2838

<210> 2
<211> 784
<212> PRT
<213> Mus musculus

<400> 2

Met Leu Arg Ala Leu Trp Leu Phe Trp Ile Leu Val Ala Ile Thr Val
1               5                   10                  15

Leu Phe Ser Lys Arg Cys Ser Ala Gln Glu Ser Leu Ser Cys Asp Ala
            20                  25                  30

Ser Gly Val Cys Asp Gly Arg Ser Arg Ser Phe Thr Ser Ile Pro Ser
        35                  40                  45

Gly Leu Thr Ala Ala Met Lys Ser Leu Asp Leu Ser Phe Asn Lys Ile
    50                  55                  60

Thr Tyr Ile Gly His Gly Asp Leu Arg Ala Cys Ala Asn Leu Gln Val
65                  70                  75                  80

Leu Ile Leu Lys Ser Ser Arg Ile Asn Thr Ile Glu Gly Asp Ala Phe
                85                  90                  95

Tyr Ser Leu Gly Ser Leu Glu His Leu Asp Leu Ser Asp Asn His Leu
            100                 105                 110

Ser Ser Leu Ser Ser Ser Trp Phe Gly Pro Leu Ser Ser Leu Lys Tyr
        115                 120                 125

Leu Asn Leu Met Gly Asn Pro Tyr Gln Thr Leu Gly Val Thr Ser Leu
    130                 135                 140

Phe Pro Asn Leu Thr Asn Leu Gln Thr Leu Arg Ile Gly Asn Val Glu
145                 150                 155                 160

Thr Phe Ser Glu Ile Arg Arg Ile Asp Phe Ala Gly Leu Thr Ser Leu
                165                 170                 175

Asn Glu Leu Glu Ile Lys Ala Leu Ser Leu Arg Asn Tyr Gln Ser Gln
            180                 185                 190

Ser Leu Lys Ser Ile Arg Asp Ile His His Leu Thr Leu His Leu Ser
        195                 200                 205

Glu Ser Ala Phe Leu Leu Glu Ile Phe Ala Asp Ile Leu Ser Ser Val
    210                 215                 220

Arg Tyr Leu Glu Leu Arg Asp Thr Asn Leu Ala Arg Phe Gln Phe Ser
225                 230                 235                 240

Pro Leu Pro Val Asp Glu Val Ser Ser Pro Met Lys Lys Leu Ala Phe

                245                 250                 255

Arg Gly Ser Val Leu Thr Asp Glu Ser Phe Asn Glu Leu Leu Lys Leu
            260                 265                 270

Leu Arg Tyr Ile Leu Glu Leu Ser Glu Val Glu Phe Asp Asp Cys Thr
        275                 280                 285

Leu Asn Gly Leu Gly Asp Phe Asn Pro Ser Glu Ser Asp Val Val Ser
    290                 295                 300

Glu Leu Gly Lys Val Glu Thr Val Thr Ile Arg Arg Leu His Ile Pro
305                 310                 315                 320

Gln Phe Tyr Leu Phe Tyr Asp Leu Ser Thr Val Tyr Ser Leu Leu Glu
                325                 330                 335

Lys Val Lys Arg Ile Thr Val Glu Asn Ser Lys Val Phe Leu Val Pro
            340                 345                 350

Cys Ser Phe Ser Gln His Leu Lys Ser Leu Glu Phe Leu Asp Leu Ser
        355                 360                 365

Glu Asn Leu Met Val Glu Glu Tyr Leu Lys Asn Ser Ala Cys Lys Gly
    370                 375                 380

Ala Trp Pro Ser Leu Gln Thr Leu Val Leu Ser Gln Asn His Leu Arg
385                 390                 395                 400

Ser Met Gln Lys Thr Gly Glu Ile Leu Leu Thr Leu Lys Asn Leu Thr
                405                 410                 415

Ser Leu Asp Ile Ser Arg Asn Thr Phe His Pro Met Pro Asp Ser Cys
            420                 425                 430

Gln Trp Pro Glu Lys Met Arg Phe Leu Asn Leu Ser Ser Thr Gly Ile
        435                 440                 445

Arg Val Val Lys Thr Cys Ile Pro Gln Thr Leu Glu Val Leu Asp Val
    450                 455                 460

Ser Asn Asn Asn Leu Asp Ser Phe Ser Leu Phe Leu Pro Arg Leu Gln
465                 470                 475                 480

Glu Leu Tyr Ile Ser Arg Asn Lys Leu Lys Thr Leu Pro Asp Ala Ser
                485                 490                 495

Leu Phe Pro Val Leu Leu Val Met Lys Ile Arg Glu Asn Ala Val Ser
            500                 505                 510

Thr Phe Ser Lys Asp Gln Leu Gly Ser Phe Pro Lys Leu Glu Thr Leu
        515                 520                 525

Glu Ala Gly Asp Asn His Phe Val Cys Ser Cys Glu Leu Leu Ser Phe
    530                 535                 540

Thr Met Glu Thr Pro Ala Leu Ala Gln Ile Leu Val Asp Trp Pro Asp
545                 550                 555                 560

Ser Tyr Leu Cys Asp Ser Pro Pro Arg Leu His Gly His Arg Leu Gln
                565                 570                 575

Asp Ala Arg Pro Ser Val Leu Glu Cys His Gln Ala Ala Leu Val Ser
            580                 585                 590

Gly Val Cys Cys Ala Leu Leu Leu Leu Ile Leu Leu Val Gly Ala Leu
        595                 600                 605

Cys His His Phe His Gly Leu Trp Tyr Leu Arg Met Met Trp Ala Trp
    610                 615                 620

Leu Gln Ala Lys Arg Lys Pro Lys Lys Ala Pro Cys Arg Asp Val Cys
625                 630                 635                 640

Tyr Asp Ala Phe Val Ser Tyr Ser Glu Gln Asp Ser His Trp Val Glu
                645                 650                 655

Asn Leu Met Val Gln Gln Leu Glu Asn Ser Asp Pro Pro Phe Lys Leu
            660                 665                 670

Cys Leu His Lys Arg Asp Phe Val Pro Gly Lys Trp Ile Ile Asp Asn
        675                 680                 685

Ile Ile Asp Ser Ile Glu Lys Ser His Lys Thr Val Phe Val Leu Ser
    690                 695                 700

Glu Asn Phe Val Arg Ser Glu Trp Cys Lys Tyr Glu Leu Asp Phe Ser
705                 710                 715                 720

His Phe Arg Leu Phe Asp Glu Asn Asn Asp Ala Ala Ile Leu Val Leu
                725                 730                 735

Leu Glu Pro Ile Glu Arg Lys Ala Ile Pro Gln Arg Phe Cys Lys Leu
            740                 745                 750

Arg Lys Ile Met Asn Thr Lys Thr Tyr Leu Glu Trp Pro Leu Asp Glu
        755                 760                 765

Gly Gln Gln Glu Val Phe Trp Val Asn Leu Arg Thr Ala Ile Lys Ser
    770                 775                 780

<210>  3
<211>  2756
<212>  DNA
<213>  Homo sapiens

<400>  3
gagaaagcgc agccaggcgg ctgctcggcg ttctctcagg tgactgctcg gagttctccc      60

agtgtttggt gttgcaagca ggatccaaag gagacctata gtgactccca ggagctctta     120

gtgaccaagt gaaggttgaa gcactggaca atgccacata ctttgtggat ggtgtgggtc     180

ttgggggtca tcatcagcct ctccaaggaa gaatcctcca atcaggcttc tctgtcttgt     240

gaccgcaatg gtatctgcaa gggcagctca ggatctttaa actccattcc ctcagggctc     300

acagaagctg taaaaagcct tgacctgtcc aacaacagga tcacctacat tagcaacagt     360

gacctacaga ggtgtgtgaa cctccaggct ctggtgctga catccaatgg aattaacaca     420

atagaggaag attctttttc ttccctgggc agtcttgaac atttagactt atcctataat     480

tacttatcta atttatcgtc ttcctggttc aagccccttt cttctttaac attcttaaac     540

ttactgggaa atccttacaa aaccctaggg gaaacatctc ttttttctca tctcacaaaa     600

ttgcaaatcc tgagagtggg aaatatggac accttcacta agattcaaag aaaagatttt     660

gctggactta ccttccttga ggaacttgag attgatgctt cagatctaca gagctatgag     720

ccaaaaagtt tgaagtcaat tcagaacgta agtcatctga tccttcatat gaagcagcat     780

attttactgc tggagatttt tgtagatgtt acaagttccg tggaatgttt ggaactgcga     840

gatactgatt tggacacttt ccatttttca gaactatcca ctggtgaaac aaattcattg     900

attaaaaagt ttacatttag aaatgtgaaa atcaccgatg aaagtttgtt tcaggttatg     960

aaacttttga atcagatttc tggattgtta gaattagagt ttgatgactg tacccttaat    1020

ggagttggta attttagagc atctgataat gacagagtta tagatccagg taaagtggaa    1080

acgttaacaa tccggaggct gcatattcca aggttttact tattttatga tctgagcact    1140

ttatattcac ttacagaaag agttaaaaga atcacagtag aaaacagtaa agtttttctg    1200

gttccttgtt tactttcaca acatttaaaa tcattagaat acttggatct cagtgaaaat    1260

ttgatggttg aagaatactt gaaaaattca gcctgtgagg atgcctggcc ctctctacaa    1320

actttaattt taaggcaaaa tcatttggca tcattggaaa aaaccggaga gactttgctc    1380

actctgaaaa acttgactaa cattgatatc agtaagaata gttttcattc tatgcctgaa    1440

acttgtcagt ggccagaaaa gatgaaatat ttgaacttat ccagcacacg aatacacagt 1500
gtaacaggct gcattcccaa gacactggaa attttagatg ttagcaacaa caatctcaat 1560
ttattttctt tgaatttgcc gcaactcaaa gaactttata tttccagaaa taagttgatg 1620
actctaccag atgcctccct cttacccatg ttactagtat tgaaaatcag taggaatgca 1680
ataactacgt tttctaagga gcaacttgac tcatttcaca cactgaagac tttggaagct 1740
ggtggcaata acttcatttg ctcctgtgaa ttcctctcct tcactcagga gcagcaagca 1800
ctggccaaag tcttgattga ttggccagca aattacctgt gtgactctcc atcccatgtg 1860
cgtggccagc aggttcagga tgtccgcctc tcggtgtcgg aatgtcacag gacagcactg 1920
gtgtctggca tgtgctgtgc tctgttcctg ctgatcctgc tcacgggggt cctgtgccac 1980
cgtttccatg gcctgtggta tatgaaaatg atgtgggcct ggctccaggc caaaaggaag 2040
cccaggaaag ctcccagcag gaacatctgc tatgatgcat ttgtttctta cagtgagcgg 2100
gatgcctact gggtggagaa ccttatggtc caggagctgg agaacttcaa tccccccttc 2160
aagttgtgtc ttcataagcg ggacttcatt cctggcaagt ggatcattga caatatcatt 2220
gactccattg aaaagagcca caaaactgtc tttgtgcttt ctgaaaactt tgtgaagagt 2280
gagtggtgca agtatgaact ggacttctcc catttccgtc tttttgatga gaacaatgat 2340
gctgccattc tcattcttct ggagcccatt gagaaaaaag ccattcccca gcgcttctgc 2400
aagctgcgga agataatgaa caccaagacc tacctggagt ggcccatgga cgaggctcag 2460
cgggaaggat tttgggtaaa tctgagagct gcgataaagt cctaggttcc catatttaag 2520
accagtcttt gtctagttgg gatctttatg tcactagtta tagttaagtt cattcagaca 2580
taattatata aaaactacgt ggatgtaccg tcatttgagg acttgcttac taaaactaca 2640
aaacttcaaa ttttgtctgg ggtgctgttt tataaacata tgccagattt aaaaaaaaaa 2700
aaaaaaaaaa aaaaaaaaaa aaaaaaaaaa aaaaaaaaaa aaaaaaaaaa aaaaaa 2756

<210> 4
<211> 784
<212> PRT
<213> Homo sapiens

<400> 4

Met Pro His Thr Leu Trp Met Val Trp Val Leu Gly Val Ile Ile Ser
1               5                   10                  15

Leu Ser Lys Glu Glu Ser Ser Asn Gln Ala Ser Leu Ser Cys Asp Arg
            20                  25                  30

Asn Gly Ile Cys Lys Gly Ser Ser Gly Ser Leu Asn Ser Ile Pro Ser
        35                  40                  45

Gly Leu Thr Glu Ala Val Lys Ser Leu Asp Leu Ser Asn Asn Arg Ile
    50                  55                  60

Thr Tyr Ile Ser Asn Ser Asp Leu Gln Arg Cys Val Asn Leu Gln Ala
65                  70                  75                  80

Leu Val Leu Thr Ser Asn Gly Ile Asn Thr Ile Glu Glu Asp Ser Phe
                85                  90                  95

Ser Ser Leu Gly Ser Leu Glu His Leu Asp Leu Ser Tyr Asn Tyr Leu
            100                 105                 110

Ser Asn Leu Ser Ser Ser Trp Phe Lys Pro Leu Ser Ser Leu Thr Phe
        115                 120                 125

Leu Asn Leu Leu Gly Asn Pro Tyr Lys Thr Leu Gly Glu Thr Ser Leu
    130                 135                 140

Phe Ser His Leu Thr Lys Leu Gln Ile Leu Arg Val Gly Asn Met Asp
145                 150                 155                 160

Thr Phe Thr Lys Ile Gln Arg Lys Asp Phe Ala Gly Leu Thr Phe Leu
                165                 170                 175

Glu Glu Leu Glu Ile Asp Ala Ser Asp Leu Gln Ser Tyr Glu Pro Lys
            180                 185                 190

Ser Leu Lys Ser Ile Gln Asn Val Ser His Leu Ile Leu His Met Lys
        195                 200                 205

Gln His Ile Leu Leu Leu Glu Ile Phe Val Asp Val Thr Ser Ser Val
    210                 215                 220

Glu Cys Leu Glu Leu Arg Asp Thr Asp Leu Asp Thr Phe His Phe Ser
225                 230                 235                 240

Glu Leu Ser Thr Gly Glu Thr Asn Ser Leu Ile Lys Lys Phe Thr Phe
                245                 250                 255

Arg Asn Val Lys Ile Thr Asp Glu Ser Leu Phe Gln Val Met Lys Leu
            260                 265                 270

Leu Asn Gln Ile Ser Gly Leu Leu Glu Leu Glu Phe Asp Asp Cys Thr
        275                 280                 285

Leu Asn Gly Val Gly Asn Phe Arg Ala Ser Asp Asn Asp Arg Val Ile
    290                 295                 300

Asp Pro Gly Lys Val Glu Thr Leu Thr Ile Arg Arg Leu His Ile Pro
305                 310                 315                 320

Arg Phe Tyr Leu Phe Tyr Asp Leu Ser Thr Leu Tyr Ser Leu Thr Glu
                325                 330                 335

Arg Val Lys Arg Ile Thr Val Glu Asn Ser Lys Val Phe Leu Val Pro
            340                 345                 350

Cys Leu Leu Ser Gln His Leu Lys Ser Leu Glu Tyr Leu Asp Leu Ser
        355                 360                 365

Glu Asn Leu Met Val Glu Glu Tyr Leu Lys Asn Ser Ala Cys Glu Asp
    370                 375                 380

Ala Trp Pro Ser Leu Gln Thr Leu Ile Leu Arg Gln Asn His Leu Ala
385                 390                 395                 400

Ser Leu Glu Lys Thr Gly Glu Thr Leu Leu Thr Leu Lys Asn Leu Thr
                405                 410                 415

Asn Ile Asp Ile Ser Lys Asn Ser Phe His Ser Met Pro Glu Thr Cys
            420                 425                 430

Gln Trp Pro Glu Lys Met Lys Tyr Leu Asn Leu Ser Ser Thr Arg Ile
        435                 440                 445

His Ser Val Thr Gly Cys Ile Pro Lys Thr Leu Glu Ile Leu Asp Val
    450                 455                 460

Ser Asn Asn Asn Leu Asn Leu Phe Ser Leu Asn Leu Pro Gln Leu Lys
465                 470                 475                 480

Glu Leu Tyr Ile Ser Arg Asn Lys Leu Met Thr Leu Pro Asp Ala Ser
                485                 490                 495

Leu Leu Pro Met Leu Leu Val Leu Lys Ile Ser Arg Asn Ala Ile Thr
            500                 505                 510

Thr Phe Ser Lys Glu Gln Leu Asp Ser Phe His Thr Leu Lys Thr Leu
        515                 520                 525

Glu Ala Gly Gly Asn Asn Phe Ile Cys Ser Cys Glu Phe Leu Ser Phe
    530                 535                 540

Thr Gln Glu Gln Gln Ala Leu Ala Lys Val Leu Ile Asp Trp Pro Ala

545                 550                 555                 560

Asn Tyr Leu Cys Asp Ser Pro Ser His Val Arg Gly Gln Gln Val Gln
                565                 570                 575

Asp Val Arg Leu Ser Val Ser Glu Cys His Arg Thr Ala Leu Val Ser
            580                 585                 590

Gly Met Cys Cys Ala Leu Phe Leu Leu Ile Leu Leu Thr Gly Val Leu
        595                 600                 605

Cys His Arg Phe His Gly Leu Trp Tyr Met Lys Met Met Trp Ala Trp
    610                 615                 620

Leu Gln Ala Lys Arg Lys Pro Arg Lys Ala Pro Ser Arg Asn Ile Cys
625                 630                 635                 640

Tyr Asp Ala Phe Val Ser Tyr Ser Glu Arg Asp Ala Tyr Trp Val Glu
                645                 650                 655

Asn Leu Met Val Gln Glu Leu Glu Asn Phe Asn Pro Pro Phe Lys Leu
            660                 665                 670

Cys Leu His Lys Arg Asp Phe Ile Pro Gly Lys Trp Ile Ile Asp Asn
        675                 680                 685

Ile Ile Asp Ser Ile Glu Lys Ser His Lys Thr Val Phe Val Leu Ser
    690                 695                 700

Glu Asn Phe Val Lys Ser Glu Trp Cys Lys Tyr Glu Leu Asp Phe Ser
705                 710                 715                 720

His Phe Arg Leu Phe Asp Glu Asn Asn Asp Ala Ala Ile Leu Ile Leu
                725                 730                 735

Leu Glu Pro Ile Glu Lys Lys Ala Ile Pro Gln Arg Phe Cys Lys Leu
            740                 745                 750

Arg Lys Ile Met Asn Thr Lys Thr Tyr Leu Glu Trp Pro Met Asp Glu
        755                 760                 765

Ala Gln Arg Glu Gly Phe Trp Val Asn Leu Arg Ala Ala Ile Lys Ser
    770                 775                 780

<210>  5
<211>  5
<212>  PRT
<213>  artificial

<220>
<223>  synthetic peptide

<400>  5

Asp Pro Asp Ser Gly
1               5


<210>  6
<211>  5
<212>  PRT
<213>  artificial

<220>
<223>  synthetic peptide

<400>  6

Ile Gly Arg Phe Arg
1               5


<210>  7
<211>  5
<212>  PRT
<213>  artificial

<220>
<223>  synthetic peptide

<400>  7

Met Gly Thr Leu Pro
1               5


<210>  8
<211>  5
<212>  PRT
<213>  artificial

<220>
<223>  synthetic peptide

<400>  8

Ala Asp Thr His Gln
1               5


<210>  9
<211>  5
<212>  PRT
<213>  artificial

<220>
<223>  synthetic peptide

<400>  9

His Leu Leu Pro Gly

1                   5

<210>  10
<211>  5
<212>  PRT
<213>  artificial

<220>
<223>  synthetic peptide

<400>  10

Gly Pro Leu Leu His
1               5

<210>  11
<211>  5
<212>  PRT
<213>  artificial

<220>
<223>  synthetic peptide

<400>  11

Asn Tyr Arg Arg Trp
1               5

<210>  12
<211>  5
<212>  PRT
<213>  artificial

<220>
<223>  synthetic peptide

<400>  12

Leu Arg Gln Gly Arg
1               5

<210>  13
<211>  5
<212>  PRT
<213>  artificial

<220>
<223>  synthetic peptide

<400>  13

Ile Met Trp Phe Pro
1               5

<210>  14
<211>  5
<212>  PRT
<213>  artificial

<220>
<223>  synthetic peptide

<400>  14

Arg Val Val Ala Pro
1               5


<210>  15
<211>  5
<212>  PRT
<213>  artificial

<220>
<223>  synthetic peptide

<400>  15

Ile His Val Val Pro
1               5


<210>  16
<211>  5
<212>  PRT
<213>  artificial

<220>
<223>  synthetic peptide

<400>  16

Met Phe Gly Val Pro
1               5


<210>  17
<211>  5
<212>  PRT
<213>  artificial

<220>
<223>  synthetic peptide

<400>  17

Cys Val Trp Leu Gln
1               5


<210>  18
<211>  5
<212>  PRT
<213>  artificial

<220>
<223>  synthetic peptide

<400>  18

Ile Tyr Lys Leu Ala

1                    5

<210>  19
<211>  4
<212>  PRT
<213>  artificial

<220>
<223>  synthetic peptide

<400>  19

Lys Gly Trp Phe
1

<210>  20
<211>  5
<212>  PRT
<213>  artificial

<220>
<223>  synthetic peptide

<400>  20

Lys Tyr Met Pro His
1               5

<210>  21
<211>  5
<212>  PRT
<213>  artificial

<220>
<223>  synthetic peptide

<400>  21

Val Gly Lys Asn Asp
1               5

<210>  22
<211>  5
<212>  PRT
<213>  artificial

<220>
<223>  synthetic peptide

<400>  22

Thr His Lys Pro Lys
1               5

<210>  23
<211>  5
<212>  PRT
<213>  artificial

<220>
<223>  synthetic peptide

<400>  23

Ser His Ile Ala Leu
1               5


<210>  24
<211>  5
<212>  PRT
<213>  artificial

<220>
<223>  synthetic peptide

<400>  24

Ala Trp Ala Gly Thr
1               5


<210>  25
<211>  20
<212>  PRT
<213>  artificial

<220>
<223>  synthetic peptide

<400>  25

Lys Gly Gly Val Gly Pro Val Arg Arg Ser Ser Arg Leu Arg Arg Thr
1               5                   10                  15


Thr Gln Pro Gly
            20


<210>  26
<211>  23
<212>  PRT
<213>  artificial

<220>
<223>  synthetic peptide

<400>  26

Gly Arg Arg Gly Leu Cys Arg Gly Cys Arg Thr Arg Gly Arg Ile Lys
1               5                   10                  15


Gln Leu Gln Ser Ala His Lys
            20


<210>  27
<211>  22
<212>  PRT

<213>  artificial

<220>
<223>  synthetic peptide

<400>  27

Arg Trp Gly Tyr His Leu Arg Asp Arg Lys Tyr Lys Gly Val Arg Ser
1               5                   10                  15

His Lys Gly Val Pro Arg
            20

<210>  28
<211>  22
<212>  DNA
<213>  artificial

<220>
<223>  universal oligonucleotide

<400>  28
gtctcgaaag cttttatcct cc                                              22

<210>  29
<211>  1854
<212>  DNA
<213>  Measles virus

<400>  29
atgtcaccac aacgagaccg aataaatgcc ttctacaaag acaaccccca tcctaaggga      60

agtaggatag ttattaacag agaacatctt atgattgata gaccttatgt tttgctggct     120

gttctattcg tcatgtttct gagcttgatc gggttgctag ccattgcagg cattagactt     180

catcgggcag ccatctacac tgcagagatc cataaaagcc tcagcaccaa tctagatgta     240

actaactcaa tcgagcatca ggtcaaggac gtgctgacac cactcttcaa gatcatcggt     300

gatgaagtgg gcctgaggac acctcagaga ttcactgacc tagtgaaatt catctctgac     360

aagattaaat tccttaatcc ggatagggag tacgacttca gggatctcac ttggtgtatc     420

aacccgccag agagaatcaa attggattat gatcaatact gtgcagatgt ggctgctgaa     480

gaactcatga atgcattggt gaacgcaact ctactggagg ccagggcaac caatcagttc     540

ctagctgtct caaagggaaa ctgctcaggg cccactacaa tcagaggtca attctcaaac     600

atgtcgctgt ccctgttgga cttgtattta agtcgaggtt acaatgtgtc atctatagtc     660

actatgacat cccagggaat gtacggggga acttacctag tggaaaagcc taatctgagc     720

agtaaagggt cagagttgtc acaactgagc atgcaccgag tgtttgaagt aggtgtgatc     780

agaaatccgg gtttgggggc tccggtgttc catatgacaa actattttga gcaatcagtc     840

agtaatgatt tcagcaactg catggtggct ttaggggagc tcaaattcgc agccctctgt     900

cacagggaag attctatcac aattccctat caggggtcag ggaaaggtgt cagcttccag 960
ctcgtcaagc taggtgtctg gaaatcccca accgacatgc aatcctgggt ccccctatca 1020
acggatgatc cagtgataga taggctttac ctctcatctc acagaggtgt tatcgctgac 1080
aatcaagcaa aatgggctgt cccgacaaca cggacagatg acaagttgcg aatggagaca 1140
tgcttccagc aggcgtgtaa gggcaaaatc caagcactct gcgagaatcc cgagtgggca 1200
ccactgaagg acaacaggat tccttcatac ggggtcttgt ctgttaatct gagtctgaca 1260
gttgagctca aaatcaaaat tgcttcagga ttcgggccat tgatcacaca cggttcaggg 1320
atggacctat acaaatccaa ccacaacaat gtgtattggc tgaccatccc gccaatgaag 1380
aacctagcct taggtgtaat caacacatta gagtggatac cgagattcaa ggttagtccc 1440
aacctcttca ctgttccaat caaggaagca ggcgaggact gccatgcccc aacataccta 1500
cctgcggagg tggatggtga tgtcaaactc agttccaatc tggtgattct acctggtcaa 1560
gatctccaat atgttttggc aacctacgat acttccaggg ttgaacatgc tgtggtttat 1620
tatgtttaca gcccgggccg ctcattttct tacttttatc cttttaggtt gcctataaag 1680
ggggtcccca tcgaattaca agtggaatgc ttcacatggg accaaaaact ctggtgccgt 1740
cacttctgtg tgcttgcgga ctcagaatct ggtggacata tcactcactc tgggatggtg 1800
ggcatgggag tcagctgcac agtcactcgg gaagatggaa ccaataccag atag 1854

<210> 30
<211> 617
<212> PRT
<213> Measles virus

<400> 30

Met Ser Pro Gln Arg Asp Arg Ile Asn Ala Phe Tyr Lys Asp Asn Pro
1               5                   10                  15

His Pro Lys Gly Ser Arg Ile Val Ile Asn Arg Glu His Leu Met Ile
            20                  25                  30

Asp Arg Pro Tyr Val Leu Leu Ala Val Leu Phe Val Met Phe Leu Ser
        35                  40                  45

Leu Ile Gly Leu Leu Ala Ile Ala Gly Ile Arg Leu His Arg Ala Ala
    50                  55                  60

Ile Tyr Thr Ala Glu Ile His Lys Ser Leu Ser Thr Asn Leu Asp Val
65                  70                  75                  80

Thr Asn Ser Ile Glu His Gln Val Lys Asp Val Leu Thr Pro Leu Phe
                85                  90                  95

Lys Ile Ile Gly Asp Glu Val Gly Leu Arg Thr Pro Gln Arg Phe Thr
            100                 105                 110

Asp Leu Val Lys Phe Ile Ser Asp Lys Ile Lys Phe Leu Asn Pro Asp
        115                 120                 125

Arg Glu Tyr Asp Phe Arg Asp Leu Thr Trp Cys Ile Asn Pro Pro Glu
    130                 135                 140

Arg Ile Lys Leu Asp Tyr Asp Gln Tyr Cys Ala Asp Val Ala Ala Glu
145                 150                 155                 160

Glu Leu Met Asn Ala Leu Val Asn Ala Thr Leu Leu Glu Ala Arg Ala
                165                 170                 175

Thr Asn Gln Phe Leu Ala Val Ser Lys Gly Asn Cys Ser Gly Pro Thr
            180                 185                 190

Thr Ile Arg Gly Gln Phe Ser Asn Met Ser Leu Ser Leu Leu Asp Leu
        195                 200                 205

Tyr Leu Ser Arg Gly Tyr Asn Val Ser Ser Ile Val Thr Met Thr Ser
    210                 215                 220

Gln Gly Met Tyr Gly Gly Thr Tyr Leu Val Glu Lys Pro Asn Leu Ser
225                 230                 235                 240

Ser Lys Gly Ser Glu Leu Ser Gln Leu Ser Met His Arg Val Phe Glu
                245                 250                 255

Val Gly Val Ile Arg Asn Pro Gly Leu Gly Ala Pro Val Phe His Met
            260                 265                 270

Thr Asn Tyr Phe Glu Gln Ser Val Ser Asn Asp Phe Ser Asn Cys Met
        275                 280                 285

Val Ala Leu Gly Glu Leu Lys Phe Ala Ala Leu Cys His Arg Glu Asp
    290                 295                 300

Ser Ile Thr Ile Pro Tyr Gln Gly Ser Gly Lys Gly Val Ser Phe Gln
305                 310                 315                 320

Leu Val Lys Leu Gly Val Trp Lys Ser Pro Thr Asp Met Gln Ser Trp
                325                 330                 335

Val Pro Leu Ser Thr Asp Asp Pro Val Ile Asp Arg Leu Tyr Leu Ser
            340                 345                 350

Ser His Arg Gly Val Ile Ala Asp Asn Gln Ala Lys Trp Ala Val Pro
        355                 360                 365

Thr Thr Arg Thr Asp Asp Lys Leu Arg Met Glu Thr Cys Phe Gln Gln
    370                 375                 380

Ala Cys Lys Gly Lys Ile Gln Ala Leu Cys Glu Asn Pro Glu Trp Ala
385                 390                 395                 400

Pro Leu Lys Asp Asn Arg Ile Pro Ser Tyr Gly Val Leu Ser Val Asn
                405                 410                 415

Leu Ser Leu Thr Val Glu Leu Lys Ile Lys Ile Ala Ser Gly Phe Gly
            420                 425                 430

Pro Leu Ile Thr His Gly Ser Gly Met Asp Leu Tyr Lys Ser Asn His
        435                 440                 445

Asn Asn Val Tyr Trp Leu Thr Ile Pro Pro Met Lys Asn Leu Ala Leu
    450                 455                 460

Gly Val Ile Asn Thr Leu Glu Trp Ile Pro Arg Phe Lys Val Ser Pro
465                 470                 475                 480

Asn Leu Phe Thr Val Pro Ile Lys Glu Ala Gly Glu Asp Cys His Ala
                485                 490                 495

Pro Thr Tyr Leu Pro Ala Glu Val Asp Gly Asp Val Lys Leu Ser Ser
            500                 505                 510

Asn Leu Val Ile Leu Pro Gly Gln Asp Leu Gln Tyr Val Leu Ala Thr
        515                 520                 525

Tyr Asp Thr Ser Arg Val Glu His Ala Val Val Tyr Tyr Val Tyr Ser
    530                 535                 540

Pro Gly Arg Ser Phe Ser Tyr Phe Tyr Pro Phe Arg Leu Pro Ile Lys
545                 550                 555                 560

Gly Val Pro Ile Glu Leu Gln Val Glu Cys Phe Thr Trp Asp Gln Lys
                565                 570                 575

Leu Trp Cys Arg His Phe Cys Val Leu Ala Asp Ser Glu Ser Gly Gly
            580                 585                 590

His Ile Thr His Ser Gly Met Val Gly Met Gly Val Ser Cys Thr Val

595                 600                 605

Thr Arg Glu Asp Gly Thr Asn Thr Arg
    610                 615

<210>  31
<211>  1725
<212>  DNA
<213>  Homo sapiens

<400>  31
atggagttgc tgatccacag gtcaagtgca atcttcctaa ctcttgctgt taatgcattg      60

tacctcacct caagtcagaa cataactgag gagttttacc aatcgacatg tagtgcagtt     120

agcagaggtt attttagtgc tttaagaaca ggttggtata ccagtgtcat aacaatagaa     180

ttaagtaata taaaagaaac caaatgcaat ggaactgaca ctaaagtaaa acttataaaa     240

caagaattag ataagtataa gaatgcagta acagaattac agctacttat gcaaaacacg     300

ccagctgcca acaaccgggc cagaagagaa gcaccacagt acatgaacta cacaatcaat     360

accacaaaaa acctaaatgt atcaataagc aagaaaagga aacgaagatt tctgggcttc     420

ttgttaggtg taggatctgc aatagcaagt ggtatagctg tatccaaagt tttacacctt     480

gaaggagaag tgaacaaaat caaaaatgct ttgttgtcta caaacaaagc tgtagtcagt     540

ctatcaaatg gggtcagtgt tttaaccagc aaagtgttag atctcaagaa ttacataaat     600

aaccgattat tacccatagt aaatcaacag agctgtcgca tctccaacat tgaaacagtt     660

atagaattcc agcagatgaa tagcagattg ttggaaatca ccagagaatt tagtgttaat     720

gcaggtgtaa caacaccttt aagcacttac atgttaacaa acagtgagtt actatcattg     780

atcaatgata tgcctataac aaatgaccag aaaaaattaa tgtcaagcaa tgttcagata     840

gtaaggcaac aaagttattc tatcatgtct ataataaagg aagaagtcct tgcatatgtt     900

gtacagctac ctatctatgg tgtaatagat acaccttgct ggaaattaca cacatcacct     960

ctatgcacca ccaacatcaa agaaggatca aatatttgtt taacaaggac tgatagagga    1020

tggtattgtg ataatgcagg atcagtatcc ttcttcccac aggctgatac ttgcaaagta    1080

cagtccaatc gagtattttg tgacactatg aacagtttaa cattaccaag tgaagtcagc    1140

ctttgtaaca ctgacatatt caattccaag tatgactgca aaattatgac atcaaaaaca    1200

gacataagca gctcagtaat tacttctctt ggagctatag tgtcatgcta tggaaaaact    1260

aaatgcactg catccaataa aaatcgtggg attataaaga cattttctaa tggttgtgac    1320

tatgtgtcaa acaaaggagt agatactgtg tcagtgggca acactttata ctatgtaaac    1380

aagctggaag gcaaaaacct ttatgtaaaa ggggaaccta taataaatta ctatgatcct    1440

ctagtgtttc cttctgatga gtttgatgca tcaatatctc aagtcaatga aaaaatcaat    1500

caaagtttag cttttattcg tagatctgat gaattactac ataatgtaaa tactggcaaa 1560

tctactacaa atattatgat aactacaatt attatagtaa tcattgtagt attgttatca 1620

ttaatagcta ttggtttact gttgtattgc aaagccaaaa acacaccagt tacactaagc 1680

aaagaccaac taagtggaat caataatatt gcattcagca aatag 1725

<210> 32
<211> 574
<212> PRT
<213> Homo sapiens

<400> 32

Met Glu Leu Leu Ile His Arg Ser Ser Ala Ile Phe Leu Thr Leu Ala
1               5                   10                  15

Val Asn Ala Leu Tyr Leu Thr Ser Ser Gln Asn Ile Thr Glu Glu Phe
            20                  25                  30

Tyr Gln Ser Thr Cys Ser Ala Val Ser Arg Gly Tyr Phe Ser Ala Leu
        35                  40                  45

Arg Thr Gly Trp Tyr Thr Ser Val Ile Thr Ile Glu Leu Ser Asn Ile
    50                  55                  60

Lys Glu Thr Lys Cys Asn Gly Thr Asp Thr Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Asn Thr Pro Ala Ala Asn Asn Arg Ala Arg Arg Glu Ala Pro
            100                 105                 110

Gln Tyr Met Asn Tyr Thr Ile Asn Thr Thr Lys Asn Leu Asn Val Ser
        115                 120                 125

Ile Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu
145                 150                 155                 160

Glu Gly Glu Val Asn Lys Ile Lys Asn Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asn Asn Arg Leu Leu Pro Ile Val Asn
        195                 200                 205

Gln Gln Ser Cys Arg Ile Ser Asn Ile Glu Thr Val Ile Glu Phe Gln
    210                 215                 220

Gln Met Asn Ser Arg Leu Leu Glu Ile Thr Arg Glu Phe Ser Val Asn
225                 230                 235                 240

Ala Gly Val Thr Thr Pro Leu Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Ser Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Ile Tyr Gly Val Ile Asp Thr Pro Cys Trp Lys Leu His Thr Ser Pro
305                 310                 315                 320

Leu Cys Thr Thr Asn Ile Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Asp Thr Cys Lys Val Gln Ser Asn Arg Val Phe Cys Asp
        355                 360                 365

Thr Met Asn Ser Leu Thr Leu Pro Ser Glu Val Ser Leu Cys Asn Thr
    370                 375                 380

Asp Ile Phe Asn Ser Lys Tyr Asp Cys Lys Ile Met Thr Ser Lys Thr
385                 390                 395                 400

Asp Ile Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile
            420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Leu Glu Gly
    450                 455                 460

Lys Asn Leu Tyr Val Lys Gly Glu Pro Ile Ile Asn Tyr Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Arg Ser Asp Glu Leu
            500                 505                 510

Leu His Asn Val Asn Thr Gly Lys Ser Thr Thr Asn Ile Met Ile Thr
        515                 520                 525

Thr Ile Ile Ile Val Ile Ile Val Val Leu Leu Ser Leu Ile Ala Ile
    530                 535                 540

Gly Leu Leu Leu Tyr Cys Lys Ala Lys Asn Thr Pro Val Thr Leu Ser
545                 550                 555                 560

Lys Asp Gln Leu Ser Gly Ile Asn Asn Ile Ala Phe Ser Lys
                565                 570

<210>  33
<211>  1497
<212>  DNA
<213>  Escherichia coli

<400>  33
atggcacaag tcattaatac caacagcctc tcgctgatca ctcaaaataa tatcaacaag      60

aaccagtctg cgctgtcgag ttctatcgag cgtctgtctt ctggcttgcg tattaacagc     120

gcgaaggatg acgcagcggg tcaggcgatt gctaaccgtt tcacctctaa cattaaaggc     180

ctgactcagg cggcccgtaa cgccaacgac ggtatctccg ttgcgcagac caccgaaggc     240

gcgctgtccg aaatcaacaa caacttacag cgtgtgcgtg aactgacggt acaggccact     300

accggtacta actctgagtc tgatctgtct tctatccagg acgaaattaa atcccgtctg     360

gatgaaattg accgcgtatc tggtcagacc cagttcaacg gcgtgaacgt gctggcaaaa     420

aatggctcca tgaaaatcca ggttggcgca aatgataacc agactatcac tatcgatctg     480

aagcagattg atgctaaaac tcttggcctt gatggtttta gcgttaaaaa taacgataca     540

gttaccacta gtgctccagt aactgctttt ggtgctacca ccacaaacaa tattaaactt     600

actggaatta ccctttctac ggaagcagcc actgatactg gcggaactaa cccagcttca     660

attgagggtg tttatactga taatggtaat gattactatg cgaaaatcac cggtggtgat     720

aacgatggga agtattacgc agtaacagtt gctaatgatg gtacagtgac aatggcgact 780
ggagcaacgg caaatgcaac tgtaactgat gcaaatacta ctaaagctac aactatcact 840
tcaggcggta cacctgttca gattgataat actgcaggtt ccgcaactgc caaccttggt 900
gctgttagct tagtaaaact gcaggattcc aagggtaatg ataccgatac atatgcgctt 960
aaagatacaa atggcaatct ttacgctgcg gatgtgaatg aaactactgg tgctgtttct 1020
gttaaaacta ttacctatac tgactcttcc ggtgccgcca gttctccaac cgcggtcaaa 1080
ctgggcggag atgatggcaa aacagaagtg gtcgatattg atggtaaaac atacgattct 1140
gccgatttaa atggcggtaa tctgcaaaca ggtttgactg ctggtggtga ggctctgact 1200
gctgttgcaa atggtaaaac cacggatccg ctgaaagcgc tggacgatgc tatcgcatct 1260
gtagacaaat tccgttcttc cctcggtgcg gtgcaaaacc gtctggattc cgcggttacc 1320
aacctgaaca acaccactac caacctgtct gaagcgcagt cccgtattca ggacgccgac 1380
tatgcgaccg aagtgtccaa tatgtcgaaa gcgcagatca tccagcaggc cggtaactcc 1440
gtgttggcaa aagctaacca ggtaccgcag caggttctgt ctctgctgca gggttaa 1497

<210> 34
<211> 498
<212> PRT
<213> Escherichia coli

<400> 34

Met Ala Gln Val Ile Asn Thr Asn Ser Leu Ser Leu Ile Thr Gln Asn
1               5                   10                  15

Asn Ile Asn Lys Asn Gln Ser Ala Leu Ser Ser Ser Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ser Asn Ile Lys Gly Leu Thr Gln Ala
    50                  55                  60

Ala Arg Asn Ala Asn Asp Gly Ile Ser Val Ala Gln Thr Thr Glu Gly
65                  70                  75                  80

Ala Leu Ser Glu Ile Asn Asn Asn Leu Gln Arg Val Arg Glu Leu Thr
                85                  90                  95

Val Gln Ala Thr Thr Gly Thr Asn Ser Glu Ser Asp Leu Ser Ser Ile
            100                 105                 110

Gln Asp Glu Ile Lys Ser Arg Leu Asp Glu Ile Asp Arg Val Ser Gly
        115                 120                 125

Gln Thr Gln Phe Asn Gly Val Asn Val Leu Ala Lys Asn Gly Ser Met
    130                 135                 140

Lys Ile Gln Val Gly Ala Asn Asp Asn Gln Thr Ile Thr Ile Asp Leu
145                 150                 155                 160

Lys Gln Ile Asp Ala Lys Thr Leu Gly Leu Asp Gly Phe Ser Val Lys
                165                 170                 175

Asn Asn Asp Thr Val Thr Thr Ser Ala Pro Val Thr Ala Phe Gly Ala
            180                 185                 190

Thr Thr Thr Asn Asn Ile Lys Leu Thr Gly Ile Thr Leu Ser Thr Glu
        195                 200                 205

Ala Ala Thr Asp Thr Gly Gly Thr Asn Pro Ala Ser Ile Glu Gly Val
    210                 215                 220

Tyr Thr Asp Asn Gly Asn Asp Tyr Tyr Ala Lys Ile Thr Gly Gly Asp
225                 230                 235                 240

Asn Asp Gly Lys Tyr Tyr Ala Val Thr Val Ala Asn Asp Gly Thr Val
                245                 250                 255

Thr Met Ala Thr Gly Ala Thr Ala Asn Ala Thr Val Thr Asp Ala Asn
            260                 265                 270

Thr Thr Lys Ala Thr Thr Ile Thr Ser Gly Gly Thr Pro Val Gln Ile
        275                 280                 285

Asp Asn Thr Ala Gly Ser Ala Thr Ala Asn Leu Gly Ala Val Ser Leu
    290                 295                 300

Val Lys Leu Gln Asp Ser Lys Gly Asn Asp Thr Asp Thr Tyr Ala Leu
305                 310                 315                 320

Lys Asp Thr Asn Gly Asn Leu Tyr Ala Ala Asp Val Asn Glu Thr Thr
                325                 330                 335

Gly Ala Val Ser Val Lys Thr Ile Thr Tyr Thr Asp Ser Ser Gly Ala
            340                 345                 350

Ala Ser Ser Pro Thr Ala Val Lys Leu Gly Gly Asp Asp Gly Lys Thr
        355                 360                 365

Glu Val Val Asp Ile Asp Gly Lys Thr Tyr Asp Ser Ala Asp Leu Asn
    370                 375                 380

Gly Gly Asn Leu Gln Thr Gly Leu Thr Ala Gly Gly Glu Ala Leu Thr
385                 390                 395                 400

Ala Val Ala Asn Gly Lys Thr Thr Asp Pro Leu Lys Ala Leu Asp Asp
                405                 410                 415

Ala Ile Ala Ser Val Asp Lys Phe Arg Ser Ser Leu Gly Ala Val Gln
            420                 425                 430

Asn Arg Leu Asp Ser Ala Val Thr Asn Leu Asn Asn Thr Thr Thr Asn
        435                 440                 445

Leu Ser Glu Ala Gln Ser Arg Ile Gln Asp Ala Asp Tyr Ala Thr Glu
    450                 455                 460

Val Ser Asn Met Ser Lys Ala Gln Ile Ile Gln Gln Ala Gly Asn Ser
465                 470                 475                 480

Val Leu Ala Lys Ala Asn Gln Val Pro Gln Gln Val Leu Ser Leu Leu
                485                 490                 495

Gln Gly


<210>  35
<211>  51
<212>  DNA
<213>  artificial

<220>
<223>  S-Tag nucleotide sequence

<400>  35
atgaaagaaa ccgctgctgc taaattcgaa cgccagcaca tggacagccc a          51


<210>  36
<211>  17
<212>  PRT
<213>  artificial

<220>
<223>  S-Tag polypeptide

<400>  36

Met Lys Glu Thr Ala Ala Ala Lys Phe Glu Arg Gln His Met Asp Ser
1               5                   10                  15

Pro

<210> 37
<211> 20
<212> DNA
<213> artificial

<220>
<223> primer

<400> 37
ggagctgtcg tattccagtc 20

<210> 38
<211> 20
<212> DNA
<213> artificial

<220>
<223> primer

<400> 38
aacccctcaa gacccgttta 20

<210> 39
<211> 459
<212> PRT
<213> Listeria monocytogenes

<400> 39

Pro Ile Glu Lys Lys His Ala Asp Glu Ile Asp Lys Tyr Ile Gln Gly
1               5                   10                  15

Leu Asp Tyr Asn Lys Asn Asn Val Leu Val Tyr His Gly Asp Ala Val
            20                  25                  30

Thr Asn Val Pro Pro Arg Lys Gly Tyr Lys Asp Gly Asn Glu Tyr Ile
        35                  40                  45

Val Val Glu Lys Lys Lys Lys Ser Ile Asn Gln Asn Asn Ala Asp Ile
    50                  55                  60

Gln Val Val Asn Ala Ile Ser Ser Leu Thr Tyr Pro Gly Ala Leu Val
65                  70                  75                  80

Lys Ala Asn Ser Glu Leu Val Glu Asn Gln Pro Asp Val Leu Pro Val
                85                  90                  95

Lys Arg Asp Ser Leu Thr Leu Ser Ile Asp Leu Pro Gly Met Thr Asn
            100                 105                 110

Gln Asp Asn Lys Ile Val Val Lys Asn Ala Thr Lys Ser Asn Val Asn
        115                     120                     125

Asn Ala Val Asn Thr Leu Val Glu Arg Trp Asn Glu Lys Tyr Ala Gln
    130                     135                     140

Ala Tyr Pro Asn Val Ser Ala Lys Ile Asp Tyr Asp Asp Glu Met Ala
145                     150                     155                     160

Tyr Ser Glu Ser Gln Leu Ile Ala Lys Phe Gly Thr Ala Phe Lys Ala
                165                     170                     175

Val Asn Asn Ser Leu Asn Val Asn Phe Gly Ala Ile Ser Glu Gly Lys
            180                     185                     190

Met Gln Glu Glu Val Ile Ser Phe Lys Gln Ile Tyr Tyr Asn Val Asn
        195                     200                     205

Val Asn Glu Pro Thr Arg Pro Ser Arg Phe Phe Gly Lys Ala Val Thr
    210                     215                     220

Lys Glu Gln Leu Gln Ala Leu Gly Val Asn Ala Glu Asn Pro Pro Ala
225                     230                     235                     240

Tyr Ile Ser Ser Val Ala Tyr Gly Arg Gln Val Tyr Leu Lys Leu Ser
                245                     250                     255

Thr Asn Ser His Ser Thr Lys Val Lys Ala Ala Phe Asp Ala Ala Val
            260                     265                     270

Ser Gly Lys Ser Val Ser Gly Asp Val Glu Leu Thr Asn Ile Ile Lys
        275                     280                     285

Asn Ser Ser Phe Lys Ala Val Ile Tyr Gly Gly Ser Ala Lys Asp Glu
    290                     295                     300

Val Gln Ile Ile Asp Gly Asn Leu Gly Asp Leu Arg Asp Ile Leu Lys
305                     310                     315                     320

Lys Gly Ala Thr Phe Asn Arg Glu Thr Pro Gly Val Val Asp Gln Asn
                325                     330                     335

Ala Thr Thr His Ala Val Lys Ser Gly Asp Thr Ile Trp Ala Leu Ser
            340                     345                     350

Val Lys Tyr Gly Val Ser Val Gln Asp Ile Met Ser Trp Asn Asn Leu
        355                     360                     365

Ser Ser Ser Ser Ile Tyr Val Gly Gln Lys Leu Ala Ile Lys Gln Thr
    370                 375                 380

Ala Asn Thr Ala Thr Pro Lys Ala Glu Val Lys Thr Glu Ala Pro Ala
385                 390                 395                 400

Ala Glu Lys Gln Ala Ala Pro Val Val Lys Glu Asn Thr Asn Thr Asn
                405                 410                 415

Thr Ala Thr Thr Glu Lys Lys Glu Thr Ala Thr Gln Gln Gln Thr Ala
            420                 425                 430

Pro Lys Ala Pro Thr Glu Ala Ala Lys Pro Ala Pro Ala Pro Ser Thr
        435                 440                 445

Asn Thr Asn Ala Asn Lys Thr Asn Thr Asn Thr
    450                 455

<210>  40
<211>  14
<212>  PRT
<213>  artificial

<220>
<223>  V5 epitope

<400>  40

Gly Lys Pro Ile Pro Asn Pro Leu Leu Gly Leu Asp Ser Thr
1               5                   10

<210>  41
<211>  47
<212>  DNA
<213>  artificial

<220>
<223>  primer

<400>  41
gaattgtaat acgactcact atagggaggt gatgaagata ccccacc                47

<210>  42
<211>  41
<212>  DNA
<213>  artificial

<220>
<223>  primer

<400>  42
taatacgact cactataggg cgaagtgtat caacaagctg g                      41

<210> 43
<211> 47
<212> DNA
<213> artificial

<220>
<223> primer

<400> 43
gaattgtaat acgactcact atagggaggt gatgaagata ccccacc 47

<210> 44
<211> 41
<212> DNA
<213> artificial

<220>
<223> primer

<400> 44
taatacgact cactataggg cgaagtgtat caacaagctg g 41

<210> 45
<211> 20
<212> DNA
<213> artificial

<220>
<223> primer

<400> 45
ggagctgtcg tattccagtc 20

<210> 46
<211> 20
<212> DNA
<213> artificial

<220>
<223> primer

<400> 46
aacccctcaa gacccgttta 20

<210> 47
<211> 37
<212> DNA
<213> artificial

<220>
<223> primer

<400> 47
cttaaagaat tcccaatcga aaagaaacac gcggatg 37

<210> 48
<211> 30
<212> DNA

<213>  artificial

<220>
<223>  primer

<400>  48
ttctactaat tccgagttcg cttttacgag                                        30


<210>  49
<211>  30
<212>  DNA
<213>  artificial

<220>
<223>  primer

<400>  49
ctcgtaaaag cgaactcgga attagtagaa                                        30


<210>  50
<211>  37
<212>  DNA
<213>  artificial

<220>
<223>  primer

<400>  50
agaggtctcg agtgtatttg ttttattagc atttgtg                                37


<210>  51
<211>  479
<212>  PRT
<213>  Listeria monocytogenes

<400>  51

Lys Gly Gly Val Gly Pro Val Arg Arg Ser Ser Arg Leu Arg Arg Thr
1               5                   10                  15


Thr Gln Pro Gly Pro Ile Glu Lys Lys His Ala Asp Glu Ile Asp Lys
            20                  25                  30


Tyr Ile Gln Gly Leu Asp Tyr Asn Lys Asn Asn Val Leu Val Tyr His
        35                  40                  45


Gly Asp Ala Val Thr Asn Val Pro Pro Arg Lys Gly Tyr Lys Asp Gly
    50                  55                  60


Asn Glu Tyr Ile Val Val Glu Lys Lys Lys Lys Ser Ile Asn Gln Asn
65                  70                  75                  80


Asn Ala Asp Ile Gln Val Val Asn Ala Ile Ser Ser Leu Thr Tyr Pro
                85                  90                  95

Gly Ala Leu Val Lys Ala Asn Ser Glu Leu Val Glu Asn Gln Pro Asp
            100                 105                 110

Val Leu Pro Val Lys Arg Asp Ser Leu Thr Leu Ser Ile Asp Leu Pro
        115                 120                 125

Gly Met Thr Asn Gln Asp Asn Lys Ile Val Val Lys Asn Ala Thr Lys
    130                 135                 140

Ser Asn Val Asn Asn Ala Val Asn Thr Leu Val Glu Arg Trp Asn Glu
145                 150                 155                 160

Lys Tyr Ala Gln Ala Tyr Pro Asn Val Ser Ala Lys Ile Asp Tyr Asp
                165                 170                 175

Asp Glu Met Ala Tyr Ser Glu Ser Gln Leu Ile Ala Lys Phe Gly Thr
            180                 185                 190

Ala Phe Lys Ala Val Asn Asn Ser Leu Asn Val Asn Phe Gly Ala Ile
        195                 200                 205

Ser Glu Gly Lys Met Gln Glu Glu Val Ile Ser Phe Lys Gln Ile Tyr
    210                 215                 220

Tyr Asn Val Asn Val Asn Glu Pro Thr Arg Pro Ser Arg Phe Phe Gly
225                 230                 235                 240

Lys Ala Val Thr Lys Glu Gln Leu Gln Ala Leu Gly Val Asn Ala Glu
                245                 250                 255

Asn Pro Pro Ala Tyr Ile Ser Ser Val Ala Tyr Gly Arg Gln Val Tyr
            260                 265                 270

Leu Lys Leu Ser Thr Asn Ser His Ser Thr Lys Val Lys Ala Ala Phe
        275                 280                 285

Asp Ala Ala Val Ser Gly Lys Ser Val Ser Gly Asp Val Glu Leu Thr
    290                 295                 300

Asn Ile Ile Lys Asn Ser Ser Phe Lys Ala Val Ile Tyr Gly Gly Ser
305                 310                 315                 320

Ala Lys Asp Glu Val Gln Ile Ile Asp Gly Asn Leu Gly Asp Leu Arg
                325                 330                 335

Asp Ile Leu Lys Lys Gly Ala Thr Phe Asn Arg Glu Thr Pro Gly Val
            340                 345                 350

Val Asp Gln Asn Ala Thr Thr His Ala Val Lys Ser Gly Asp Thr Ile
        355                 360                 365

Trp Ala Leu Ser Val Lys Tyr Gly Val Ser Val Gln Asp Ile Met Ser
    370                 375                 380

Trp Asn Asn Leu Ser Ser Ser Ser Ile Tyr Val Gly Gln Lys Leu Ala
385                 390                 395                 400

Ile Lys Gln Thr Ala Asn Thr Ala Thr Pro Lys Ala Glu Val Lys Thr
                405                 410                 415

Glu Ala Pro Ala Ala Glu Lys Gln Ala Ala Pro Val Val Lys Glu Asn
            420                 425                 430

Thr Asn Thr Asn Thr Ala Thr Thr Glu Lys Lys Glu Thr Ala Thr Gln
        435                 440                 445

Gln Gln Thr Ala Pro Lys Ala Pro Thr Glu Ala Ala Lys Pro Ala Pro
    450                 455                 460

Ala Pro Ser Thr Asn Thr Asn Ala Asn Lys Thr Asn Thr Asn Thr
465                 470                 475

<210>  52
<211>  482
<212>  PRT
<213>  Listeria monocytogenes

<400>  52

Gly Arg Arg Gly Leu Cys Arg Gly Cys Arg Thr Arg Gly Arg Ile Lys
1               5                   10                  15

Gln Leu Gln Ser Ala His Lys Pro Ile Glu Lys Lys His Ala Asp Glu
            20                  25                  30

Ile Asp Lys Tyr Ile Gln Gly Leu Asp Tyr Asn Lys Asn Asn Val Leu
        35                  40                  45

Val Tyr His Gly Asp Ala Val Thr Asn Val Pro Pro Arg Lys Gly Tyr
    50                  55                  60

Lys Asp Gly Asn Glu Tyr Ile Val Val Glu Lys Lys Lys Lys Ser Ile
65                  70                  75                  80

Asn Gln Asn Asn Ala Asp Ile Gln Val Val Asn Ala Ile Ser Ser Leu
                85                  90                  95

Thr Tyr Pro Gly Ala Leu Val Lys Ala Asn Ser Glu Leu Val Glu Asn
            100                 105                 110

Gln Pro Asp Val Leu Pro Val Lys Arg Asp Ser Leu Thr Leu Ser Ile
        115                 120                 125

Asp Leu Pro Gly Met Thr Asn Gln Asp Asn Lys Ile Val Val Lys Asn
    130                 135                 140

Ala Thr Lys Ser Asn Val Asn Asn Ala Val Asn Thr Leu Val Glu Arg
145                 150                 155                 160

Trp Asn Glu Lys Tyr Ala Gln Ala Tyr Pro Asn Val Ser Ala Lys Ile
                165                 170                 175

Asp Tyr Asp Asp Glu Met Ala Tyr Ser Glu Ser Gln Leu Ile Ala Lys
            180                 185                 190

Phe Gly Thr Ala Phe Lys Ala Val Asn Asn Ser Leu Asn Val Asn Phe
        195                 200                 205

Gly Ala Ile Ser Glu Gly Lys Met Gln Glu Glu Val Ile Ser Phe Lys
    210                 215                 220

Gln Ile Tyr Tyr Asn Val Asn Val Asn Glu Pro Thr Arg Pro Ser Arg
225                 230                 235                 240

Phe Phe Gly Lys Ala Val Thr Lys Glu Gln Leu Gln Ala Leu Gly Val
                245                 250                 255

Asn Ala Glu Asn Pro Pro Ala Tyr Ile Ser Ser Val Ala Tyr Gly Arg
            260                 265                 270

Gln Val Tyr Leu Lys Leu Ser Thr Asn Ser His Ser Thr Lys Val Lys
        275                 280                 285

Ala Ala Phe Asp Ala Ala Val Ser Gly Lys Ser Val Ser Gly Asp Val
    290                 295                 300

Glu Leu Thr Asn Ile Ile Lys Asn Ser Ser Phe Lys Ala Val Ile Tyr
305                 310                 315                 320

Gly Gly Ser Ala Lys Asp Glu Val Gln Ile Ile Asp Gly Asn Leu Gly
                325                 330                 335

Asp Leu Arg Asp Ile Leu Lys Lys Gly Ala Thr Phe Asn Arg Glu Thr

340                    345                    350

Pro Gly Val Val Asp Gln Asn Ala Thr Thr His Ala Val Lys Ser Gly
        355                 360                 365

Asp Thr Ile Trp Ala Leu Ser Val Lys Tyr Gly Val Ser Val Gln Asp
    370                 375                 380

Ile Met Ser Trp Asn Asn Leu Ser Ser Ser Ser Ile Tyr Val Gly Gln
385                 390                 395                 400

Lys Leu Ala Ile Lys Gln Thr Ala Asn Thr Ala Thr Pro Lys Ala Glu
                405                 410                 415

Val Lys Thr Glu Ala Pro Ala Ala Glu Lys Gln Ala Ala Pro Val Val
            420                 425                 430

Lys Glu Asn Thr Asn Thr Asn Thr Ala Thr Thr Glu Lys Lys Glu Thr
        435                 440                 445

Ala Thr Gln Gln Gln Thr Ala Pro Lys Ala Pro Thr Glu Ala Ala Lys
    450                 455                 460

Pro Ala Pro Ala Pro Ser Thr Asn Thr Asn Ala Asn Lys Thr Asn Thr
465                 470                 475                 480

Asn Thr

<210>  53
<211>  481
<212>  PRT
<213>  Listeria monocytogenes

<400>  53

Arg Trp Gly Tyr His Leu Arg Asp Arg Lys Tyr Lys Gly Val Arg Ser
1               5                   10                  15

His Lys Gly Val Pro Arg Pro Ile Glu Lys Lys His Ala Asp Glu Ile
            20                  25                  30

Asp Lys Tyr Ile Gln Gly Leu Asp Tyr Asn Lys Asn Asn Val Leu Val
        35                  40                  45

Tyr His Gly Asp Ala Val Thr Asn Val Pro Pro Arg Lys Gly Tyr Lys
    50                  55                  60

Asp Gly Asn Glu Tyr Ile Val Val Glu Lys Lys Lys Lys Ser Ile Asn

65                  70                  75                  80

Gln Asn Asn Ala Asp Ile Gln Val Val Asn Ala Ile Ser Ser Leu Thr
                85                  90                  95

Tyr Pro Gly Ala Leu Val Lys Ala Asn Ser Glu Leu Val Glu Asn Gln
            100                 105                 110

Pro Asp Val Leu Pro Val Lys Arg Asp Ser Leu Thr Leu Ser Ile Asp
        115                 120                 125

Leu Pro Gly Met Thr Asn Gln Asp Asn Lys Ile Val Val Lys Asn Ala
    130                 135                 140

Thr Lys Ser Asn Val Asn Asn Ala Val Asn Thr Leu Val Glu Arg Trp
145                 150                 155                 160

Asn Glu Lys Tyr Ala Gln Ala Tyr Pro Asn Val Ser Ala Lys Ile Asp
                165                 170                 175

Tyr Asp Asp Glu Met Ala Tyr Ser Glu Ser Gln Leu Ile Ala Lys Phe
            180                 185                 190

Gly Thr Ala Phe Lys Ala Val Asn Asn Ser Leu Asn Val Asn Phe Gly
        195                 200                 205

Ala Ile Ser Glu Gly Lys Met Gln Glu Glu Val Ile Ser Phe Lys Gln
    210                 215                 220

Ile Tyr Tyr Asn Val Asn Val Asn Glu Pro Thr Arg Pro Ser Arg Phe
225                 230                 235                 240

Phe Gly Lys Ala Val Thr Lys Glu Gln Leu Gln Ala Leu Gly Val Asn
                245                 250                 255

Ala Glu Asn Pro Pro Ala Tyr Ile Ser Ser Val Ala Tyr Gly Arg Gln
            260                 265                 270

Val Tyr Leu Lys Leu Ser Thr Asn Ser His Ser Thr Lys Val Lys Ala
        275                 280                 285

Ala Phe Asp Ala Ala Val Ser Gly Lys Ser Val Ser Gly Asp Val Glu
    290                 295                 300

Leu Thr Asn Ile Ile Lys Asn Ser Ser Phe Lys Ala Val Ile Tyr Gly
305                 310                 315                 320

Gly Ser Ala Lys Asp Glu Val Gln Ile Ile Asp Gly Asn Leu Gly Asp
                325                 330                 335

Leu Arg Asp Ile Leu Lys Lys Gly Ala Thr Phe Asn Arg Glu Thr Pro
            340                 345                 350

Gly Val Val Asp Gln Asn Ala Thr Thr His Ala Val Lys Ser Gly Asp
        355                 360                 365

Thr Ile Trp Ala Leu Ser Val Lys Tyr Gly Val Ser Val Gln Asp Ile
    370                 375                 380

Met Ser Trp Asn Asn Leu Ser Ser Ser Ser Ile Tyr Val Gly Gln Lys
385                 390                 395                 400

Leu Ala Ile Lys Gln Thr Ala Asn Thr Ala Thr Pro Lys Ala Glu Val
                405                 410                 415

Lys Thr Glu Ala Pro Ala Ala Glu Lys Gln Ala Ala Pro Val Val Lys
            420                 425                 430

Glu Asn Thr Asn Thr Asn Thr Ala Thr Thr Glu Lys Lys Glu Thr Ala
        435                 440                 445

Thr Gln Gln Gln Thr Ala Pro Lys Ala Pro Thr Glu Ala Ala Lys Pro
    450                 455                 460

Ala Pro Ala Pro Ser Thr Asn Thr Asn Ala Asn Lys Thr Asn Thr Asn
465                 470                 475                 480

Thr

<210> 54
<211> 5
<212> PRT
<213> artificial

<220>
<223> synthetic

<400> 54

Asn Pro Pro Thr Thr
1               5

<210> 55
<211> 5
<212> PRT
<213> artificial

<220>
<223>  synthetic

<400>  55

Met Arg Arg Ile Leu
1               5


<210>  56
<211>  4
<212>  PRT
<213>  artificial

<220>
<223>  synthetic

<400>  56

Met Ile Ser Ser
1


<210>  57
<211>  5
<212>  PRT
<213>  artificial

<220>
<223>  synthetic

<400>  57

Arg Gly Gly Ser Lys
1               5


<210>  58
<211>  4
<212>  PRT
<213>  artificial

<220>
<223>  synthetic

<400>  58

Arg Gly Gly Phe
1


<210>  59
<211>  5
<212>  PRT
<213>  artificial

<220>
<223>  synthetic

<400>  59

Asn Arg Thr Val Phe
1               5

<210>  60
<211>  5
<212>  PRT
<213>  artificial

<220>
<223>  synthetic

<400>  60

Asn Arg Phe Gly Leu
1               5


<210>  61
<211>  5
<212>  PRT
<213>  artificial

<220>
<223>  synthetic

<400>  61

Ser Arg His Gly Arg
1               5


<210>  62
<211>  5
<212>  PRT
<213>  artificial

<220>
<223>  synthetic

<400>  62

Ile Met Arg His Pro
1               5


<210>  63
<211>  5
<212>  PRT
<213>  artificial

<220>
<223>  synthetic

<400>  63

Glu Val Cys Ala Pro
1               5


<210>  64
<211>  5
<212>  PRT
<213>  artificial

<220>
<223>  synthetic

<400>  64

Ala Cys Gly Val Tyr
1               5


<210>  65
<211>  5
<212>  PRT
<213>  artificial

<220>
<223>  synthetic

<400>  65

Cys Gly Pro Lys Leu
1               5


<210>  66
<211>  5
<212>  PRT
<213>  artificial

<220>
<223>  synthetic

<400>  66

Ala Gly Cys Phe Ser
1               5


<210>  67
<211>  5
<212>  PRT
<213>  artificial

<220>
<223>  synthetic

<400>  67

Ser Gly Gly Leu Phe
1               5


<210>  68
<211>  5
<212>  PRT
<213>  artificial

<220>
<223>  synthetic

<400>  68

Ala Val Arg Leu Ser
1               5

<210> 69
<211> 5
<212> PRT
<213> artificial

<220>
<223> synthetic

<400> 69

Gly Gly Lys Leu Ser
1               5


<210> 70
<211> 5
<212> PRT
<213> artificial

<220>
<223> synthetic

<400> 70

Val Ser Glu Gly Val
1               5


<210> 71
<211> 5
<212> PRT
<213> artificial

<220>
<223> synthetic

<400> 71

Lys Cys Gln Ser Phe
1               5


<210> 72
<211> 5
<212> PRT
<213> artificial

<220>
<223> synthetic

<400> 72

Phe Cys Gly Leu Gly
1               5


<210> 73
<211> 5
<212> PRT
<213> artificial

<220>
<223>  synthetic

<400>  73

Pro Glu Ser Gly Val
1               5


<210>  74
<211>  49
<212>  DNA
<213>  artificial

<220>
<223>  primer

<400>  74
gacgacgaca agattgagac cactcagaac aaggccgcac ttaccgacc                    49


<210>  75
<211>  39
<212>  DNA
<213>  artificial

<220>
<223>  primer

<400>  75
gaggagaagc ccggtctatt actcgagtgc ggccgcaag                               39


<210>  76
<211>  57
<212>  DNA
<213>  artificial

<220>
<223>  primer

<400>  76
tatgcatcat caccatcacc atgatgacga cgacaagagc ccgggcttct cctcagc           57


<210>  77
<211>  58
<212>  DNA
<213>  artificial

<220>
<223>  primer

<400>  77
tcagctgagg agaagcccgg gctcttgtcg tcgtcatcat ggtgatggtg atgatgca          58


<210>  78
<211>  57
<212>  DNA
<213>  artificial

<220>

<223>  primer

<400>  78
gatctcatca tcaccatcac catgatgacg acgacaagag cccgggcttc tcctcaa          57


<210>  79
<211>  57
<212>  DNA
<213>  artificial

<220>
<223>  primer

<400>  79
cgcgttgagg agaagcccgg gctcttgtcg tcgtcatcat ggtgatggtg atgatga          57


<210>  80
<211>  76
<212>  DNA
<213>  artificial

<220>
<223>  synthetic oligonucleotide


<220>
<221>  misc_feature
<222>  (1)..(76)
<223>  n is a, c, t, or g

<220>
<221>  misc_feature
<222>  (1)..(76)
<223>  k is t or g

<400>  80
catgcccgga attcctgcnn knnknnknnk nnknnknnkn nknnknnktg cggaggagga       60

taaaagcttt cgagac                                                       76


<210>  81
<211>  67
<212>  DNA
<213>  artificial

<220>
<223>  synthetic oligonucleotide


<220>
<221>  misc_feature
<222>  (1)..(67)
<223>  n is a, c, t, or g

<220>
<221>  misc_feature
<222>  (1)..(67)
<223>  k is t or g

<400>  81

catgcccgga attcctgcnn knnknnknnk nnknnknnkt gcggaggagg ataaaagctt 60
tcgagac 67

## Description

This application claims priority to U.S. Provisional Patent Application Serial No. 60/648,923, filed on January 31, 2005.

### FIELD OF THE INVENTION

The present invention provides novel polypeptide ligands for Toll-like Receptor 2 (TLR2). Preferrably, the novel polypeptide ligands modulate TLR2 signaling and thereby regulate the Innate Immune Response. The invention also provides vaccines comprising the novel polypeptide TLR2 ligands and an antigen. The invention further provides methods of modulating TLR2 signaling using the polypeptide ligands or vaccines of the invention.

The research leading to this invention was supported, in part, by contract # HHSN266200400043C/N01-AI-40043 awarded by the National Institutes of Health. Accordingly, the United States government may have certain rights to this invention.

### BACKGROUND OF THE INVENTION

Multicellular organisms have developed two general systems of immunity to infectious agents. The two systems are innate or natural immunity (usually referred to as "innate immunity") and adaptive (acquired) or specific immunity. The major difference between the two systems is the mechanism by which they recognize infectious agents. Recent studies have demonstrated that the innate immune system plays a crucial role in the control of initiation of the adaptive immune response and in the induction of appropriate cell effector responses (Fearon et al. Science 1996;272:50-53 and Medzhitov et at. Cell 1997;91:295-298).

The innate immune system uses a set of germline-encoded receptors for the recognition of conserved molecular patterns present in microorganisms. These molecular patterns occur in certain constituents of microorganisms including: lipopolysaccharides, peptidoglycans, lipoteichoic acids, phosphatidyl cholines, bacterial proteins, including lipoproteins, bacterial DNAs, viral single and double-stranded RNAs, unmethylated CpG-DNAs, mannans, and a variety of other bacterial and fungal cell wall components. Such molecular patterns can also occur in other molecules such as plant alkaloids. These targets of innate immune recognition are called Pathogen Associated Molecular Patterns (PAMPs) since they are produced by microorganisms and not by the infected host organism (Janeway et al. Cold Spring Harb. Symp. Quant. Biol. 1989;54:1-13 and Medzhitov et al. Curr. Opin Immunol. 1997;94:4-9). PAMPs are discrete molecular structures that are shared by a large group of microorganisms. They are conserved products of microbial metabolism, which are not subject to antigenic variability (Medzhitov et al. Cur Op Immun 1997;94:4-9).

The receptors of the innate immune system that recognize PAMPs are called Pattern Recognition Receptors (PRRs) (Janeway et al. Cold Spring Harb. Symp. Quant. Biol. 1989;54:1-13 and Medzhitov et al. Curr. Opin. Immunol. 1997;94:4-9). These receptors vary in structure and belong to several different protein families. Some of these receptors recognize PAMPs directly (*e*.*g*., CD14, DEC205, collectins), while others (*e*.*g*., complement receptors) recognize the products generated by PAMP recognition.

Cellular PRRs are expressed on effector cells of the innate immune system, including cells that function as professional antigen-presenting cells (APC) in adaptive immunity. Such effector cells include, but are not limited to, macrophages, dendritic cells, B lymphocytes, and surface epithelia. This expression profile allows PRRs to directly induce innate effector mechanisms, and also to alert the host organism to the presence of infectious agents by inducing the expression of a set of endogenous signals, such as inflammatory cytokines and chemokines. This latter function allows efficient mobilization of effector forces to combat the invaders.

The best characterized class of cellular PRRs are members of the family of Toll-like Receptors (TLRs), so called because they are homologous to the *Drosophila* Toll protein which is involved both in dorsoventral patterning in *Drosophila* embryos and in the immune response in adult flies (Lemaitre et al. Cell 1996;86:973-83). At least 12 mammalian TLRs, TLRs 1 through 11 and TLR13, have been identified to date (see, for example, Medzhitov et al. Nature 1997;388:394-397; Rock et al. Proc Natl Acad Sci USA 1998;95:588-593; Takeuchi et at. Gene 1999;231:59-65; and Chuang and Ulevitch. Biochim Biophys Acta. 2001;1518:157-61).

In mammalian organisms, such TLRs have been shown to recognize PAMPs such as the bacterial products LPS (Schwandner et al. J. Biol. Chem. 1999;274:17406-9 and Hoshino et al. J. Immunol 1999;162:3749-3752), lipoteichoic acid (Schwandner et al. J. Biol. Chem. 1999;274:17406-9), peptidoglycan (Yoshimura et al. J. Immunol. 1999;163:1-5), lipoprotein (Aliprantis et al. Science 1999;285:736-9), CpG-DNA (Hemmi et al. Nature 2000;408:740-745), and flagellin (Hayashi et al. Nature 2001;410:1099-1103), as well as the viral product double-stranded RNA (Alexopoulou et al. Nature 2001;413:732-738) and the yeast product zymosan (Underhill. J Endotoxin Res. 2003;9:176-80).

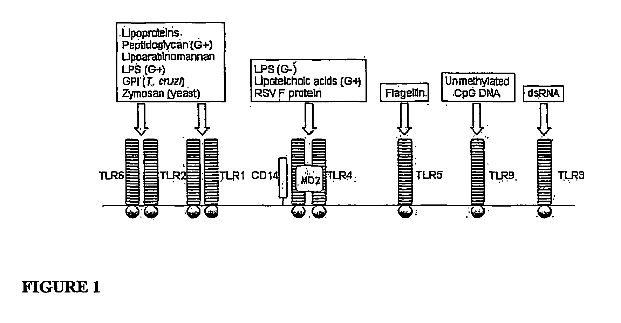

TLR2 is essential for the recognition of a variety of PAMPs, including bacterial lipoproteins, peptidoglycan, and lipoteichoic acids. TLR3 is implicated in recognition of viral double-stranded RNA. TLR4 is predominantly activated by lipopolysaccharide. TLR5 detects bacterial flagellin and TLR9 is required for response to unmethylated CpG DNA. Recently, TLR7 and TLR8 have been shown to recognize small synthetic antiviral molecules (Jurk M. et al. Nat Immunol 2002;3:499). Furthermore, in many instances, TLRs require the presence of a co-receptor to initiate the signaling cascade. One example is TLR4 which interacts with MD2 and CD14, a protein that exists both in soluble form and as a GPI-anchored protein, to induce NF-kB in response to LPS stimulation (Takeuchi and Akira. Microbes Infect 2002;4:887-95). Figure 1 illustrates some of the known interactions between PAMPs and TLRs (reviewed in Janeway and Medzhitov. Annu Rev Immunol 2002;20:197-216).

TLR2 is involved in the recognition of, *e*.*g*., multiple products of Gram-positive bacteria, mycobacteria and yeast, including LPS and lipoproteins. TLR2 is known to heterodimerize with other TLRs, a property believed to extend the range of PAMPs that TLR2 can recognize. For example, TLR2 cooperates with TLR6 in the response to peptidoglycan (Ozinsky et al. Proc Natl Acad Sci U S A 2000;97:13766-71) and diacylated mycoplasmal lipopeptide (Takeuchi et al. Int Immunol 2001;13:933-40), and associates with TLR1 to recognize triacylated lipopeptides (Takeuchi et al. J Immunol 2002;169:10-4). Pathogen recognition by TLR2 is strongly enhanced by CD 14. A pentapeptide derived from fimbrial subunit protein, ALTTE, was shown to activate monocytes and epithelial cells via TLR2 signaling (Ogawa et al. FEMS Immunol Med Microbiol 1995;11:197-206; Asai et al. Infect Immun 2001;69:7378-7395; and Ogawa et al. Eur J Immunol 2002;32:2543-2550). A single amino acid substitution (A to G) in the peptide (GLTTE) was shown to antagonize the activity of the wild-type peptide and full-length protein (Ogawa et al. FEMS Immunol Med Microbiol 1995;11:197-206).

Activation of signal transduction pathways by TLRs leads to the induction of various genes including inflammatory cytokines, chemokines, major histocompatability complex, and co-stimulatory molecules (*e*.*g*., B7). The intracellular signaling pathways initiated by activated TLRs vary slightly from TLR to TLR, with some signaling pathways being common to all TLRs (shared pathways), and some being specific to particular TLRs (specific pathways).

In one of the shared pathways, the cytoplasmic adaptor proteins myeloid differentiation factor 88 (MyD88) and TOLLIP (Toll-interacting protein) independently associate with the cytoplasmic tail of the TLR. Each of these adaptors recruits the serine/threonine kinase IRAK to the receptor complex, each with different kinetics. Recruitment of IRAK to the receptor complex results in auto-phosphorylation of IRAK. Phosphorylated IRAK then associates with another adaptor protein, TRAF6. TRAF6, in turn, associates with and activates the MAP kinase kinases TAK-1 and MKK6. Activation of TAK-1 leads, via one or more intermediate steps, to the activation of the IκB kinase (IKK), whose activity directs the degradation of IκB and the activation of NF-κB. Activation of MKK6 leads to the activation of JNK (c-Jun N-terminal kinase) and the MAP kinase p38 (Medzhitov and Janeway. Trends in Microbiology 2000;8:452-456 and Medzhitov. Nature Reviews 2001;1:135-145). Other cytoplasmic proteins implicated in TLR signaling include the RHO family GTPase RAC1 and protein kinase B (PKB), as well as the adapter protein TIRAP and its associated proteins protein kinase R (PKR) and the PKR regulatory proteins PACT and p58 (Medzhitov. Nature Reviews 2001;1:135-145). Cytoplasmic proteins specifically implicated in TLR-signaling by mutational studies include MyD88 (Schnare et al. Nature Immunol 2001;2:947-950), TIRAP (Horng et al. Nature Immunol 2001;2:835-842), IRAK and TRAF6 (Medzhitov et al. Mol Cell 1998;2:253-258), RICK/Rip2/CARDIAK (Kobayashi et al. Nature 2002;416:194-199), IRAK-4 (Suzuki et al. Nature 2002;416:750-746), and Mal (MyD88-adapter like) (Fitzgerald et al. Nature 2001;413:78-83).

Due to TLR signaling through shared pathways (*e*.*g*. NF-κB, see above), some biological responses will likely be globally induced by any TLR signaling event. However, an emerging body of evidence demonstrates divergent responses induced by the specific pathways of individual TLRs. For example, TLR2 and TLR4 activate different immunological programs in human and murine cells, manifested in divergent patterns of cytokine expression (Hirschfeld et al. Infect Immun 2001;69:1477-1482 and Re and Strominger. J Biol Chem 2001;276:37692-37699). These divergent phenotypes could be detected in an antigen-specific response, when lipopolysaccharides that signal through TLR2 or TLR4 were used to guide the response (Pulendran et al. J Immun 2001;167:5067-5076). TLR4 and TLR2 signaling requires the adaptor TIRAP/Mal, which is involved in the MyD88-dependent pathway (Horng et al. Nature 2002;420:329-33). TLR3 triggers the production of IFNß in response to double-stranded RNA, in a MyD88-independent manner. This response is mediated by the adaptor TRIF/TICAM-1 (Yamamoto et al. J Immunol. 2002;169:6668-72). TRAM/TICAM2 is another adaptor molecule involved in the MyD88-independent pathway (Miyake. Int Immunopharmacol. 2003;3:119-28) which function is restricted to the TLR4 pathway (Yamamoto et al. Nat Immunol. 2003;4:1144-50).

Thus, different TLR "switches" turn on different immune response "circuits", where activation of a particular TLR determines the type of antigen-specific response that is triggered. Depending upon the cell type exposed to a PAMP and the particular TLR that binds to that PAMP, the profile of cytokines produced and secreted can vary. This variation in TLR signaling response can influence, for example, whether the resultant adaptive immune response will be predominantly T-cell- or B-cell-mediated, as well as the degree of inflammation accompanying the response.

As discussed above, the innate immune system plays a crucial role in the control of initiation of the adaptive immune response and in the induction of appropriate cell effector responses. Recent evidence demonstrates that fusing a polypeptide ligand specific for a Toll-like Receptor (TLR) to an antigen of interest generates a vaccine that is more potent and selective than the antigen alone. The inventors have previously shown that immunization with recombinant TLR-ligand:antigen fusion proteins: a) induces antigen-specific T-cell and B-cell responses comparable to those induced by the use of conventional adjuvant, b) results in significantly reduced non-specific inflammation; and c) results in CD8 T-cell-mediated protection that is specific for the fused antigen epitopes (see, for example, US published patent applications 2002/0061312 and 2003/0232055 to Medzhitov, and US published patent application 2003/0175287 to Medzhitov and Kopp, all incorporated herein by reference). Mice immunized with a fusion protein consisting of the polypeptide PAMP BLP linked to *Leishmania major* antigens mounted a Type 1 immune response characterized by antigen-induced production of γ-interferon and antigen-specific IgG₂ₐ (Cote-Sierra et al. Infect Immun 2002;70:240-248). The response was protective, as demonstrated by experiments in which immunized mice developed smaller lesions than control mice did following challenge with live *L. major*.

Thus, the binding of PAMPs to TLRs activates immune pathways that can be mobilized for the development of more potent vaccines. Ideally, a vaccine design should ensure that every cell that is exposed to pathogen-derived antigen also receives a TLR receptor innate immune signal and *vice versa*. This can be effectively achieved by designing the vaccine to contain a chimeric macromolecule of antigen plus PAMP, *e*.*g*., a fusion protein of PAMP and antigen(s). Such molecules trigger signal transduction pathways in their target cells that result in the display of co-stimulatory molecules on the cell surface, as well as antigenic peptide in the context of major histocompatability complex molecules.

Although polypeptide ligands to some TLRs are known (see Figure 1), a need exists in the art for the identification of additional TLR-ligands. In particular, the need exists for the identification of polypeptide ligands specific for individual TLR receptors, which can be used to specifically tune the innate immune system response. Such TLR-specific polypeptide ligands can be incorporated into TLR-ligand:antigen conjugate vaccines, whereby the TLR-ligand will provide for an enhanced antigen-specific immune response as regulated by signaling through a particular TLR.

The present invention relates to novel polypeptide ligands for Toll-like Receptor 2 (TLR2). Preferrable, these novel polypeptide TLR2 ligands modulate TLR2 signaling. These polypeptide TLR2 ligands may be incorporated into novel polypeptide TLR2ligand:antigen vaccines.

Phage display is a selection technique in which a peptide or protein is genetically fused to a coat protein of a bacteriophage (Smith. Science 1985;228:1315-1317). The fusion protein is displayed on the exterior of the phage virion, while the DNA encoding the fusion protein resides within the virion. This physical linkage between the displayed protein and the DNA encoding it allows screening of vast numbers of variants of the protein by a simple *in vitro* selection procedure termed "biopanning". Phage display technology offers a very powerful tool for the isolation of new ligands from large collections of potential ligands including short peptides, antibody fragments and randomly modified physiological ligands to receptors (Scott and Smith. Science 1990;249:386-390; Smith and Scott. Meth Enz 1993;217:228-257; and Smith and Petrenko. Chem Rev 1997;97:391-410). These systems have been effectively employed in studies of structural and functional aspects of receptor-ligand interactions using either purified receptors immobilized on a polymer surface (Smith and Petrenko. Chem Rev 1997;97:391-410), or the receptors in their natural environment on the surface of living cells (Fong. et al. Drug Dev Res 199433:64-70; Doorbar and Winter. J Mol Biol 1994;244:361369; Goodson et al. Proc Natl Acad Sci USA 1994;91:7129-7133; and Szardenings et al. J Biol Chem 1997;272:27943-27948).

Cationic antimicrobial peptides (CAMPs) are relatively small (∼20-50 amino acids), cationic and amphipathic peptides of variable length, sequence and structure. These peptides contain a high percentage (20 to 60%) of the positively charged amino acids histidine, lysine and/or arginine. Several hundred CAMPs have been isolated from a wide variety of animals (both vertebrates and invertebrates), plants, bacteria and fungi. These peptides have been obtained from many different cellular sources, *e*.*g*. macrophages, neutrophils, epithelial cells, haemocytes, fat bodies, and the reproductive tract. CAMPs form part of the innate immune response of a wide variety of animal species, including insects, amphibians and mammals. In humans CAMPs, such as defensins, cathelicidins and thrombocidins, protect the skin and epithelia against invading microorganisms and assist neutrophils and platelets in host defense.

To our knowledge, none of the reported CAMPs is a ligand for a TLR. In particular, none of the CAMPs is known to be a ligand for TLR2.

### SUMMARY OF THE INVENTION

The invention is directed to a polypeptide TLR2 ligand comprising at least one amino acid sequence selected from the group consisting of:

| | |
|---|---|
| NPPTT | (SEQ ID NO: 54), |
| MRRIL | (SEQ ID NO: 55), |
| MISS | (SEQ ID NO: 56), |
| RGGSK | (SEQ ID NO: 57), |
| RGGF | (SEQ ID NO: 58), |
| NRTVF | (SEQ ID NO: 59), |
| NRFGL | (SEQ ID NO: 60), |
| SRHGR | (SEQ ID NO: 61), |
| IMRHP | (SEQ ID NO: 62), |
| EVCAP | (SEQ ID NO: 63), |
| ACGVY | (SEQ ID NO: 64), |
| CGPKL | (SEQ ID NO: 65), |
| AGCFS | (SEQ ID NO: 66), |
| SGGLF | (SEQ ID NO: 67), |
| AVRLS | (SEQ ID NO: 68), |
| GGKLS | (SEQ ID NO: 69), |
| VSEGV | (SEQ ID NO: 70), |
| KCQSF | (SEQ ID NO: 71), |
| FCGLG | (SEQ ID NO: 72), and |
| PESGV | (SEQ ID NO: 73). |

The invention is further directed to a polypeptide TLR2 ligand comprising at least one amino acid sequence selected from the group consisting of:

| | |
|---|---|
| DPDSG | (SEQ ID NO: 5), |
| IGRFR | (SEQ ID NO: 6), |
| MGTLP | (SEQ ID NO: 7), |
| ADTHQ | (SEQ ID NO: 8), |
| HLLPG | (SEQ ID NO: 9), |
| GPLLH | (SEQ ID NO: 10), |
| NYRRW | (SEQ ID NO: 11), |
| LRQGR | (SEQ ID NO: 12), |
| IMWFP | (SEQ ID NO: 13), |
| RVVAP | (SEQ ID NO: 14), |
| IHVVP | (SEQ ID NO: 15), |
| MFGVP | (SEQ ID NO: 16), |
| CVWLQ | (SEQ ID NO: 17), |
| IYKLA | (SEQ ID NO: 18), |
| KGWF | (SEQ ID NO: 19), |
| KYMPH | (SEQ ID NO: 20), |
| VGKND | (SEQ ID NO: 21), |
| THKPK | (SEQ ID NO: 22), |
| SHIAL | (SEQ ID NO: 23), and |
| AWAGT | (SEQ ID NO: 24), |

with the proviso that the polypeptide TLR2 ligand is not a polypeptide selected from the group consisting of:
flagellin modification protein FlmB of *Caulobacter crescentus*,
Bacterial Type III secretion system protein,
invasin protein of *Salmonella*,
Type 4 fimbrial biogenesis protein (PilX) of *Pseudomonas*,
*Salmonella* SciJ protein,
putative integral membrane protein of *Streptomyces*,
membrane protein of *Pseudomonas*,
adhesin of *Bordetella pertusis*,
peptidase B of *Vibrio cholerae*,
virulence sensor protein of *Bordetella*,
putative integral membrane protein of *Neisseria meningitidis*,
fusion of flagellar biosynthesis proteins FliR and FlhB of *Clostridium*,
outer membrane protein (porin) of *Acinetobacter*,
flagellar biosynthesis protein, FlhF of *Helicobacter*,
ompA related protein of *Xanthomonas*,
omp2a porin of *Brucella*,
putative porin/fimbrial assembly protein (LHrE) of *Salmonella*,
wbdk of *Salmonella*,
Glycosyltransferase involved in LPS biosynthesis, and
*Salmonella* putative permease.

The invention is also directed to a polypeptide TLR2 ligand comprising at least one amino acid sequence of from 20 to 30 amino acids in length, wherein the amino acid sequence comprises at least 30% positively charged amino acids. In preferred embodiments, the amino acid sequence is selected from the group consisting of:

| | |
|---|---|
| KGGVGPVRRSSRLRRTTQPG | (SEQ ID NO: 25), |
| GRRGLCRGCRTRGRIKQLQSAHK | (SEQ ID NO: 26), and |
| RWGYHLRDRKYKGVRSHKGVPR | (SEQ ID NO: 27). |

The invention is further directed to a polypeptide comprising:
i) a polypeptide TLR2 ligand comprising at least one amino acid sequence selected from the group consisting of:

| | |
|---|---|
| NPPTT | (SEQ ID NO: 54), |
| MRRIL | (SEQ ID NO: 55), |
| MISS | (SEQ ID NO: 56), |
| RGGSK | (SEQ ID NO: 57), |
| RGGF | (SEQ ID NO: 58), |
| NRTVF | (SEQ ID NO: 59), |
| NRFGL | (SEQ ID NO: 60), |
| SRHGR | (SEQ ID NO: 61), |
| IMRHP | (SEQ ID NO: 62), |
| EVCAP | (SEQ ID NO: 63), |
| ACGVY | (SEQ ID NO: 64), |
| CGPKL | (SEQ ID NO: 65), |
| AGCFS | (SEQ ID NO: 66), |
| SGGLF | (SEQ ID NO: 67), |
| AVRLS | (SEQ ID NO: 68), |
| GGKLS | (SEQ ID NO: 69), |
| VSEGV | (SEQ ID NO: 70), |
| KCQSF | (SEQ ID NO: 71), |
| FCGLG | (SEQ ID NO: 72), and |
| PESGV | (SEQ ID NO: 73); and. |

ii) at least one antigen.

The invention is further directed to a polypeptide comprising:
i) a polypeptide TLR2 ligand comprising at least one amino acid sequence selected from the group consisting of:

| | |
|---|---|
| DPDSG | (SEQ ID NO: 5), |
| IGRFR | (SEQ ID NO: 6), |
| MGTLP | (SEQ ID NO: 7), |
| ADTHQ | (SEQ ID NO: 8), |
| HLLPG | (SEQ ID NO: 9), |
| GPLLH | (SEQ ID NO: 10), |
| NYRRW | (SEQ ID NO: 11), |
| LRQGR | (SEQ ID NO: 12), |
| IMWFP | (SEQ ID NO: 13), |
| RVVAP | (SEQ ID NO: 14), |
| IHVVP | (SEQ ID NO: 15), |
| MFGVP | (SEQ ID NO: 16), |
| CVWLQ | (SEQ ID NO: 17), |
| IYKLA | (SEQ ID NO: 18), |
| KGWF | (SEQ ID NO: 19), |
| KYMPH | (SEQ ID NO: 20), |
| VGKND | (SEQ ID NO: 21), |
| THKPK | (SEQ ID NO: 22), |
| SHIAL | (SEQ ID NO: 23), and |
| AWAGT | (SEQ ID NO: 24); and |

ii) at least one antigen, wherein if the the at least one antigen is a polypeptide antigen, the polypeptide antigen is heterologous to the polypeptide TLR2 ligand.

The invention is also directed to a polypeptide comprising: i) a polypeptide TLR2 ligand comprising at least one amino acid sequence of from 20 to 30 amino acids in length, wherein the amino acid sequence comprises at least 30% positively charged amino acids; and ii) at least one antigen. In preferred embodiments, the polypeptide TLR2 ligand comprises at least one amino acid sequence selected from the group consisting of:

| | |
|---|---|
| KGGVGPVRRSSRLRRTTQPG | (SEQ ID NO: 25), |
| GRRGLCRGCRTRGRIKQLQSAHK | (SEQ ID NO: 26), and |
| RWGYHLRDRKYKGVRSHKGVPR | (SEQ ID NO: 27). |

In certain embodiments, the antigen is a polypeptide antigen. In certain embodiments, the antigen is a tumor-associated antigen, an allergen-related antigen, or a pathogen-related antigen. In certain embodiments, the pathogen-related antigen is an *Influenza* antigen, a *Listeria monocytogenes* antigen, or a West Nile Virus antigen.

The invention is also directed to vaccine comprising a polypeptide of the invention and a pharmaceutically acceptable carrier.

The invention is further directed to a vaccine comprising:
i) a polypeptide TLR2 ligand comprising at least one amino acid sequence selected from the group consisting of:

| | |
|---|---|
| NPPTT | (SEQ ID NO: 54), |
| MRRIL | (SEQ ID NO: 55), |
| MISS | (SEQ ID NO: 56), |
| RGGSK | (SEQ ID NO: 57), |
| RGGF | (SEQ ID NO: 58), |
| NRTVF | (SEQ ID NO: 59), |
| NRFGL | (SEQ ID NO: 60), |
| SRHGR | (SEQ ID NO: 61), |
| IMRHP | (SEQ ID NO: 62), |
| EVCAP | (SEQ ID NO: 63), |
| ACGVY | (SEQ ID NO: 64), |
| CGPKL | (SEQ ID NO: 65), |
| AGCFS | (SEQ ID NO: 66), |
| SGGLF | (SEQ ID NO: 67), |
| AVRLS | (SEQ ID NO: 68), |
| GGKLS | (SEQ ID NO: 69), |
| VSEGV | (SEQ ID NO: 70), |
| KCQSF | (SEQ ID NO: 71), |
| FCGLG | (SEQ ID NO: 72), and |
| PESGV | (SEQ ID NO: 73); |

ii) at least one antigen; and
iii) a pharmaceutically acceptable carrier.

The invention is also directed to a vaccine comprising:
i) a polypeptide TLR2 ligand comprising at least one amino acid sequence selected from the group consisting of:

| | |
|---|---|
| DPDSG | (SEQ ID NO: 5), |
| IGRFR | (SEQ ID NO: 6), |
| MGTLP | (SEQ ID NO: 7), |
| ADTHQ | (SEQ ID NO: 8), |
| HLLPG | (SEQ ID NO: 9), |
| GPLLH | (SEQ ID NO: 10), |
| NYRRW | (SEQ ID NO: 11), |
| LRQGR | (SEQ ID NO: 12), |
| IMWFP | (SEQ ID NO: 13), |
| RVVAP | (SEQ ID NO: 14), |
| IHVVP | (SEQ ID NO: 15), |
| MFGVP | (SEQ ID NO: 16), |
| CVWLQ | (SEQ ID NO: 17), |
| IYKLA | (SEQ ID NO: 18), |
| KGWF | (SEQ ID NO: 19), |
| KYMPH | (SEQ ID NO: 20), |
| VGKND | (SEQ ID NO: 21), |
| THKPK | (SEQ ID NO: 22), |
| SHIAL | (SEQ ID NO: 23), and |
| AWAGT | (SEQ ID NO: 24); |

ii) at least one antigen; and
iii) a pharmaceutically acceptable carrier, wherein if the at least one antigen is a polypeptide antigen, the polypeptide antigen is heterologous to the polypeptide TLR2 ligand.

The invention is also directed to a vaccine comprising: i) a polypeptide TLR2 ligand comprising at least one amino acid sequence of from 20 to 30 amino acids in length, wherein the amino acid sequence comprises at least 30% positively charged amino acids; ii) at least one antigen; and iii) a pharmaceutically acceptable carrier. In preferred embodiments, the polypeptide TLR2 ligand comprises at least one amino acid sequence selected from the group consisting of:

| | |
|---|---|
| KGGVGPVRRSSRLRRTTQPG | (SEQ ID NO: 25), |
| GRRGLCRGCRTRGRIKQLQSAHK | (SEQ ID NO: 26), and |
| RWGYHLRDRKYKGVRSHKGVPR | (SEQ ID NO: 27). |

In preferred embodiments of such vaccines, the polypeptide TLR2 ligand and the antigen are covalently linked.

In preferred embodiments of such vaccines, the antigen is a polypeptide antigen.

In certain embodiments of such vaccines, the antigen is a tumor-associated antigen, an allergen-related antigen, or a pathogen-related antigen. In certain embodiments, the pathogen-related antigen is an *Influenza* antigen, a *Listeria monocytogenes* antigen, or a West Nile Virus antigen.

The invention is also directed to a method of modulating TLR2 signaling in a subject comprising administering to a subject in need thereof a polypeptide or vaccine of the invention. In preferred embodiments, the subject is a mammal.

The invention is also directed to a method of modulating TLR2 signaling in a cell comprising contacting a cell, wherein the cell comprises TLR2, with a polypeptide of the invention.

The invention is also directed to a method of modulating TLR2 signaling in a cell comprising contacting a cell, wherein the cell comprises TLR2, with a polypeptide TLR2 ligand comprising at least one amino acid sequence selected from the group consisting of:

| | |
|---|---|
| DPDSG | (SEQ ID NO: 5), |
| IGRFR | (SEQ ID NO: 6), |
| MGTLP | (SEQ ID NO: 7), |
| ADTHQ | (SEQ ID NO: 8), |
| HLLPG | (SEQ ID NO: 9), |
| GPLLH | (SEQ ID NO: 10), |
| NYRRW | (SEQ ID NO: 11), |
| LRQGR | (SEQ ID NO: 12), |
| IMWFP | (SEQ ID NO: 13), |
| RVVAP | (SEQ ID NO: 14), |
| IHVVP | (SEQ ID NO: 15), |
| MFGVP | (SEQ ID NO: 16), |
| CVWLQ | (SEQ ID NO: 17), |
| IYKLA | (SEQ ID NO: 18), |
| KGWF | (SEQ ID NO: 19), |
| KYMPH | (SEQ ID NO: 20), |
| VGKND | (SEQ ID NO: 21), |
| THKPK | (SEQ ID NO: 22), |
| SHIAL | (SEQ ID NO: 23), |
| AWAGT | (SEQ ID NO: 24), |
| NPPTT | (SEQ ID NO: 54), |
| MRRIL | (SEQ ID NO: 55), |
| MISS | (SEQ ID NO: 56), |
| RGGSK | (SEQ ID NO: 57), |
| RGGF | (SEQ ID NO: 58), |
| NRTVF | (SEQ ID NO: 59), |
| NRFGL | (SEQ ID NO: 60), |
| SRHGR | (SEQ ID NO: 61), |
| IMRHP | (SEQ ID NO: 62), |
| EVCAP | (SEQ ID NO: 63), |
| ACGVY | (SEQ ID NO: 64), |
| CGPKL | (SEQ ID NO: 65), |
| AGCFS | (SEQ ID NO: 66), |
| SGGLF | (SEQ ID NO: 67), |
| AVRLS | (SEQ ID NO: 68), |
| GGKLS | (SEQ ID NO: 69), |
| VSEGV | (SEQ ID NO: 70), |
| KCQSF | (SEQ ID NO: 71), |
| FCGLG | (SEQ ID NO: 72), and |
| PESGV | (SEQ ID NO: 73). |

The invention is further directed to a method of modulating TLR2 signaling in a cell comprising contacting a cell, wherein the cell comprises TLR2, with a polypeptide TLR2 ligand comprising at least one amino acid sequence of from 20 to 30 amino acids in length, wherein the amino acid sequence comprises at least 30% positively charged amino acids. In preferred embodiments, the polypeptide TLR2 ligand comprises at least one amino acid sequence selected from the group consisting of:

| | |
|---|---|
| KGGVGPVRRSSRLRRTTQPG | (SEQ ID NO: 25), |
| GRRGLCRGCRTRGRIKQLQSAHK | (SEQ ID NO: 26), and |
| RWGYHLRDRKYKGVRSHKGVPR | (SEQ ID NO: 27). |

In preferred embodiments of the method of modulating TLR2 signaling in a cell, the cell is a mammalian cell.

### DESCRIPTION OF THE DRAWINGS

Figure 1 depicts known interactions of PAMPs with various Toll-like Receptors (TLRs). (G+) = Gram-positive. (G-) = Gram-negative.
**Figure 2** is a schematic depicting the steps of the phage display screening assay ("biopanning" assay) strategy for identification of phage displaying polypeptide TLR ligands.
**Figure 3** is a bar graph showing activation of NF-κB-dependent luciferase activity in 293 ("293") and 293.hTLR5 ("293/hTLR5") cells exposed to T7 phage displaying the fliC protein ("Phage", black bar) or to medium alone ("Medium", striped bar); and in 293.hTLR5 cells exposed to T7 phage displaying the S-tag polypeptide. ("S-Tag", "Phage", black bar) or to medium alone ("S-Tag", "Medium", striped bar). "RLU" = relative luciferase units.
**Figure 4** is a bar graph depicting enrichment for TLR5-binding fliC phage using the phage display screening assay ("biopanning" assay). Results are presented as the enrichment percentage (%), calculated as the percentage of input phage recovered after each indicated round of the assay.
**Figure 5** is a bar graph depicting enrichment of TLR2-binding pentapeptide phage using the phage display screening assay ("biopanning" assay). Results are presented as the enrichment percentage (%), calculated as the percentage of input phage recovered after each indicated round of the assay.
**Figure 6** is a Coommassie stained SDS-PAGE gel of Ni-NTA purified recombinant polypeptide TLR2 ligands. Lane M = molecular weight markers. Lane 1 = recombinant protein ID#1. Lane 2 = recombinant protein ID#2. Lane 3 = recombinant protein ID#3.
**Figure 7** is a bar graph depicting induction of IL-8 (in pg/mL) secretion from 293 (black bar) and 293.hTLR2.hCD14 (white bar) cells exposed to Ni-NTA purified recombinant polypeptide TLR2 ligands or Pam₃Cys. Pam3 = Pam₃Cys positive control. ID#1 = recombinant protein ID#1. ID#2 = recombinant protein ID#2. ID#3 = recombinant protein ID#3. Left panel includes the Pam₃Cys control, whereas the right panel shows only the Ni-NTA purified recombinant polypeptide TLR2 ligands.
**Figure 8** depicts a schematic of exemplary plasmid vector T7.LIST. T7.LIST is designed to express recombinant LLO-p60 (SEQ ID NO: 39) protein with a V5 epitope (SEQ ID NO: 40) and a polyhistidine tag (6xHis). T7 = T7 promoter. rbs = ribosome binding site.
**Figure 9** depicts the amino acid sequence of human TLR2 (SEQ ID NO: 4).

### DETAILED DESCRIPTION

The present invention provides novel polypeptide ligands for Toll-like Receptor 2 (TLR2). In preferred embodments, the novel polypeptide ligands modulate TLR2 signaling and thereby regulate the Innate Immune Response. The polypeptide ligands of the invention will find utility in a variety of applications. For example, the invention also provides vaccines comprising the novel polypeptide TLR2 ligands and an antigen. The invention further provides methods of modulating TLR2 signaling using the polypeptide ligands or vaccines of the invention.

### Novel polypeptide ligands for TLR2

As used herein, the term "Toll-like Receptor" or "TLR" refers to any of a family of pattern recognition receptor (PRR) proteins that are homologous to the *Drosophila melanogaster* Toll protein. TLRs are type I transmembrane signaling receptor proteins that are characterized by an extracellular leucine-rich repeat domain and an intracellular domain homologous to that of the interleukin 1 receptor. The TLR family includes, but is not limited to, mammalian TLRs 1 through 11 and 13, including mouse and human TLRs 1-11 and 13.

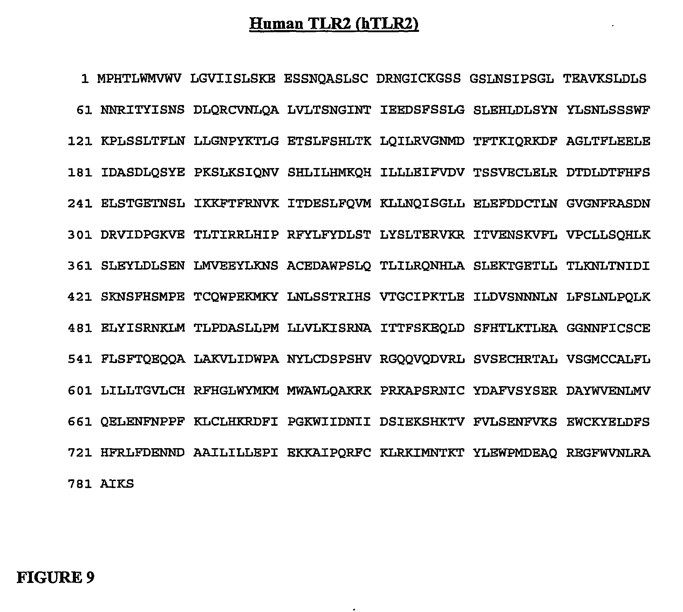

Toll-like receptor 2 (TLR2) is involved in the recognition of, *e*.*g*., multiple products of Gram-positive bacteria, mycobacteria and yeast, including LPS and lipoproteins. TLR2 is known to heterodimerize with other TLRs, a property believed to extend the range of PAMPs that TLR2 can recognize. For example, TLR2 cooperates with TLR6 in the response to peptidoglycan and diacylated mycoplasmal lipopeptide, and associates with TLR1 to recognize triacylated lipopeptides. Pathogen recognition by TLR2 is strongly enhanced by CD14. The nucleotide and amino acid sequence for TLR2 has been reported for a variety of species, including, mouse, human, Rhesus monkey, rat, zebrafish, dog, pig and chicken. The nucleotide and amino acids sequences of mouse TLR2 are set forth in SEQ ID NOs: 1 and 2, respectively. The nucleotide and amino acid sequences of human TLR2 are set forth in SEQ ID NOs: 3 and 4, respectively. The amino acid sequence of human TLR2 is shown in Figure 9 (SEQ ID NO: 4). In preferred embodiments, TLR2 is a mammalian TLR2. In particularly preferred embodiments, TLR2 is mouse TLR2 (mTLR2) or human TLR2 (hTLR2).

The invention provides novel polypeptide ligands for Toll-like Receptor 2 (TLR2), which modulate TLR2 signaling and thereby regulate the Innate Immune Response. The terms "polypeptide ligand for TLR2" and "polypeptide TLR2 ligand" are used interchangeably herein.

By the term "polypeptide TLR2 ligand" is meant a polypeptide that binds to the extracellular portion of a TLR2 protein. For example, in context of the present invention, novel polypeptide TLR2 ligands were identified based upon their ability to bind to the extracellular domain of a TLR2 protein in a phage display-based "biopanning" assay. In preferred embodiments, the polypeptide TLR2 ligands of the invention are functional TLR2 ligands, *i*.*e*. they modulate TLR2 signaling. As used herein, the term "TLR2 signaling" refers to any intracellular signaling pathway initiated by activated TLR2, including shared pathways (*e*.*g*., activation of NF-κB) and TLR2-specific pathways. As used herein the term "modulating TLR2 signaling" includes both activating (*i*.*e*. agonizing) TLR2 signaling and suppressing (*i*.*e*. antagonizing) TLR2 signaling. Thus, a polypeptide TLR2 ligand that modulates TLR2 signaling agonizes or antagonizes TLR2 signaling.

As used herein, the term "polypeptide" or "protein" refers to a polymer of amino acid monomers that are alpha amino acids joined together through amide bonds. The terms "polypeptide" and "protein" are used interchangeably herein. Polypeptides are therefore at least two amino acid residues in length, and are usually longer. Generally, the term "peptide" refers to a polypeptide that is only a few amino acid residues in length, *e*.*g*. from three to 50 amino acid residues. A polypeptide, in contrast with a peptide, may comprise any number of amino acid residues. Hence, the term polypeptide includes peptides as well as longer sequences of amino acids.

As used herein, the term "positively charged amino acid" refers to an amino acid selected from the group consisting of lysine (Lys or K), arginine (Arg or R), and Histidine (His or H). The percent (%) positively charged amino acids of a polypeptide is calculated as (Total number of K + R + H amino acids of polypeptide)/(Total amino acid length of polypeptide).

Amino acid residues are abbreviated as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; and Glycine is Gly or G.

In one embodiment, the polypeptide TLR2 ligands of the invention comprise at least one peptide, wherein the peptide is selected from the peptides set forth in Table 1.

**Table 1: Novel peptide ligands for TLR2**

| **PEPTIDE** | **SEQ ID NO** | **HOMOLOGY** |
|---|---|---|
| DPDSG | 5 | flagellin modification protein FlmB of *Caulobacter crescentus* |
| IGRFR | 6 | Bacterial Type III secretion system protein |
| MGTLP | 7 | invasin protein of *Salmonella* |
| ADTHQ | 8 | Type 4 fimbrial biogenesis protein (PilX) of *Pseudomonas* |
| HLLPG | 9 | *Salmonella* SciJ protein |
| GPLLH | 10 | putative integral membrane protein of *Streptomyces* |
| NYRRW | 11 | membrane protein of *Pseudomonas* |
| LRQGR | 12 | adhesin of *Bordetella pertusis* |
| IMWFP | 13 | peptidase B of *Vibrio cholerae* |
| RVVAP | 14 | virulence sensor protein of *Bordetella* |
| IHVVP | 15 | putative integral membrane protein of *Neisseria meningitidis* |
| MFGVP | 16 | fusion of flagellar biosynthesis proteins FliR and FlhB of *Clostridium* |
| CVWLQ | 17 | outer membrane protein (porin) of *Acinetobacter* |
| IYKLA | 18 | flagellar biosynthesis protein, FlhF of *Helicobacter* |
| KGWF | 19 | ompA related protein of *Xanthomonas* |
| KYMPH | 20 | omp2a porin of *Brucella* |
| VGKND | 21 | putative porin/fimbrial assembly protein (LHrE) of *Salmonella* |
| THKPK | 22 | wbdk of *Salmonella* |
| SHIAL | 23 | Glycosyltransferase involved in LPS biosynthesis |
| AWAGT | 24 | *Salmonella* putative permease |

In some embodiments, the polypeptide TLR2 ligands of the invention comprise at least one of the peptide sequences set forth in Table 1 within the context of a longer polypeptide. For example, the polypeptide TLR2 ligands of the invention may comprise a peptide sequence as set forth in Table 1 and additional polypeptide sequences attached to the N-terminus, the C-terminus, or both the N- and C- termini of the peptide sequence. In such embodiments, the additional polypeptide sequences are preferably heterologous to the peptide sequence, *i*.*e*., they are not sequences which are endogenously associated with the given peptide sequence. By "endogenously associated" is meant that the given peptide sequence and the additional polypeptide sequence may be found contiguously linked in N-terminal to C-terminal amino acid sequence orientation within a naturally occurring protein. However, embodiments wherein the polypeptide TLR2 ligand comprises at least one of the peptide sequences set forth in Table 1 and additional polypeptide sequences, where the additional polypeptide sequences are sequences which are endogenously associated with said peptide sequence, are also contemplated.

In another embodiment, the polypeptide TLR2 ligands of the invention comprise at least one peptide, wherein the peptide is selected from the peptides set forth in Table 2.

**Table 2: Novel peptide ligands for TLR2**

| **PEPTIDE** | **SEQ ID NO** |
|---|---|
| NPPTT | 54 |
| MRRIL | 55 |
| MISS | 56 |
| RGGSK | 57 |
| RGGF | 58 |
| NRTVF | 59 |
| NRFGL | 60 |
| SRHGR | 61 |
| IMRHP | 62 |
| EVCAP | 63 |
| ACGVY | 64 |
| CGPKL | 65 |
| AGCFS | 66 |
| SGGLF | 67 |
| AVRLS | 68 |
| GGKLS | 69 |
| VSEGV | 70 |
| KCQSF | 71 |
| FCGLG | 72 |
| PESGV | 73 |

In some embodiments, the polypeptide TLR2 ligands of the invention comprise at least one of the peptide sequences set forth in Table 2 within the context of a longer polypeptide. For example, the polypeptide TLR2 ligands of the invention may comprise a peptide sequence as set forth in Table 2 and additional polypeptide sequences attached to the N-terminus, the C-terminus, or both the N- and C- termini of the peptide sequence. In such embodiments, the additional polypeptide sequences are preferably heterologous to the peptide sequence, *i*.*e*., they are not sequences which are endogenously associated with the given peptide sequence. However, embodiments wherein the polypeptide TLR2 ligand comprises at least one of the peptide sequences set forth in Table 2 and additional polypeptide sequences, where the additional polypeptide sequences are sequences which are endogenously associated with said peptide sequence, are also contemplated.

In another embodiment, the polypeptide TLR2 ligands of the invention comprise at least one peptide of 20 amino acids to 30 amino acids in length, wherein the peptide comprises at least 30% positively charged amino acids. In preferred embodiments, the polypeptide TLR2 ligands of the invention comprise at least one peptide selected from the peptides set forth in Table 3.

**Table 3: Novel peptide ligands for TLR2**

| **PEPTIDE** | **SEQ ID NO** | **POSITIVELY CHARGED AA (%)** |
|---|---|---|
| KGGVGPVRRSSRLRRTTQPG | 25 | 6/20 (30%) |
| GRRGLCRGCRTRGRIKQLQSAHK | 26 | 9/23 (39%) |
| RWGYHLRDRKYKGVRSHKGVPR | 27 | 10/22 (45%) |

According to some embodiments of the invention, two or more amino acid residues, independently selected from any of the 20 genetically encoded L-amino acids or the stereoisomeric D-amino acids, may be coupled to either or both ends of the polypeptide TLR2 ligands described above. For example, the sequence GG may be appended to either terminus or both termini of a polypeptide TLR2 ligand.

Polypeptide TLR2 ligands comprising sequence variants of the polypeptide sequences set forth in Tables 1, 2 and 3 are also contemplated. Such sequence variants include conservative variants of the polypeptide TLR2 ligands in which amino acids have been substituted for one another within one of the following groups: small aliphatic, nonpolar or slightly polar residues (Ala, Ser, Thr, Pro and Gly); polar, negatively charged residues and their amides (Asp, Asn, Glu and Gln); polar, positively charged residues (His, Arg and Lys); large aliphatic, nonpolar residues (Met, Leu, Ile, Val and Cys); and aromatic residues (Phe, Tyr and Trp). The types of substitutions selected may be based, for example, on analyses of structure-forming potentials (see, for example, Chou et al. Biochemistry 1974;13:211 and Schulz et al. Principles in Protein Structure. Springer Verlag: 1978. pp. 108-130), and on the analysis of hydrophobicity patterns in proteins (see, for example, Kyte et al. J. Mol. Biol. 1982;157:105-132). Such sequence variants may also include polypeptide TLR2 ligands with altered overall charge, structure, hydrophobicity/hydrophilicity properties produced by amino acid substitution, insertion, or deletion that retain and/or improve the ability to modulate TLR2 signaling.

Stereoisomers (*e*.*g*., D-amino acids) of the twenty conventional amino acids, unnatural amino acids such as a,a-disubstituted amino acids, N-alkyl amino acids, lactic acid, and other unconventional amino acids may also be suitable components for polypeptide TLR2 ligands of the present invention. Examples of unconventional amino acids include, but are not limited to: β-alanine, 3-pyridylalanine, 4-hydroxyproline, O-phosphoserine, N-methylglycine (also known and sarcosine), N-acetylserine, N-formylmethionine, 3-methylhistidine, 5-hydroxylysine, nor-leucine, 1-naphthylalanine (1-nal), 2-naphthylalanine (2-nal), homoserine methylether (Hsm), N-acetylglycine, and other similar amino acids and imino acids.

Other modifications are also possible, including modification of the amino terminus, modification of the carboxy terminus, replacement of one or more of the naturally occurring genetically encoded amino acids with an unconventional amino acid, modification of the side chain of one or more amino acid residues, peptide phosphorylation, and the like. For example, the amino terminus of the peptide may be modified by acetylation (*e*.*g*., with acetic acid or a halogen substituted acetic acid). See also the section "Preparation of the polypeptide TLR2 ligands of the invention: ***Polypeptide modifications*",** below.

### Preparation of the polypeptide TLR2 ligands of the invention:

The polypeptide TLR2 ligands of the invention may be prepared by any of the techniques well known in the art, including translation from coding sequences and *in vitro* chemical synthesis.

### Translation from coding sequences

In one embodiment, the polypeptide TLR2 ligands of the invention may be prepared by translation of a nucleic acid sequence encoding the polypeptide TLR2 ligand. Such nucleic acids may be obtained by any of the synthetic or recombinant DNA methods well known in the art. See, for example, DNA Cloning: A Practical Approach, Vol I and II (Glover ed.:1985); Oligonucleotide Synthesis (Gait ed.:1984); Transcription And Translation (Hames & Higgins, eds.:1984); Perbal. A Practical Guide To Molecular Cloning (1984); Ausubel et al., eds. Current Protocols in Molecular Biology, (John Wiley & Sons, Inc.:1994); PCR Primer: A Laboratory Manual, 2nd Edition. Dieffenbach and Dveksler, eds. (Cold Spring Harbor Laboratory Press: 2003); and Sambrook et al. Molecular Cloning: A Laboratory Manual, 3rd Edition (Cold Spring Harbor Laboratory Press: 2001). For example, nucleic acids encoding a polypeptide TLR2 ligand (*e*.*g*., synthetic oligo and polynucleotides) can easily be synthesized by chemical techniques, for example, the phosphotriester method (see, for example, Matteucci et al. J. Am. Chem. Soc. 1981;103:3185-3191) or using automated synthesis methods.

Translation of the polypeptide TLR2 ligands of the invention may be achieved *in vitro* (*e*.*g*. via *in vitro* translation of a linear nucleic acid encoding the polypeptide TLR2 ligand) or *in vivo* (*e*.*g*. by recombinant expression of an expression construct encoding the polypeptide TLR2 ligand). Techniques for *in vitro* and *in vivo* expression of peptides from a coding sequence are well known in the art. See, for example, DNA Cloning: A Practical Approach, Vol I and II (Glover ed.:1985); Oligonucleotide Synthesis (Gait ed.:1984); Transcription And Translation (Hames & Higgins, eds.:1984); Animal Cell Culture (Freshney, ed.:1986); Perbal, A Practical Guide To Molecular Cloning (1984); Ausubel et al., eds. Current Protocols in Molecular Biology, (John Wiley & Sons, Inc.:1994); and Sambrook et al. Molecular Cloning: A Laboratory Manual, 3rd Edition (Cold Spring Harbor Laboratory Press: 2001).

In one embodiment, the polypeptide TLR2 ligands of the invention are prepared by *in vitro* translation of a nucleic acid encoding the polypeptide TLR2 ligand. A number of cell-free translation systems have been developed for the translation of isolated mRNA, including rabbit reticulocyte lysate, wheat germ extract, and *E. coli* S30 extract systems (Jackson and Hunt. Meth Enz 1983;96:50-74; Ambion Technical Bulletin #187; and Hurst. Promega Notes 1996;58:8). Kits for *in vitro* transcription and translation are available from a wide variety of commercial sources including Promega, Ambion, Roche Applied Science, Novagen, Invitrogen, PanVera, and Qiagen. For example, kits for *in vitro* translation using reticulocyte or wheat germ lysates are commercially available from Ambion. For example, using the rabbit reticulocyte lysate system, reticulocyte lysate is programmed with PCR DNA using a TNT T7 Quick for PCR DNA kit (Promega), which couples transcription to translation. To initiate a TNT reaction, the DNA template is incubated at 30°C for 60-90 min in the presence of rabbit reticulocyte lysate, RNA polymerase, an amino acid mixture and RNAsin ribonuclease inhibitor.

In another embodiment, the polypeptide TLR2 ligands are translated from an expression construct, wherein a nucleic acid encoding the polypeptide TLR2 ligand is operatively associated with expression control sequence elements which provide for the proper transcription and translation of the polypeptide TLR2 ligand within the chosen host cells. Such sequence elements may include a promoter, a polyadenylation signal, and optionally internal ribosome entry sites (IRES) and other ribosome binding site sequences, enhancers, response elements, suppressors, signal sequences, and the like. Codon selection, where the target nucleic acid sequence of the construct is engineered or chosen so as to contain codons preferentially used within the desired host call, may be used to minimize premature translation termination and thereby maximize expression.

The nucleic acid sequence may also encode a peptide tag for easy identification and purification of the translated polypeptide TLR2 ligand. Preferred peptide tags include GST, myc, His, and FLAG tags. The encoded peptide tag may include recognition sites for site-specific proteolysis or chemical agent cleavage to facilitate removal of the peptide tag following protein purification. For example a thrombin cleavage site could be incorporated between a polypeptide TLR2 ligand and its peptide tag.

The promoter sequences may be endogenous or heterologous to the host cell to be modified, and may provide ubiquitous (*i*.*e*., expression occurs in the absence of an apparent external stimulus) or inducible (*i*.*e*., expression only occurs in presence of particular stimuli) expression. Promoters that may be used to control gene expression include, but are not limited to, cytomegalovirus (CMV) promoter (U.S. Patents No. 5,385,839 and No. 5,168,062), the SV40 early promoter region (Benoist and Chambon. Nature 1981;290:304-310), the promoter contained in the 3' long terminal repeat of Rous sarcoma virus (Yamamoto et al. Cell 1980;22:787-797), the herpes thymidine kinase promoter (Wagner et al. Proc. Natl. Acad. Sci. USA 1981;78:1441-1445), the regulatory sequences of the metallothionein gene (Brinster et al. Nature 1982;296:39-42); prokaryotic promoters such as the alkaline phosphatase promoter, the trp-lac promoter, the bacteriophage lambda P_{L} promoter, the T7 promoter, the beta-lactamase promoter (Villa-Komaroff et al. Proc. Natl. Acad. Sci. USA 1978;75:3727-3731), or the tac promoter (DeBoer et al. Proc. Natl. Acad Sci. USA 1983;80:21-25); and promoter elements from yeast or other fungi such as the Ga14 promoter, the ADC (alcohol dehydrogenase) promoter, and the PGK (phosphoglycerol kinase) promoter.

The expression constructs may further comprise vector sequences that facilitate the cloning and propagation of the expression constructs. A large number of vectors, including plasmid and fungal vectors, have been described for replication and/or expression in a variety of eukaryotic and prokaryotic host cells. Standard vectors useful in the current invention are well known in the art and include (but are not limited to) plasmids, cosmids, phage vectors, viral vectors, and yeast artificial chromosomes. The vector sequences may contain, for example, a replication origin for propagation in *E. coli*; the SV40 origin of replication; an ampicillin, neomycin, or puromycin resistance gene for selection in host cells; and/or genes (*e*.*g*., dihydrofolate reductase gene) that amplify the dominant selectable marker plus the nucleic acid of interest. For example, a plasmid is a common type of vector. A plasmid is generally a self-contained molecule of double-stranded DNA, usually of bacterial origin, that can readily accept additional foreign DNA and that can readily be introduced into a suitable host cell. A plasmid vector generally has one or more unique restriction sites suitable for inserting foreign DNA. Examples of plasmids that may be used for expression in prokaryotic cells include, but are not limited to, pBR322-derived plasmids, pEMBL-derived plasmids, pEX-derived plasmids, pBTac-derived plasmids, pUC-derived plasmids, and pET-LIC-derived plasmids.

Techniques for introduction of nucleic acids to host cells are well established in the art, including, but not limited to, electroporation, microinjection, liposome-mediated transfection, calcium phosphate-mediated transfection, or virus-mediated transfection. See, for example, Felgner et al., eds. Artificial self-assembling systems for gene delivery. Oxford University Press:1996; Lebkowski et al. Mol Cell Biol 1988;8:3988-3996; Sambrook et al. Molecular Cloning: A Laboratory Manual. 2nd Edition (Cold Spring Harbor Laboratory:1989); and Ausubel et al., eds. Current Protocols in Molecular Biology (John Wiley & Sons: 1989).

Expression constructs encoding polypeptide TLR2 ligands may be transfected into host cells *in vitro*. Exemplary host cells include various strains of *E. coli*, yeast, *Drosophila* cells (*e*.*g*. S-2 cells), and mammalian cells. Preferred *in vitro* host cells are mammalian cell lines including BHK-21, MDCK, Hu609, MAC-T (U.S. Patent No. 5,227,301), R1 embryonic stem cells, embryonal carcinoma cells, COS, or HeLa cells. Protocols for *in vitro* culture of mammalian cells are wells established in the art. See, for example, Animal Cell Culture: A Practical Approach 3rd Edition. J. Masters, ed. (Oxford University Press: 2000) and Basic Cell Culture 2nd Edition. Davis, ed. (Oxford University Press:2002).

### In vitro chemical synthesis

The polypeptide TLR2 ligands of the invention may be prepared via *in vitro* chemical synthesis by classical methods known in the art. These standard methods include exclusive solid phase synthesis, partial solid phase synthesis, fragment condensation, and classical solution synthesis methods (see, *e*.*g*., Merrifield. J. Am. Chem. Soc. 1963;85:2149).

A preferred method for polypeptide synthesis is solid phase synthesis. Solid phase polypeptide synthesis procedures are well-known in the art. See, *e*.*g*., Stewart Solid Phase Peptide Syntheses (Freeman and Co.: San Francisco:1969); 2002/2003 General Catalog from Novabiochem Corp, San Diego, USA; and Goodman Synthesis of Peptides and Peptidomimetics (Houben-Weyl, Stuttgart:2002). In solid phase synthesis, synthesis is typically commenced from the C-terminal end of the polypeptide using an α-amino protected resin. A suitable starting material can be prepared, for example, by attaching the required α-amino acid to a chloromethylated resin, a hydroxymethyl resin, a polystyrene resin, a benzhydrylamine resin, or the like. One such chloromethylated resin is sold under the trade name BIO-BEADS SX-1 by Bio Rad Laboratories (Richmond, CA). The preparation of a hydroxymethyl resin has been described (see, for example, Bodonszky et al. Chem. Ind. London 1966;38:1597). A benzhydrylamine (BHA) resin has been described (see, for example, Pietta and Marshall. Chem. Commun. 1970;650), and a hydrochloride form is commercially available from Beckman Instruments, Inc. (Palo Alto, CA). For example, an α-amino protected amino acid may be coupled to a chloromethylated resin with the aid of a cesium bicarbonate catalyst (see, for example, Gisin. *Helv. Chim. Acta* 1973;56:1467).

After initial coupling, the α-amino protecting group is removed, for example, using trifluoroacetic acid (TFA) or hydrochloric acid (HCl) solutions in organic solvents at room temperature. Thereafter, α-amino protected amino acids are successively coupled to a growing support-bound polypeptide chain. The α-amino protecting groups are those known to be useful in the art of stepwise synthesis of polypeptides, including: acyl-type protecting groups (*e*.*g*., formyl, trifluoroacetyl, acetyl), aromatic urethane-type protecting groups [*e*.*g*., benzyloxycarboyl (Cbz) and substituted Cbz], aliphatic urethane protecting groups [*e*.*g*., t-butyloxycarbonyl (Boc), isopropyloxycarbonyl, cyclohexyloxycarbonyl], and alkyl type protecting groups (*e*.*g*., benzyl, triphenylmethyl), fluorenylmethyl oxycarbonyl (Fmoc), allyloxycarbonyl (Alloc), and 1-(4,4-dimethyl-2,6-dioxocyclohex-1-ylidene)ethyl (Dde).

The side chain protecting groups (typically ethers, esters, trityl, PMC, and the like) remain intact during coupling and are not split off during the deprotection of the amino-terminus protecting group or during coupling. The side chain protecting group must be removable upon the completion of the synthesis of the final polypeptide and under reaction conditions that will not alter the target polypeptide. The side chain protecting groups for Tyr include tetrahydropyranyl, tert-butyl, trityl, benzyl, Cbz, Z-Br-Cbz, and 2,5-dichlorobenzyl. The side chain protecting groups for Asp include benzyl, 2,6-dichlorobenzyl, methyl, ethyl, and cyclohexyl. The side chain protecting groups for Thr and Ser include acetyl, benzoyl, trityl, tetrahydropyranyl, benzyl, 2,6-dichlorobenzyl, and Cbz. The side chain protecting groups for Arg include nitro, Tosyl (Tos), Cbz, adamantyloxycarbonyl mesitoylsulfonyl (Mts), 2,2,4,6,7-pentamethyldihydrobenzofurane-5-sulfonyl (Pbf), 4-methoxy-2,3,6-trimethyl-benzenesulfonyl (Mtr), or Boc. The side chain protecting groups for Lys include Cbz, 2-chlorobenzyloxycarbonyl (2-Cl-Cbz), 2-bromobenzyloxycarbonyl (2-Br-Cbz), Tos, or Boc.

After removal of the α-amino protecting group, the remaining protected amino acids are coupled stepwise in the desired order. Each protected amino acid is generally reacted in about a 3-fold excess using an appropriate carboxyl group activator such as 2-(1H-benzotriazol-1-yl)-1,1,3,3 tetramethyluronium hexafluorophosphate (HBTU) or dicyclohexylcarbodimide (DCC) in solution, for example, in methylene chloride (CH₂Cl₂), N-methyl pyrrolidone, dimethyl formamide (DMF), or mixtures thereof.

After the desired amino acid sequence has been completed, the desired polypeptide is decoupled from the resin support by treatment with a reagent, such as trifluoroacetic acid (TFA) or hydrogen fluoride (HF), which not only cleaves the polypeptide from the resin, but also cleaves all remaining side chain protecting groups. When a chloromethylated resin is used, hydrogen fluoride treatment results in the formation of the free peptide acids. When a benzhydrylamine resin is used, hydrogen fluoride treatment results directly in the free peptide amide. Alternatively, when a chloromethylated resin is employed, the side chain protected polypeptide can be decoupled by treatment of the polypeptide resin with ammonia to give the desired side chain protected amide or with an alkylamine to give a side chain protected alkylamide or dialkylamide. Side chain protection is then removed in the usual fashion by treatment with hydrogen fluoride to give the free amides, alkylamides, or dialkylamides. In preparing esters, the resins used to prepare the peptide acids are employed, and the side chain protected polypeptide is cleaved with base and the appropriate alcohol (*e*.*g*., methanol). Side chain protecting groups are then removed in the usual fashion by treatment with hydrogen fluoride to obtain the desired ester.

These procedures can also be used to synthesize polypeptides in which amino acids other than the 20 naturally occurring, genetically encoded amino acids are substituted at one, two, or more positions of any of the compounds of the invention. Synthetic amino acids that can be substituted into the polypeptides of the present invention include, but are not limited to, N-methyl, L-hydroxypropyl, L-3, 4-dihydroxyphenylalanyl, δ amino acids such as L- δ-hydroxylysyl and D- δ-methylalanyl, L-α-methylalanyl, β amino acids, and isoquinolyl. D-amino acids and non-naturally occurring synthetic amino acids can also be incorporated into the polypeptides of the present invention.

### Polypeptide modifications

One can also modify the amino and/or carboxy termini of the polypeptide TLR ligands of the invention. Amino terminus modifications include methylation (*e*.*g*., --NHCH₃ or --N(CH₃)₂), acetylation (*e*.*g*., with acetic acid or a halogenated derivative thereof such as α-chloroacetic acid, α-bromoacetic acid, or α-iodoacetic acid), adding a benzyloxycarbonyl (Cbz) group, or blocking the amino terminus with any blocking group containing a carboxylate functionality defined by RCOO-- or sulfonyl functionality defined by R--SO₂--, where R is selected from alkyl, aryl, heteroaryl, alkyl aryl, and the like, and similar groups. One can also incorporate a desamino acid at the N-terminus (so that there is no N-terminal amino group) to decrease susceptibility to proteases or to restrict the conformation of the polypeptide compound. For example, the N-terminus may be acetylated to yield N-acetylglycine.

Carboxy terminus modifications include replacing the free acid with a carboxamide group or forming a cyclic lactam at the carboxy terminus to introduce structural constraints. One can also cyclize the polypeptides of the invention, or incorporate a desamino or descarboxy residue at the termini of the polypeptide, so that there is no terminal amino or carboxyl group, to decrease susceptibility to proteases or to restrict the conformation of the polypeptide. C-terminal functional groups of the compounds of the present invention include amide, amide lower alkyl, amide di(lower alkyl), lower alkoxy, hydroxy, and carboxy, and the lower ester derivatives thereof, and the pharmaceutically acceptable salts thereof.

One can replace the naturally occurring side chains of the 20 genetically encoded amino acids (or the stereoisomeric D amino acids) with other side chains, for instance with groups such as alkyl, lower alkyl, cyclic 4-, 5-, 6-, to 7-membered alkyl, amide, amide lower alkyl, amide di(lower alkyl), lower alkoxy, hydroxy, carboxy and the lower ester derivatives thereof, or 4-, 5-, 6-, to 7-membered heterocyclic. In particular, proline analogues in which the ring size of the proline residue is changed from 5 members to 4, 6, or 7 members can be employed. Cyclic groups can be saturated or unsaturated, and if unsaturated, can be aromatic or non-aromatic. Heterocyclic groups preferably contain one or more nitrogen, oxygen, and/or sulfur heteroatoms. Examples of such groups include furazanyl, furyl, imidazolidinyl, imidazolyl, imidazolinyl, isothiazolyl, isoxazolyl, morpholinyl (*e*.*g*. morpholino), oxazolyl, piperazinyl (*e*.*g*., 1-piperazinyl), piperidyl (*e*.*g*., 1-piperidyl, piperidino), pyranyl, pyrazinyl, pyrazolidinyl, pyrazolinyl, pyrazolyl, pyridazinyl, pyridyl, pyrimidinyl, pyrrolidinyl (*e*.*g*., 1-pyrrolidinyl), pyrrolinyl, pyrrolyl, thiadiazolyl, thiazolyl, thienyl, thiomorpholinyl (*e*.*g*., thiomorpholino), and triazolyl. Heterocyclic groups can be substituted or unsubstituted. Where a group is substituted, the substituent can be alkyl, alkoxy, halogen, oxygen, or substituted or unsubstituted phenyl.

One can also readily modify polypeptides by phosphorylation, and other methods (*e*.*g*., as described in Hruby et al. Biochem J. 1990;268:249-262).

The invention also contemplates partially or wholly non-peptidic analogs of the polypeptide TLR2 ligands of the invention. For example, the peptide compounds of the invention serve as structural models for non-peptidic compounds with similar biological activity. Those of skill in the art recognize that a variety of techniques are available for constructing compounds with the same or similar desired biological activity as the lead peptide compound, but with more favorable activity than the lead with respect to solubility, stability, and susceptibility to hydrolysis and proteolysis (see, *e*.*g*., Morgan and Gainor. Ann. Rep. Med. Chem. 1989;24:243-252). These techniques include replacing the polypeptide backbone with a backbone composed of phosphonates, amidates, carbamates, sulfonamides, secondary amines, or N-methylamino acids.

In one form, the contemplated analogs of polypeptide TLR2 ligands are polypeptide-containing molecules that mimic elements of protein secondary structure (see, for example, Johnson et al. "Peptide Turn Mimetics," Biotechnology and Pharmacy. Pezzuto et al., eds. Chapman and Hall: 1993). Such molecules are expected to permit molecular interactions similar to the natural molecule. In another form, analogs of polypeptides are commonly used in the pharmaceutical industry as non-polypeptide drugs with properties analogous to those of a subject polypeptide (see, for example, Fauchere Adv. Drug Res. 1986;15:29-69; Veber et al. Trends Neurosci. 1985;8:392-396; and Evans et al. J Med. Chem. 1987;30:1229-1239), and are usually developed with the aid of computerized molecular modeling. Generally, analogs of polypeptides are structurally similar to the reference polypeptide, but have one or more peptide linkages optionally replaced by a linkage selected from the group consisting of :-CH₂NH-, -CH₂S-, -CH₂-CH₂-, -CH=CH- (cis or trans), -COCH₂-, - CH(OH)CH₂-, -CH₂SO-, and the like. See, for example, Morley Trends Pharmacol. Sci. 1980;1:463468; Hudson et al. Int J Pept Protein Res. 1979;14:177-185; Spatola et al. Life Sci. 1986;38:1243-1249; Hann. J. Chem. Soc. Perkin Trans. 1982;1:307-314; Ahnquist et al. J. Med. Chem. 1980;23:1392-1398; Jennings-White et al. Tetrahedron Lett. 1982;23:2533; Holladay et al. Tetrahedron Lett. 1983;24:4401-4404; and Hruby Life Sci. 1982;31:189-199.

Fully synthetic analogs of the polypeptide TLR2 ligands of the invention can be constructed by structure-based drug design through replacement of amino acids by organic moieties. See, for example, Hughes Philos. Trans. R Soc. Lond. 1980;290:387-394; Hodgson Biotechnol. 1991;9:19-21 and Suckling. Sci. Prog. 1991;75:323-359.

### The polypeptide TLR2 ligands of the invention can modulate TLR2 signaling:

In preferred embodiments, the polypeptide TLR2 ligands of the invention are functional TLR2 ligands, *i*.*e*. they modulate TLR2 signaling. Without intending to be limited by mechanism, it is believed that the polypeptide TLR2 ligands can modulate TLR2 signaling by binding to the extracellular portion of TLR2, thereby modulating the intracellular signaling cascade(s) of TLR2.

The ability of a polypeptide TLR2 ligand of the invention to modulate TLR2 signaling may be assessed using a variety of assay systems well known in the art.

In one embodiment, the ability of a polypeptide TLR2 ligand to modulate TLR2 signaling is measured in a dendritic cell (DC) activation assay. For this assay murine or human dendritic cell cultures may be obtained. For example, murine DCs may be generated *in vitro* as previously described (see, for example, Lutz et al. J Immun Meth. 1999;223:77-92). In brief, bone marrow cells from 6-8 week old C57BL/6 mice are isolated and cultured for 6 days in medium supplemented with 100 U/ml GMCSF (Granulocyte Macrophage Colony Stimulating Factor), replenishing half the medium every two days. On day 6, nonadherant cells are harvested and resuspended in medium without GMSCF and used in the DC activation assay. For example, human DCs may obtained commercially (for example, from Cambrex, Walkersville, MD) or generated *in vitro* from peripheral blood obtained from healthy donors as previously described (see, for example, Sallusto and Lanzavecchia. J Exp Med 1994;179:1109-1118). In brief, peripheral blood mononuclear cells (PBMC) are isolated by Ficoll gradient centrifugation. Cells from the 42.5-50% interface are harvested and further purified following magnetic bead depletion of B- and T-cells using antibodies to CD19 and CD2, respectively. The resulting DC enriched suspension is cultured for 6 days in medium supplemented with 100 U/ml GMCSF and 1000 U/ml IL-4 (Interleukin-4). On day 6, nonadherant cells are harvested and resuspended in medium without cytokines and used in the DC activation assay. For example, in a dendritic cell assay, a polypeptide TLR2 ligand may be added to DC cells in culture and the cultures incubated for 16 hours. Supernatants may be harvested, and cytokine (*e*.*g*., IFNγ, TNFα, IL-12, IL-10 and/or IL-6) concentrations may be determined, *e*.*g*., by sandwich enzyme-linked immunosorbent assay (ELISA) using matched antibody pairs (commercialy available, for example, from BD Pharmingen or R&D Systems) following the manufacturer's instructions. Cells may be harvested, and co-stimulatory molecule expression (*e*.*g*., B7-2) determined by flow cytometry using antibodies (commercially available, for example, from BD Pharmingen or Southern Biotechnology Associates) following the manufacturer's instructions. Analysis may be performed on a Becton Dickinson FACScan running Cellquest software. Polypeptide TLR2 ligands that modulate TLR2 signaling modulate cytokine and/or co-stimulatory molecule expression in the DC assay.

In another embodiment, the ability of a polypeptide TLR2 ligand to modulate expression of an NF-κB-reporter gene in a TLR2-dependent manner is assessed. As discussed above, one of the shared pathways of TLR signaling results in the activation of the transcription factor NF-κB. Therefore, expression of an NF-κB-dependent reporter gene can serve as an indicator of TLR signaling. In such an assay, the ability of a polypeptide TLR2 ligand to modulate expression of an NF-κB-dependent reporter gene in a TLR2 non-expressing cell (*i*.*e*., a cell that expresses very little or no TLR2) versus in a TLR2 expressing cell may be compared. For example, a polypeptide TLR2 ligand may significantly induce NF-κB-dependent reporter gene expression in a TLR2 expressing cell, but not in a TLR2 non-espressing cell. For example, HEK293 cells do not express detectable levels of endogenous TLR2. HEK293 cells harboring an NF-κB-dependent luciferase reporter gene, and ectopically expressing human or mouse TLR2 are available from Invivogen (Catalogue numbers 293-htlr2 and 293-mtlr2, respectively). For example, in such an assays, HEK293-TLR2 cells may grown in standard Dulbecco's Modified Eagle Medium (DMEM) medium with 10% Fetal Bovine Serum (FBS) supplemented with blasticidin (10 µg/ml) and then exposed to a polypeptide TLR2 ligand. Luciferase activity may be quantitated using commercial reagents.

In another embodiment, the ability of a polypeptide TLR2 ligand to modulate interleukin-8 (IL-8) expression in a TLR2-dependent manner is assessed. In such an assay, the ability of a polypeptide TLR2 ligand to modulate IL-8 expression in a TLR2 non-expressing cell (*i*.*e*., a cell that expresses very little or no TLR2) versus in a TLR2 expressing cell may be compared. For example, a polypeptide TLR ligand may significantly induce IL-8 expression in a TLR2 expressing cell, but not in a TLR2 non-espressing cell. For example, HEK293 cells do not express detectable levels of endogenous TLR2. HEK293 cells ectopically expressing human or mouse TLR2 are available from Invivogen (Catalogue numbers 293-htlr2 and 293-mtlr2, respectively). For example, for such an assay, HEK293-TLR2 cells may be grown in standard Dulbecco's Modified Eagle Medium (DMEM) medium with 10% Fetal Bovine Serum (FBS) supplemented with blasticidin (10 µg/ml), and then exposed to a polypeptide TLR2 ligand. IL-8 expression may then be quantitated by standard methods well known in the art, including Northern Blotting to detect IL-8 mRNA, immunostaining of a Western Blot to detect IL-8 protein, and fluorescence activated cell sorter (FACS) analysis using an anti-IL-8 antibody.

### Vaccines comprising the polypeptide TLR2 ligands of the invention:

The invention also provides vaccines comprising at least one polypeptide TLR2 ligand of the invention and at least one antigen. These vaccines combine both signals required for the induction of a potent adaptive immune response: an innate immune system signal (*i*.*e*. TLR2 signaling), and an antigen receptor signal (antigen). These vaccines may be used in methods to generate a potent antigen-specific immune response. In particular, these vaccines may used in situations where TLR2 receptor signaling (versus signaling through any of the other TLRs) is specifically desired.

It is particularly preferred that in the vaccines of the invention the at least one polypeptide TLR2 ligand and at least one antigen are covalently linked. As used herein, the term "polypeptide TLR2 ligand:antigen" refers to a vaccine composition comprising at least one polypeptide TLR2 ligand of the invention and at least one antigen, wherein the polypeptide TLR2 ligand and the antigen are covalently linked. Without intending to be limited by mechanism, it is thought that covalent linkage ensures that every cell that is exposed to antigen also receives an TLR2 receptor innate immune signal and *vice versa*. However, vaccines comprising at least one polypeptide TLR2 ligand and at least one antigen, in which the polypeptide TLR2 ligand and the antigen are mixed or associated in a noncovalent fashion, e.g. electrostatic interaction, are also contemplated.

### Composition of the vaccines of the invention

The novel vaccines of the present invention comprise at least one polypeptide TLR2 ligand of the invention and at least one antigen.

In one embodiment, the vaccines of the invention comprise at least one polypeptide TLR2 ligand, where the polypeptide TLR2 ligand comprises at least one peptide selected from the peptides set forth in Table 1. In some embodiments, the vaccines of the invention comprise at least one polypeptide TLR2 ligand, wherein the polypeptide TLR2 ligand comprises at least one of the peptide sequences set forth in Table 1 within the context of a longer polypeptide. For example, the vaccine may comprise at least one polypeptide TLR2 ligand, where the polypeptide TLR2 ligand comprises a peptide sequence as set forth in Table 1, and additional polypeptide sequences attached to the N-terminus, the C-teminus, or both the N- and C- termini of the peptide sequence. In such embodiments, the additional polypeptide sequences are preferably heterologous to the peptide sequence, *i*.*e*., they are not sequences which are endogenously associated with the given peptide sequence. However, embodiments, wherein the polypeptide TLR2 ligand comprises at least one of the peptide sequences set forth in Table 1 and additional polypeptide sequences, where the additional polypeptide sequences are sequences which are endogenously associated with said peptide sequence, are also contemplated.

In another embodiment, the vaccines of the invention comprise at least one polypeptide TLR2 ligand, where the polypeptide TLR2 ligand comprises at least one peptide selected from the peptides set forth in Table 2. In some embodiments, the vaccines of the invention comprise at least one polypeptide TLR2 ligand, wherein the polypeptide TLR2 ligand comprises at least one of the peptide sequences set forth in Table 2 within the context of a longer polypeptide. For example, the vaccine may comprise at least one polypeptide TLR2 ligand, where the polypeptide TLR2 ligand comprises a peptide sequence as set forth in Table 2, and additional polypeptide sequences attached to the N-terminus, the C-terminus, or both the N- and C- termini of the peptide sequence. In such embodiments, the additional polypeptide sequences are preferably heterologous to the peptide sequence, *i*.*e*., they are not sequences which are endogenously associated with the given peptide sequence. However, embodiments, wherein the polypeptide TLR2 ligand comprises at least one of the peptide sequences set forth in Table 2 and additional polypeptide sequences, where the additional polypeptide sequences are sequences which are endogenously associated with said peptide sequence, are also contemplated.

In another embodiment, the vaccines of the invention comprise at least one polypeptide TLR2 ligand, where the polypeptide TLR2 ligand comprises at least one peptide of 20 amino acids to 30 amino acids in length, wherein the peptide comprises at least 30% positively charged amino acids. In particularly preferred embodiments, the vaccines of the invention comprise at least one polypeptide TLR2 ligand, where the polypeptide TLR2 ligand comprises at least one peptide TLR2 ligand as set forth in Table 3.

The antigens used in the vaccines of the present invention can be any type of antigen, including but not limited to pathogen-related antigens, tumor-related antigens, allergy-related antigens, neural defect-related antigens, cardiovascular disease antigens, rheumatoid arthritis-related antigens, other disease-related antigens, hormones, pregnancy-related antigens, embryonic antigens and/or fetal antigens and the like. The antigen component of the vaccine can be derived from sources that include, but are not limited to, bacteria, viruses, fungi, yeast, protozoa, metazoa, tumors, malignant cells, plants, animals, humans, allergens, hormones and amyloid-β peptide. The antigens may be composed of, *e*.*g*., polypeptides, lipoproteins, glycoproteins, mucoproteins, lipids, saccharides, lipopolysaccharides, nucleic acids, and the like.

Specific examples of pathogen-related antigens include, but are not limited to, antigens selected from the group consisting of West Nile Virus (WNV, *e*.*g*., envelope protein domain EIII antigen) or other Flaviviridae antigens, *Listeria monocytogenes* (*e*.*g*., LLO or p60 antigens), Influenza A virus (*e*.*g*., the M2e antigen), vaccinia virus, avipox virus, turkey influenza virus, bovine leukemia virus, feline leukemia virus, chicken pneumovirosis virus, canine parvovirus, equine influenza, Feline rhinotracheitis virus (FHV), Newcastle Disease Virus (NDV), infectious bronchitis virus; Dengue virus, measles virus, Rubella virus, pseudorabies, Epstein-Barr Virus, Human Immunodeficieny Virus (HIV), Simian Immunodeficiency virus (SIV), Equine Herpes Virus (EHV), Bovine Herpes Virus (BHV), cytomegalovirus (CMV), Hantaan, *C*. *tetani*, mumps, Morbillivirus, Herpes Simplex Virus type 1, Herpes Simplex Virus type 2, Human cytomegalovirus, Hepatitis A Virus, Hepatitis B Virus, Hepatitis C Virus, Hepatitis E Virus, Respiratory Syncytial Virus, Human Papilloma Virus, *Salmonella, Neisseria, Borrelia, Chlamydia, Bordetella, Plasmodium, Toxoplasma, Cryptococcus, Streptococcus, Staphylococcus, Haemophilus, Diptheria, Pertussis, Escherichia, Candida, Aspergillus, Entamoeba, Giardia*, and *Trypanasoma*.

The methods and compositions of the present invention can also be used to produce vaccines directed against tumor-associated antigens such as melanoma-associated antigens, mammary cancer-associated antigens, colorectal cancer-associated antigens, prostate cancer-associated antigens and the like. Specific examples of tumor-related or tissue-specific antigens useful in such vaccines include, but are not limited to, antigens selected from the group consisting of prostate-specific antigen (PSA), prostate-specific membrane antigen (PSMA), Her-2, epidermal growth factor receptor, gp120, and p24. In order for tumors to give rise to proliferating and malignant cells, they must become vascularized. Strategies that prevent tumor vascularization have the potential for being therapeutic. The methods and compositions of the present invention can also be used to produce vaccines directed against tumor vascularization. Examples of target antigens for such vaccines are vascular endothelial growth factors, vascular endothelial growth factor receptors, fibroblast growth factors, fibroblast growth factor receptors, and the like.

Specific examples of allergy-related antigens useful in the methods and compositions of the present invention include, but are not limited to: allergens derived from pollen, such as those derived from trees such as Japanese cedar (*Cryptomeria*, *Cryptomeriajaponica*), grasses (*Gramineae*), such as orchard-grass (*e*.*g*. *Dactylis glomerata*); weeds such as ragweed (*e*.*g*. *Ambrosia artemisiifolia*); specific examples of pollen allergens including the Japanese cedar pollen allergens Cry j 1 and Cry j 2, and the ragweed allergens Amb a I.1, Amb a I.2, Amb a 1.3, Amnb a I.4, Amb a II etc.; allergens derived from fungi (*e*.*g*. *Aspergillus, Candida*, *Alternaria*, etc.); allergens derived from mites (*e*.*g*. allergens from *Dermatophagoides pteronyssinus, Dermatophagoides farinae etc*.); specific examples of mite allergens including Der p I, Der p II, Der p III, Der p VII, Der f I, Der f II, Der f III, Der f VII *etc*.; house dust; allergens derived from animal skin debris, feces and hair (for example, the feline allergen Fel d I); allergens derived from insects (such as scaly hair or scale of moths, butterflies, *Chironomidae etc*., poisons of the *Vespidae*, such as *Vespa mandarinia*); food allergens (eggs, milk, meat, seafood, beans, cereals, fruits, nuts, vegetables, *etc*.); allergens derived from parasites (such as roundworm and nematodes, for example, *Anisakis*); and protein or peptide based drugs (such as insulin). Many of these allergens are commercially available.

Also contemplated in this invention are vaccines directed against antigens that are associated with diseases other than cancer, allergy and asthma. As one example of many, and not by limitation, an extracellular accumulation of a protein cleavage product of β-amyloid precursor protein, called "amyloid-β peptide", is associated with the pathogenesis of Alzheimer's disease (Janus et al. Nature 2000;408:979-982 and Morgan et al. Nature 2000;408:982-985). Thus, the vaccines of the present invention can comprise an amyloid-β polypeptide.

The vaccines of the invention may additionally comprise carrier molecules such as polypeptides (*e*.*g*., keyhole limpet hemocyanin (KLH)), liposomes, insoluble salts of aluminum (*e*.*g*. aluminum phosphate or aluminum hydroxide), polynucleotides, polyelectrolytes, and water soluble carriers (*e*.*g*. muramyl dipeptides). A polypeptide TLR2 ligand and/or antigen can, for example, be covalently linked to a carrier molecule using standard methods. See, for example, Hancock *et al*. "Synthesis of Peptides for Use as Immunogens," *Methods in Molecular Biology: Immunochemical Protocols*. Manson, ed. (Humana Press: 1992).

### Chemical conjugates

In one embodiment, the vaccines of the invention comprise at least one polypeptide TLR2 ligand of the invention chemically conjugated to at least one antigen. Methods for the chemical conjugation of polypeptides, carbohydrates, and/or lipids are well known in the art. See, for example, Hermanson. Bioconjugate Techniques (Academic Press; 1992); Aslam and Dent, eds. Bioconjugation: Protein coupling Techniques for the Biomedical Sciences (MacMillan: 1998); and Wong Chemistry of Protein Conjugation and Cross-linking (CRC Press: 1991). For example, in the case of carbohydrate or lipid antigens, functional amino and sulfhydryl groups may be incorporated therein by conventional chemistry. For instance, primary amino groups may be incorporated by reaction with ethylenediamine in the presence of sodium cyanoborohydride and sulfhydryls may be introduced by reaction of cysteamin dihydrochloride followed by reduction with a standard disulfide reducing agent.

Heterobifunctional crosslinkers, such as sulfosuceinimidyl (4-iodoacetyl) aminobenzoate, which link the epsilon amino group on the D-lysine residues of copolymers of D-lysine and D-glutamate to a sulfhydryl side chain from an amino terminal cysteine residue on the peptide to be coupled, may be used to increase the ratio of polypeptide TLR2 ligand to antigen in the conjugate.

Polypeptide TLR2 ligands and polypeptide antigens will contain amino acid side chains such as amino, carbonyl, hydroxyl, or sulfhydryl groups or aromatic rings that can serve as sites for linking the polypeptide TLR2 ligands and polypeptide antigens to each other, or for linking the polypeptide TLR2 ligands to an non-polypeptide antigen. Residues that have such functional groups may be added to either the polypeptide TLR2 ligands or polypeptide antigens. Such residues may be incorporated by solid phase synthesis techniques or recombinant techniques, both of which are well known in the art.

Polypeptide TLR2 ligands and polypeptide antigens may be chemically conjugated using conventional crosslinking agents such as carbodiimides. Examples of carbodiimides are 1-cyclohexyl-3-(2-morpholinyl-(4-ethyl) carbodiimide (CMC), 1-ethyl-3-(3-dimethyaminopropyl) carbodiimide (EDC), and 1-ethyl-3-(4-azonia-44-dimethylpentyl) carbodiimide.

Examples of other suitable crosslinking agents are cyanogen bromide, glutaraldehyde and succinic anhydride. In general, any of a number of homobifunctional agents including a homobifunctional aldehyde, a homobifunctional epoxide, a homobifunctional imidoester, a homobifunctional N-hydroxysuccinimide ester, a homobifunctional maleimide, a homobifunctional alkyl halide, a homobifunctional pyridyl disulfide, a homobifunctional aryl halide, a homobifunctional hydrazide, a homobifunctional diazonium derivative or a homobifunctional photoreactive compound may be used. Also included are heterobifunctional compounds, for example, compounds having an amine-reactive and a sulfhydryl-reactive group, compounds with an amine-reactive and a photoreactive group, and compounds with a carbonyl-reactive and a sulfhydryl-reactive group.

Specific examples of homobifunctional crosslinking agents include the bifunctional N-hydroxysuccinimide esters dithiobis (succinimidylpropionate), disuccinimidyl suberate, and disuccinimidyl tartarate; the bifunctional imidoesters dimethyl adipimidate, dimethyl pimelimidate, and dimethyl suberimidate; the bifunctional sulfhydryl-reactive crosslinkers 1,4-di-[3'-(2'-pyridyldithio) propion-amido]butane, bismaleimidohexane, and bis-N-maleimido-1,8-octane; the bifunctional aryl halides 1,5-difluoro-2,4-dinitrobenzene and 4,4'-difluoro-3,3'-dinitrophenylsulfone; bifunctional photoreactive agents such as bis-[b-(4-azidosalicylamide)ethyl]disulfide; the bifunctional aldehydes formaldehyde, malondialdehyde, succinaldehyde, glutaraldehyde, and adiphaldehyde; a bifunctional epoxied such as 1,4-butaneodiol diglycidyl ether; the bifunctional hydrazides adipic acid dihydrazide, carbohydrazide, and succinic acid dihydrazide; the bifunctional diazoniums o-tolidine, diazotized and bis-diazotized benzidine; the bifunctional alkylhalides N1N'-ethylene-bis(iodoacetamide), N1N'-hexamethylene-bis(iodoacetamide), N1N'-undecamethylene-bis(iodoacetamide), as well as benzylhalides and halomustards, such as a1a'-diiodo-p-xylene sulfonic acid and tri(2-chloroethyl)amine, respectively.

Examples of other common heterobifunctional crosslinking agents that may be used include, but are not limited to, SMCC (succinimidyl-4-(N-maleimidomethyl)cyclohexane-1-carboxylate), MBS (m-maleimidobenzoyl-N-hydroxysuccinimide ester), SIAB (N-succinimidyl(4-iodacteyl) aminobenzoate), SMPB (succinimidyl-4-(p-maleimidophenyl)butyrate), GMBS (N-(ÿ-maleimidobutyryloxy)succinimide ester), MPHB (4-(4-N-maleimidopohenyl) butyric acid hydrazide), M2C2H (4-(N-maleimidomethyl) cyclohexane-1-carboxyl-hydrazide), SMPT (succinimidyloxycarbonyl-á- methyl-á-(2-pyridyldithio)toluene), and SPDP (N-succinimidyl 3-(2-pyridyldithio) propionate). For example, crosslinking may be accomplished by coupling a carbonyl group to an amine group or to a hydrazide group by reductive amination.

In one embodiment, at least one polypeptide TLR2 ligand and at least one antigen are linked through polymers, such as PEG, poly-D-lysine, polyvinyl alcohol, polyvinylpyrollidone, immunoglobulins, and copolymers of D-lysine and D-glutamic acid. Conjugation of a polypeptide TLR2 ligand and an antigen to a polymer linker may be achieved in any number of ways, typically involving one or more crosslinking agents and functional groups on the polypeptide TLR2 ligand and the antigen. The polymer may be derivatized to contain functional groups if it does not already possess appropriate functional groups.

### Fusion proteins

In preferred embodiments, the vaccines of the invention comprise a fusion protein, wherein the fusion protein comprises at least one polypeptide TLR2 ligand of the invention and at least one polypeptide antigen. In one embodiment the polypeptide TLR2 ligand:antigen fusion protein is obtained by *in vitro* synthesis of the fusion protein. Such *in vitro* synthesis may be performed according to any methods well known in the art (see the Section Preparation of the polypeptide TLR2 ligands of the invention: **In vitro *chemical synthesis*,** above).

In particularly preferred embodiments, the polypeptide TLR2 ligand:antigen fusion protein is obtained by translation of a nucleic acid sequence encoding the fusion protein. A nucleic acid sequence encoding a polypeptide TLR2 ligand:antigen fusion protein may be obtained by any of the synthetic or recombinant DNA methods well known in the art. See, for example, DNA Cloning: A Practical Approach, Vol I and II (Glover ed.:1985); Oligonucleotide Synthesis (Gait ed.:1984); Transcription And Translation (Hames & Higgins, eds.:1984); Perbal, A Practical Guide To Molecular Cloning (1984); Ausubel et al., eds. Current Protocols in Molecular Biology, (John Wiley & Sons, Inc.:1994); PCR Primer: A Laboratory Manual, 2nd Edition. Dieffenbach and Dveksler, eds. (Cold Spring Harbor Laboratory Press: 2003); and Sambrook et al. Molecular Cloning: A Laboratory Manual, 3rd Edition (Cold Spring Harbor Laboratory Press: 2001).

Translation of a nucleic acid sequence encoding a polypeptide TLR2 ligand:antigen fusion protein may be achieved by any of the *in vitro* or *in vivo* methods well known in the art (see the section Preparation of the polypeptide TLR2 ligands of the invention: ***Translation from coding sequences*,** above).

### Vaccine formulations

Methods of formulating pharmaceutical compositions and vaccines are well-known to those of ordinary skill in the art (see, *e.g.,* Remington's Pharmaceutical Sciences, 18th Edition, Gennaro, ed. Mack Publishing Company:1990). The vaccines of the invention are administered, *e*.*g*., to human or non-human animal subjects, in order to stimulate an immune response specifically against the antigen and preferably to engender immunological memory that leads to mounting of a protective immune response should the subject encounter that antigen at some future time.

The vaccines of the invention comprise at least one polypeptide TLR2 ligand and at least one antigen, and optionally a pharmaceutically acceptable carrier. As used herein, the phrase "pharmaceutically acceptable" refers to molecular entities and compositions that are "generally regarded as safe", e.g., that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Other suitable carriers include polypeptides (*e*.*g*., keyhole limpet hemocyanin (KLH)), liposomes, insoluble salts of aluminum (*e*.*g*. aluminum phosphate or aluminum hydroxide), polynucleotides, polyelectrolytes, and water soluble carriers (*e*.*g*. muramyl dipeptides). Water or aqueous solutions, such as saline solutions and aqueous dextrose and glycerol solutions, are preferably employed as carriers, particularly for injectable solutions. Suitable pharmaceutical carriers are described in Remington's Pharmaceutical Sciences, 18th Edition, Gennaro, ed. (Mack Publishing Company: 1990).

As discussed above, the vaccines of the invention combine both signals required for the induction of a potent antigen-specific adaptive immune response: an innate immune system signal (*i.e.* TLR2 signaling) and an antigen receptor signal. This combination of signals provides for the induction of a potent immune response without the use of convention adjuvants. Thus, in preferred embodiments, the vaccines of the invention are formulated without conventional adjuvants. However, the invention also contemplates vaccines comprising at least one polypeptide TLR2 ligand and at least one antigen, wherein the vaccine additionally comprises an adjuvant. As used herein, the term "adjuvant" refers to a compound or mixture that enhances the immune response to an antigen. An adjuvant can serve as a tissue depot that slowly releases the antigen and also as a lymphoid system activator that non-specifically enhances the immune response (Hood et al., Immunology, Second Ed., 1984, Benjamin/Cummings: Menlo Park, California, p. 384). Adjuvants include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, keyhole limpet hemocyanins, and potentially useful human adjuvants such as N-acetyl-muramyl-L-threonyl-D-isoglutamine (thr-MDP), N-acetyl-nor-muramyl-L-alanyl-D-isoglutamine, N-acetylmuramyl-L-alanyl-D-isoglutaminyl-L-alanine-2-(1'-2'-dipalmitoyl-sn-glycero-3-hydroxyphosphoryloxy)-ethylamine, BCG (*bacille Calmette*-*Guerin*), and *Corynebacterium parvum*. Where the vaccine is intended for use in human subjects, the adjuvant should be pharmaceutically acceptable.

For example, vaccine administration can be by oral, parenteral (intramuscular, intraperitoneal, intravenous (TV) or subcutaneous injection), transdermal (either passively or using iontophoresis or electroporation), or transmucosal (nasal, vaginal, rectal, or sublingual) routes of administration or using bioerodible inserts and can be formulated in dosage forms appropriate for each route of administration. Moreover, the administration may be by continuous infusion or by single or multiple boluses.

The vaccine formulations may include diluents of various buffer content (*e*.*g*., Tris-HCl, acetate, phosphate), pH and ionic strength; additives such as detergents and solubilizing agents (*e*.*g*., Tween 80, Polysorbate 80); anti-oxidants (*e*.*g*., ascorbic acid, sodium metabisulfite); preservatives (*e*.*g*., Thimersol, benzyl alcohol); bulking substances (*e*.*g*., lactose, mannitol); or incorporation of the material into particulate preparations of polymeric compounds such as polylactic acid, polyglycolic acid, *etc*. or into liposomes. Hylauronic acid may also be used. See, *e.g., Remington's Pharmaceutical Sciences, 18^{th} Edition,* Gennaro, ed. (Mack Publishing Company: 1990).

The vaccines may be formulated so as to control the duration of action of the vaccine in a therapeutic application. For example, controlled release preparations can be prepared through the use of polymers to complex or adsorb the vaccine. For example, biocompatible polymers include matrices of poly(ethylene-co-vinyl acetate) and matrices of a polyanhydride copolymer of a stearic acid dimer and sebacic acid (see, for example, Sherwood *et al. Bio*/*Technology* 1992;10:1446). The rate of release of the vaccine from such a matrix depends upon the molecular weight of the construct, the amount of the construct within the matrix, and the size of dispersed particles. See, for example, Saltzman *et al*. *Biophys*. *J*. 1989;55:163; Sherwood *et al*. *Bio*/*Technology* 1992;10:1446; Ansel *et al*. *Pharmaceutical Dosage Forms and Drug Delivery Systems, 5th Edition* (Lea & Febiger 1990); and *Remington's Pharmaceutical Sciences, 18^{th} Edition,* Gennaro, ed. (Mack Publishing Company:1990). The vaccine can also be conjugated to polyethylene glycol (PEG) to improve stability and extend bioavailability times (see, *e*.*g*., U.S. Pat. No. 4,766,106).

Contemplated for use herein are oral solid dosage forms, which are described generally in *Remington's Pharmaceutical Sciences*, *18^{th} Edition,* Gennaro, ed. (Mack Publishing Company:1990) at Chapter 89, which is herein incorporated by reference. Solid dosage forms include tablets, capsules, pills, troches or lozenges, cachets, pellets, powders, or granules. Also, liposomal or proteinoid encapsulation may be used to formulate the present compositions (as, for example, proteinoid microspheres reported in U.S. Patent No. 4,925,673). Liposomal encapsulation may be used and the liposomes may be derivatized with various polymers (*e*.*g*., U.S. Patent No. 5,013,556). A description of possible solid dosage forms for a therapeutic is given by, for example, Marshall, K. In: *Modern Pharmaceutics*. Banker and Rhodes, eds. Chapter 10, 1979, herein incorporated by reference. In general, the formulation will include the therapeutic agent and inert ingredients which allow for protection against the stomach environment, and for release of the biologically active material in the intestine.

Also contemplated for use herein are liquid dosage forms for oral administration, including pharmaceutically acceptable emulsions, solutions, suspensions, and syrups, which may contain other components including inert diluents, wetting agents, emulsifying and/or suspending agents, and sweetening, flavoring, coloring, and/or perfuming agents.

For oral formulations, the location of release may be the stomach, the small intestine (the duodenum, the jejunem, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the therapeutic agent or by release of the therapeutic agent beyond the stomach environment, such as in the intestine. To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac. These coatings may be used as mixed films.

A coating or mixture of coatings can also be used on tablets, which are not intended for protection against the stomach. These coatings can include sugar coatings, or coatings which make the tablet easier to swallow. Capsules may consist of a hard shell (such as gelatin) for delivery of dry therapeutic (*i*.*e*. powder). For liquid forms a soft gelatin shell may be used. The shell material of cachets could be thick starch or other edible paper. For pills, lozenges, molded tablets or tablet triturates, moist massing techniques can be used. The formulation of a material for capsule administration could also be as a powder, lightly compressed plugs, or even as tablets. These therapeutics could be prepared by compression.

One may dilute or increase the volume of the therapeutic agent with an inert material. These diluents could include carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic agent into a solid dosage form. Materials used as disintegrants include but are not limited to starch (including the commercial disintegrant based on starch, Explotab), sodium starch glycolate, Amberlite, sodium carboxymethylcellulose, ultramylopectin, sodium alginate, gelatin, orange peel, acid carboxymethyl cellulose, natural sponge and bentonite. Disintegrants may also be insoluble cationic exchange resins. Powdered gums may be used as disintegrants and as binders, and can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Other binders include methyl cellulose (MC), ethyl cellulose (EC) and carboxymethyl cellulose (CMC). Polyvinyl pyrrolidone (PVP) and/or hydroxypropylmethyl cellulose (HPMC) may be used in alcoholic solutions to granulate a peptide (or derivative).

An antifrictional agent may be included in the formulation to prevent sticking during the formulation process. Lubricants may be used as a layer between the therapeutic agent and the die wall, and these can include, but are not limited to, stearic acid including its magnesium and calcium salts, polytetrafluoroethylene (PTFE), liquid paraffin, vegetable oils and waxes. Soluble lubricants may also be used, such as sodium lauryl sulfate, magnesium lauryl sulfate, polyethylene glycol of various molecular weights, and Carbowax 4000 and 6000.

Glidants that might improve the flow properties of the therapeutic agent during formulation and to aid rearrangement during compression may be added. The glidants may include starch, talc, pyrogenic silica and hydrated silicoaluminate.

To aid dissolution of the therapeutic agent into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. Nonionic detergents that may be included in the formulation as surfactants include lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants may be present in the formulation of the therapeutic agent either alone or as a mixture in different ratios.

Controlled release oral formulations may be desirable. The therapeutic agent may be incorporated into an inert matrix which permits release by either diffusion or leaching mechanisms, *e*.*g*., gums. Slowly degenerating matrices may also be incorporated into the formulation. Some enteric coatings also have a delayed release effect. Another form of a controlled release is by a method based on the Oros therapeutic system (Alza Corp.), *i*.*e*. the therapeutic agent is enclosed in a semipermeable membrane which allows water to enter and push agent out through a single small opening due to osmotic effects.

Other coatings may be used for the formulation. These include a variety of sugars which could be applied in a coating pan. The therapeutic agent could also be given in a film coated tablet and the materials used in this instance are divided into 2 groups. The first are the nonenteric materials and include methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, providone and the polyethylene glycols. The second group consists of the enteric materials that are commonly esters of phthalic acid. A mix of materials might be used to provide the optimum film coating. Film coating may be carried out in a pan coater or in a fluidized bed or by compression coating.

Vaccines according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants, preserving, wetting, emulsifying, and dispersing agents. They can also be manufactured using sterile water, or some other sterile injectable medium, immediately before use.

Regarding the dosage of the vaccines of the present invention, the ordinary skilled practitioner, considering the therapeutic context, age, and general health of the recipient, will be able to ascertain proper dosing. The selected dosage depends upon the desired therapeutic effect, on the route of administration, and on the duration of the treatment desired. The dosing schedule may vary, depending on the circulation half-life, and the formulation used.

The vaccines of the present invention may be administered in conjunction with one or more additional active ingredients, pharmaceutical compositions, or vaccines.

### Methods of modulating TLR2 signaling

The invention provides methods of modulating TLR2 signalling, comprising administering to a subject in need thereof a polypeptide TLR2 ligand or vaccine of the invention. In preferred embodiments, the subject is a mammal. In particularly preferred embodiments, the subject is a human.

Thus, a polypeptide TLR2 ligand or vaccine of the invention may be administered to subjects, *e*.*g*., mammals including humans, in order to modulate TLR2 signaling. For a discussion of TLR2 signaling and assays to detect modulation of TLR2 signaling see the section The polypeptide TLR2 ligands of the invention modulate TLR2 signaling, above.

In such subjects, modulation of TLR2 signaling may be used to modulate an immune response in the subject. In particular, modulation of TLR2 signaling may be used to modulate an antigen-specific immune response in the subject, *e*.*g*., to engender immunological memory that leads to mounting of a protective immune response should the subject encounter that antigen at some future time. Modulation of an immune response in a subject can be measured by standard tests including, but not limited to, the following: detection of antigen-specific antibody responses, detection of antigen specific T-cell responses, including cytotoxic T-cell responses, direct measurement of peripheral blood lymphocytes; natural killer cell cytotoxicity assays (see, for example, Provinciali et al. J. Immunol. Meth. 1992;155:19-24), cell proliferation assays (see, for example, Vollenweider et al. J. Immunol. Meth. 1992;149:133-135), immunoassays of immune cells and subsets (see, for example, Loeffler et al. Cytom. 1992;13:169-174 and Rivoltini et al. Can. Immunol. Immunother. 1992;34:241-251), and skin tests for cell mediated immunity (see, for example, Chang et al. Cancer Res. 1993;53:1043-1050). Various methods and analyses for measuring the strength of the immune system are well known in the art (see, for example, Coligan et al., eds. Current Protocols in Immunology, Vol. 1. Wiley & Sons: 2000).

The invention also provides methods of modulating TLR2 signaling comprising contacting a cell, wherein the cell comprises TLR2, with a polypeptide TLR2 ligand of the invention. As used herein, a cell that comprises TLR2 is any cell that contains TLR2 protein, including a cell that endogenously expresses TLR2; a cell that does not endogenously express TLR2 but ectopically expresses TLR2; and a cell that endogenously expresses TLR2 and ectopically expresses additional TLR2. In preferred embodiments, the cell is a mammalian cell. In particularly preferred embodiments, the cell is a mouse cell or a human cell. The cell may be a cell cultured *in vitro* or a cell *in vivo*.

Cells that endogenously express TLR2 include NIH3T3 cells (ATCC Accession # CRL-1658), RAW264.7 cells (ATCC Accession # TIB-71), dendritic cells, macrophages, B-cells, and natural killer cells. Cells that do not endogenously express TLR2 include HEK293 cells (ATCC Accession # CRI-1573). Cells that ectopically express TLR2 may be generated by standard techniques well known in the art. For example, pUNO-mTLR2, pUNO-hTLR2, and p-DUO-hCD14/hTLR2 plasmids are available from Invivogen. These plasmids provide for high level TLR2 expression in mammalian host cells (*e*.*g*., HEK293 and NIH3T3 cells)..

The TLR2 expression status of a cell may be determined by any of the techniques well established in the art including Western blotting, immunoprecipitation, flow cytometry / FACS, immunohistochemistry/immunocytochemistry, Northern blotting, RT-PCR, whole *mount in situ* hybridization, *etc*. For example, monoclonal and polyclonal antibodies to human or mouse TLR2 are commercially available, *e*.*g*., from Active Motif, BioVision, IMGENEX, R&D Systems, ProSci, Cellsciences, and eBioscience. For example, human TLR2 and mouse/rat TLR2 primer pairs are commercially available, *e*.*g*., from R&D Systems and Bioscience Corporation. For example, SuperArray RT-PCR Profiling Kits for simultaneous quantitation of the expression of mouse TLRs 1 through 9 are available from Bioscience Corporation.

### EXAMPLES

The present invention is next described by means of the following examples. However, the use of these and other examples anywhere in the specification is illustrative only, and in no way limits the scope and meaning of the invention or of any exemplified form. Likewise, the invention is not limited to any particular preferred embodiments described herein. Indeed, many modifications and variations of the invention may be apparent to those skilled in the art upon reading this specification, and can be made without departing from its spirit and scope. The invention is therefore to be limited only by the terms of the appended claims, along with the full scope of equivalents to which the claims are entitled.

In accordance with the present invention there may be employed conventional molecular biology, microbiology, protein expression and purification, antibody, and recombinant DNA techniques within the skill of the art. Such techniques are explained fully in the literature. See, *e*.*g*., DNA Cloning: A Practical Approach, Vol I and II (Glover ed.:1985); Oligonucleotide Synthesis (Gait ed.:1984); Nucleic Acid Hybridization (Hames & Higgins eds.:1985); Transcription And Translation (Hames & Higgins, eds.:1984); Animal Cell Culture (Freshney, ed.:1986); Immobilized Cells And Enzymes (IRL Press:1986); Perbal, A Practical Guide To Molecular Cloning (1984); Ausubel et al., eds. Current Protocols in Molecular Biology, (John Wiley & Sons, Inc.:1994); Sambrook et al. Molecular Cloning: A Laboratory Manual, 3rd Edition (Cold Spring Harbor Laboratory Press: 2001); Harlow and Lane. Using Antibodies: A Laboratory Manual (Cold Spring Harbor Laboratory Press: 1999); PCR Primer: A Laboratory Manual,2nd Edition. Dieffenbach and Dveksler, eds. (Cold Spring Harbor Laboratory Press: 2003); and Hockfield et al. Selected Methods for Antibody and Nucleic Acid Probes (Cold Spring Harbor Laboratory Press: 1993).

### EXAMPLE 1: CELL LINES ECTOPICALLY EXPRESSING TLRs

### Materials and Methods

***Generation of cell lines ectopically expressing TLRs:*** Parental "293.luc" cells, which are HEK293 (ATCC Accession # CRL-1573) that have been stably transfected with an NF-κB reporter gene vector containing tandem copies of the NF-κB consensus sequence upstream of a minimal promoter fused to the firefly luciferase gene (κB-LUC), were cultured at 37°C under 5% CO₂ in standard Dulbecco's Modified Eagle Medium (DMEM; *e*.*g*., Gibco) with 10% Fetal Bovine Serum (FBS; *e*.*g*., Hyclone).

Parental "3T3.luc" cells, which are NIH3T3 cells (ATCC Accession # CRL-1658) that have been stably transfected with an NF-κB reporter gene vector containing tandem copies of the NF-κB consensus sequence upstream of a minimal promoter fused to the firefly luciferase gene (κB-LUC), were cultured at 37°C under 5% CO₂ in DMEM (*e*.*g*., Gibco) with 10% FBS (*e*.*g*., Hyclone).

The following pUNO-TLR plasmids were obtained from Invivogen: human TLR2 (catalog # puno-htlr2), human TLR4 isoform a (catalog # puno-htlr4a), mouse TLR5 (catalog # puno-mtlr5), and human TLR5 (catalog # puno-htlr5). The following pDUO-CD14/TLR plasmids were obtained from Invivogen: human CD14 plus human TLR2 (catalog # pduo-hcd14/tlr2) and human CD14 plus human TLR2 (catalog # pduo-hcd14/tlr4). The pUNO-TLR and pDUO-CD14/TLR plasmids are optimized for the rapid generation of stable transformants and for high levels of expression.

The pUNO-TLR or pDUO-CD14/TLR plasmids were transfected into HEK293 and/or NIH3T3 cells lines using Lyovec (Invivogen), a cationic lipid-based transfection reagent. Transfected cells were cultured at 37°C under 5% CO₂ in DMEM (*e*.*g*., Gibco) medium with 10% FBS (*e*.*g*., Hyclone) supplemented with blasticidin (10 µg/ml). Stably transfected, individual blasticidin-resistant clones were isolated. The cell lines thereby generated are listed in Table 4.

**Table 4: HEK293 and NIH3T3 lines ectopically expressing TLRs and CD14. "293" = HEK293 cells. "3T3"= NIH3T3 cells. h=human. m=mouse.**

| **Clone designation** | **Transfected constructs** |
|---|---|
| 293.1uc | - |
| 293.hTLR2 | pUNO-hTLR2, RUB-LUG |
| 293.hTLR2.hCD14 | pDUO-hCD14/hTLR2, κB-LUC |
| 293.hTLR4 | pUNO-hTLR4a, κB-LUC |
| 293.hTLR4.hCD14 | pDUO-hCD14/hTLR4, κB-LUC |
| 293.hTLRS | pUNO-hTLR5, κB-LUC |
| 3T3.luc | - |
| 3T3.mTLR5 | pUNO-mTLR5, κB-LUC |

***Analysis of TLR expression in HEK293 and NIH3T3 cells:*** Individual blasticidin-resistant clones of transfected HEK293 and NIH3T3 have been isolated and characterized by Western blot analysis or flow cytometric analysis using polyclonal antibodies to the appropriate TLR to select clones which over-express the desired receptor.

To prepare whole cell lysate (WCE) for Western Blot analysis, sub-confluent cultures in 10 mm dishes were washed with PBS at room temperature. The following steps were then performed on ice or at 4°C using fresh, ice-cold buffers. Six hundred microliters of RIPA buffer (Santa Cruz Biotechnology Inc., catalog # sc-24948; RIPA buffer: 1XTBS, 1% NP-40, 0.5% Sodium deoxycholate, 0.1% SDS, protease inhibitor cocktail) was added to the culture plate and the contents gently rocked for 15 minutes at 4°C. The cells were then harvested by scraping with a cell scraper and the scraped lysate was transferred to a microcentrifuge tube. The plate was washed once with 0.3 ml of RIPA buffer and combined with first lysate. An aliquot of 10 µl of 10 mg/ml PMSF (Santa Cruz Biotechnology Inc., catalog # sc-3597) stock was added and the lysate passed through a 21-gauge needle to shear the DNA. The cell lysate was incubated 30-60 minutes on ice. The cell lysate was microcentrifuged 10,000xg for 10 minutes at 4°C. The lysate supernatant was transferred to a new microfuge tube and the pellet discarded. A 10 µl aliquot of lysate supernatant was loaded onto 10% SDS-PAGE gels and electrophoreses was performed according to standard protocols. The proteins were either stained by Coommassie Blue or transferred from the gels to a nitrocellulose or PVDF membrane using an electroblotting apparatus (BIORAD) according to the manufacturer's protocols. The membrane was then blotted with rabbit anti-hTLR2 polyclonal antibody (Invivogen, catalog # ab-htlr2) and reacted with a secondary antibody, goat anti-rabbit IgG Fc (Pierce, catalog # 31341).

For flow cytometric analysis, HEK293 cells were removed from culture and resuspended in FACS staining buffer (phosphate buffered saline (PBS) containing 2% bovine serum albumin (BSA) and 0.01% sodium azide). A total of 10⁵ cells were then stained in a volume of 100 µl with the biotin labeled monoclonal antibody to TLR4, clone HTA125 (BD Pharmingen, catalog # 551975) for 30 minutes at 4°C. Cells were then washed 3 times and incubated with streptavidin-FITC conjugated secondary antibody (BD Pharmingen, catalog # 554060). Following incubation at 4°C for 30 minutes samples were washed 3x with FACS buffer and then fixed in phosphate buffer containing 3% paraformaldehyde. Samples were then analyzed on a FACScan cytometer (BD Pharmingen) and analyzed using CellQuest software.

### Results and Discussion

In order to identify and affinity select potent ligands for TLRs from a peptide library displayed on bacteriophage, it is essential to employ cell lines expressing the TLR of choice. HEK293 cells and NIH3T3 cells, which had been previously stably transfected with a κB-LUC reporter gene, were stably transfected with pUNO-TLR and pDUO-CD14/TLR plasmid constructs from Invivogen. Individual blasticidin-resistant clones were isolated and
characterized by Western blot analysis or flow cytometric analysis using polyclonal antibodies to CD14 and/or the appropriate TLR to select clones which over-express the desired receptor. Using this strategy, we have generated cell lines over-expressing various TLRs and CD14 as summarized in Table 5.

**Table 5: Expression of TLRs and CD14 in HEK293 and NIH3T3 cells. h=human. m=mouse. NT = not tested.**

| **Clone** | **TLR2** | **TLR4** | **TLR5** | **CD14** |
|---|---|---|---|---|
| 293.luc | - | + | + | + |
| 293.hTLR2 | + | + | + | + |
| 293.hTLR2.hCD14 | + | + | + | + |
| 293.hTLR4 | - | ++ | + | + |
| 293.hTLR4.hCD14 | - | ++ | + | + |
| 293.hTLR5 | - | + | ++ | + |
| 3T3.luc | + | + | + | NT |
| 3T3.mTLR5 | + | + | ++ | NT |

Please note that while HEK293 (ATCC Accession # CRL-1573) obtained from the ATCC do not express TLR4 or respond to LPS (a TLR4 ligand), the parental 293.luc cell line used here does express detectable amounts of TLR4. The reason for this difference between the two cells lines is presently unclear. Notably, however, 293.luc cells (like HEK293 cells) do not to respond to LPS, indicating that they do not contain functional TLR4 protein.

As discussed above, one of the shared pathways of TLR signaling results in the activation of the transcription factor NF-κB. Therefore, in the cell lines generated here, expression of the NF-κB-dependent reporter gene serves as an indicator of TLR signaling.

### EXAMPLE 2: PHAGE DISPLAY LIBRARY CONSTRUCTION

### Materials and Methods

***Construction** of **biased peptide libraries** (BPL):* Libraries of phage displaying overlapping peptides (between 5 and 20 amino acids) spanning the entire region of Measles Virus hemagglutinin (HA, a TLR2 ligand), respiratory syncytial virus fusion protein (RSV F, a TLR4 ligand), or *E*. *coli* flagellin (fliC, a TLR5 ligand) are constructed. The nucleotide and amino acid sequences of measles HA (GenBank Accession # D28950) are set forth in SEQ ID NO: 29 and SEQ ID NO: 30, respectively. The nucleotide and amino acid sequences of RSV F (GenBank Accession # D00334) are set forth in SEQ ID NO: 31 and SEQ ID NO: 32, respectively. The nucleotide and amino acid sequences of *E*. coli fliC are set forth in SEQ ID NO: 33 and SEQ ID NO: 34, respectively.

To construct a library, synthetic oligonucleotides covering the entire coding region of the polypeptide of interest (e.g. RSV F) are converted to double-stranded molecules, digested with *EcoR*I and *Hind*III restriction enzymes, and ligated into the T7SELECT bacteriophage vector (Novagen). The ligation reactions are packaged *in vitro* and amplified by either the plate or liquid culture method (according to manufacturer's instructions). The amplified phages are titred (according to manufacturer's instructions) to evaluate the total number of independent clones present in the library. The amplified library will contain approximately 10²-10³ individual clones.

***Construction of random peptide libraries (RPL):*** Libraries of phage displaying random peptides of from 5 to 30 amino acids in length are constructed essentially as described above for biased peptide libraries, but utilizing oligonucleotides of defined length and random sequences. It is generally recognized that the major constraints of phage display are the bias and diversity (or completeness) of the RPL. To circumvent the former problem, the RPLs ARE constructed with only 32 codons (*e*.*g*. in the form NNK or NNS where N=A/T/G/C; K=G/T; S=G/C), thus reducing the redundancy inherent in the genetic code from a maximum codon number of 64 to 32 by eliminating redundant codons. For example, a 6-amino acid residue library displaying all possible hexapeptides requires 32⁶ (=10⁹) unique clones and is thus considered a complete library. Assuming a practical upper limit of ∼10⁹-10¹⁰ clones, RPLs longer than 7 residues accordingly risk being incomplete. This is not a major concern, since a longer residue library may actually increase the effective library diversity and thus is more suitable for isolating new polypeptide TLR ligands. The constructed libraries have a minimum of 10⁹ individual clones.

***Construction of cDNA libraries:*** Libraries of phage displaying bacterial-derived polypeptides ARE constructed as described above for biased peptide libraries using cDNA derived from the bacterial source of choice. In order to obtain bacterial cDNA, bacterial mRNA is isolated and reversed-transcribed into cDNA. A PCR-ready single-stranded cDNA library made from total RNA of *E. col*i strain C600 is commercially available (Qbiogene). 10-mer degenerate oligonucleotides are employed as universal primer to synthesize the second strand of the *E. coli* cDNA. The amplified products are size-selected (ranging from 500 bp to 2 kb), excised and eluted from 1% agarose gel, and ligated into the T7Select10-3b vector (Novagen), which can accommodate proteins up to 1200 amino acids in length.

***Construction of constrained cyclic peptide libraries:*** Two constrained cyclic peptide phage display libraries whose variable regions possess the following amino acid structure: C-X₇-C (cyclic 7-mer) and C-X₁₀-C (cyclic 10-mer), where C is a cysteine and X is any residue, were created. For each library, the variable region was generated using an extension reaction.

Random oligonucleotides were ordered PAGE purified from The Midland Certified Reagent Company. An EcoRI restriction enzyme site on the 5' end and a HindIII site on the 3' end were included for cloning purposes. In addition, the 3' end contained additional flanking nucleotides creating a "handle".

For the cyclic 10-mer inserts the random oligonucleotide was 5'-CAT GCC CGG AAT T CC **TGC NNK NNK NNK NNK NNK NNK NNK NNK NNK NNK** TGC GGA GGA GGA T AA AAG CTT TCG AGA C-3' (SEQ ID NO: 80).

For the cyclic 7-mer inserts the random oligonucleotide was 5'-CAT GCC CGG AAT TCC **TGC NNK NNK NNK NNK NNK NNK NNK** TGC GGA GGA GGA TAA AAG CTT TCG AGA C-3' (SEQ ID NO:81).

For both oligonucleotides the 5' EcoRI and 3' HindIII sites are indicated by underlining and the variable region of the insert and nucleotides encoding the flanking cysteine residues are in bold. Amino acids in the variable region are encoded by NNK, where N=A/T/G/C and K=G/T. This nucleotide configuration reduces the number of possible codons from 64 to 32 while preserving the relative representation of each amino acid. In addition, the NNK configuration reduces the number of possible stop codons from three to one.

A universal oligonucleotide, 5'-GTC TCG AAA GCT TTT ATC CTC C'3' (SEQ ID NO: 28) containing a HindIII site (underlined) was ordered PAGE purified from The Midland Certified Reagent Company. This universal oligonucleotide was annealed to the 3' "handle" serving as a primer for the extension reaction. The annealing reaction was performed as follows: 5 µg of random oligonucleotide were mixed with 3 molar equivalents of the universal primer in dH₂0 with 100mM NaCl. The mixture was heated to 95°C for two minutes in a heat block. After that time, the heat block was turned off and allowed to cool to room temperature.

The annealed oligonucleotides were then added to an extension reaction mediated by the Klenow fragment of DNA polymerase I (New England Biolabs). The extension reaction was performed at 37°C for 10 minutes, followed by an incubation at 65°C for 15 minutes to inactivate the Klenow. The extended duplex was digested with 50U of both EcoRI (New England Biolabs) and HindIII (New England Biolabs) for 2 hours at 37°C. The digested products were separated by polyacrylamide gel electrophoresis, the bands of the correct size were excised from the gel, placed in 500µl of elution buffer (10mM magnesium acetate, 0.1%SDS, 500mM ammonium acetate) and incubated overnight, with shaking, at 37°C. The following day the eluted DNA was purified by phenol:chloroform extraction followed by a standard ethanol precipitation.

The purified insert was ligated into T7 Select Vector arms (Novagen; cat. # 70548), using 0.6 Weiss Units of T4 DNA ligase (New England Biolabs). The entire ligation reaction was added to T7 Packaging Extract as per manufacturer's protocol (Novagen; cat. #70014). Using the bacterial strain 5615 (Novagen), the titer of the initial library was determined by a phage plaque assay (Novagen; T7Select System). Both the 7-mer and 10-mer cyclic peptide libraries have 5x10⁸ individual clones which approaches the upper achievable limit of the phage display system.

### Results and Discussion

A variety of phage display libraries are constructed for use in the screening assay to identify novel polypeptide TLR ligands. Such libraries include: 1) biased peptide libraries, which may be used to identify functional peptide TLR ligands within known polypeptide sequences; 2) random peptide libraries, which may be used to identify functional TLR ligands among randomly generated peptide sequences of between 5 and 30 amino acids in length; and 3) cDNA libraries, which may be used to identify functional TLR ligands from a microorganism of choice, *e*.*g*., the bacterium *E*. *coli;* and contrained cyclic peptide libraries, which contain random peptide sequences whose 3-dimensional conformation is restricted by cyclization via di-sulfidde bonds between flanking cysteine residues.

### EXAMPLE 3: SCREENING ASSAY FOR PEPTIDE TLR LIGANDS

### Materials and Methods

***Screening of phage display libraries by biopanning:*** Phage display libraries are screened for peptide TLR ligands according to the following procedure. The phage display library is incubated on an *in vitro* cultured monolayer of cells that express minimal amounts of the TLR of interest (TLR^{lo}) in order to reduce non-specific binding, and then transferred to an *in vitro* suspension culture of cells expressing the relevant TLR (TLR^{hi}) to capture phage with binding specificity for the target TLR. After several washes with PBS to remove phage remaining unbound to the TLR^{hi} cells, TLR^{hi} cell-bound phages are harvested by centrifugation. The TLR^{hi} cells with bound phage are incubated with *E*.*coli* (strain BLT5615) in order to amplify the phage. This process is repeated three or more times to yield a phage population enriched for high affinity binding to the target TLR.

In each round of biopanning, the harvested phage that are bound to TLR^{hi} cells can be titred prior to amplification, amplified, and then titred again prior to initiation of the next cycle of biopanning. In this way, it is possible to determine the percent (%) of input phage in each cycle that are ultimately harvested from the TLR^{hi} cells. This calculation provides a round-by-round measure of enrichment within the phage display library for phage that display TLR-binding peptides.

Individual phage clones from the enriched pool are isolated, *e*.*g*., via plaque formation in *E. coli.*

### Results and Discussion

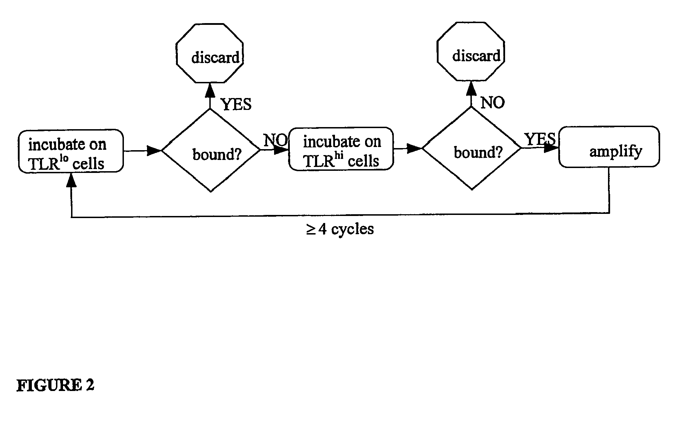

Phage display libraries are enriched for those clones that display peptides that specifically mediate TLR-binding by negative-positive panning as outlined in Figure 2. Each cycle of panning consists of negative and positive panning as follows: the phage display library is incubated on a monolayer of cells that express minimal amounts of the TLR of interest (TLR^{lo}) in order to reduce non-specific binding; 2) the portion of the library that remains unbound to the monolayer of TLR^{lo} cells is transferred to a monolayer of cells expressing the relevant TLR (TLR^{hi}) to capture phage with binding specificity for the target TLR; 3) after several washes to remove phage remaining unbound to the monolayer of TLR^{hi} cells, bound phages are harvested by hypotonic shock of the cell monolayer; and 4) the harvested phage are amplified. This process is repeated three or more times to yield a phage population enriched for high affinity binding to the target TLR.

Individual phage clones from the enriched pool are isolated, e.g., via plaque formation in E. *coli.* These individual clones contain nucleotide sequences encoding for polypeptides that specifically bind to the TLR of choice.

### EXAMPLE 4: SCREENING ASSAY FOR PEPTIDE TLR5 LIGANDS

### Materials and Methods

***Generation of phage displaying a polypeptide TLR5 ligand:*** The coding region of the *E. coli* flagellin (*fliC*) gene (SEQ ID NO: 33) was cloned into the T7SELECT phage display vector (Novagen). Double stranded DNA encoding E. *coli* fliC was ligated to the T7Select 10-3 bacteriophage vector (Novagen). The ligation reactions were packaged *in vitro* and titred using the host *E*. *coli* strain BLR5615 that was grown in M9TB (Novagen). The recombinant phage was then amplified. Ligation, packaging, and amplification were performed according to manufacturer's instructions.

***Generation of phage displaying an S-Tag polypeptide:*** The S-tag nucleotide sequence and amino acid sequences are set forth in SEQ ID NO: 35 and SEQ ID NO: 36, respectively. Double stranded DNA encoding the S-tag peptide sequence was ligated to the T7Select 10-3 bacteriophage vector (Novagen). The ligation reactions were packaged *in vitro* and titred using the host *E*. *coli* strain BLR5615 that was grown in M9TB (Novagen). The recombinant phage was then amplified. Ligation, packaging, and amplification were performed according to manufacturer's instructions. In order to simulate a random peptide library, 10³ fliC phages were mixed with 10¹⁰ S-tag phages (10⁻⁷ dilution).

***NF-κB-dependent luciferase reporter assay:*** Parental 293 cells and 293.hTLR5 cells (see Example 1, above) were incubated with an aliquot of fliC-expressing T7SELECT phage, or S-tag expressing T7SELECT phage, for four to five hours at 37°C. As a negative control, cells were incubated with medium alone. NF-κB-dependent luciferase activity was measured using the Steady-Glo Luciferase Assay System by Promega (E2510), following the manufacturer's instructions. Luminescence was measured on a microplate luminometer (FARCyte, Amersham) and expressed as relative luminescence units (RLU) after subtracting the background reading obtained by exposing cells to the DMEM medium alone.

### Results and Discussion

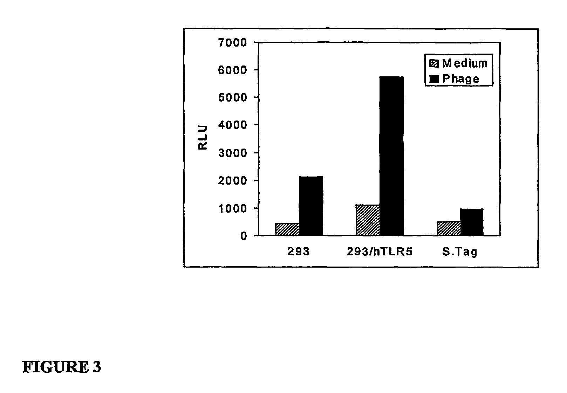

To verify the utility of the screening assay to identify TLR-binding polypeptides, we cloned the *E. coli* flagellin gene (fliC) into the T7SELECT phage display vector, expressed the protein in T7 phage, and examined binding of the recombinant fliC-phage to the cognate receptor, TLR5. The recombinant fliC-phage were incubated on parental HEK293 cells containing an NF-κB-dependent luciferase reporter construct (293) or on TLR5-overexpressing HEK293 cells containing an NF-κB-dependent luciferase reporter construct (293.hTLR5, see Example 1, above), and luciferase activity was measured. The data shown in Figure 3 demonstrate that phage displaying fliC on their surface can bind to and activate TLR5. Moreover, the activation of the reporter gene correlates with over-expression of the appropriate TLR (*i*.*e*., TLR5).

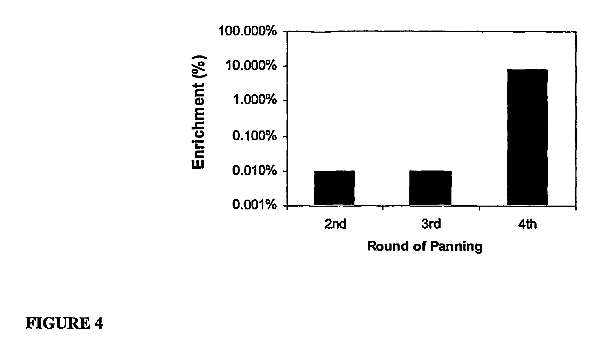

In order to simulate a random peptide library, 10³ fliC phages were mixed with 10¹⁰ control S-tag phages (10⁻⁷ dilution) and screened by biopanning as described in Example 3. For this screen, the Tor^{lo} cells were parental HEK293 (TLR5⁻) cells, and the TLR^{hi} cells were HEK293 cells ectopically expressing human TLR5 (293.hTLR5, see Example 1, above). Phage bound to 293.hTLR5 cells were harvested, titred, and amplified prior to initiation of each cycle of panning. In this way, it was possible to determine the % of input phage in each cycle that was ultimately harvested from the TLR5^{hi} cells. Results of the iterative negative-positive panning procedure are shown in Figure 4. The data clearly show that it is feasible to isolate TLR5-binding phage by this biopanning strategy.

### EXAMPLE 5: SCREENING ASSAY FOR PEPTIDE TLR2 LIGANDS

### Materials and Methods

***Construction of random peptide libraries (RPL):*** A pentameric random peptide phage display library of T7SELECT phage was constructed essentially as described in Example 2. A pair of phosphorylated oligonucleotides with the sequence NNBNNBNNBNNBNNB (where N=A/G/C/T, B=G/C/T) flanked at the 5' and 3' ends by EcoRI and HindIII sites, respectively, was synthesized. Equimolar amounts of the oligonucleotides were annealed by heating for 5 min at 90°C with gradual cooling to 25°C. The double stranded DNA was ligated to T7Select 10-3 bacteriophage vector (Novagen) that had been previously digested with *Eco*RI and *Hind*III. The ligation reactions were packaged *in vitro* and titred using the host *E*. *coli* strain BLR5615 that was grown in M9TB (Novagen), generating 2.5 x 10⁷ clones, representing about 75% coverage of the library. The recombinant phage were subjected to several rounds of amplification to generate a total library of 1.35 x 10¹² phage, ensuring representation in excess of 5 x 10⁴ fold for each clone in the library.

Libraries of phage displaying random peptides 10, 15 and 20 amino acids in length were constructed essentially as described for the pentameric random peptide library, except that the phosphorylated oligonucleotides used were 30, 45, and 60 nucleotides in length, respectively.

***Sequencing of phage inserts:*** Individual phage clones from the enriched pool are isolated via plaque formation in *E*. *coli.* The DNA inserts of individual phage are amplified in PCR using the commercially available primers T7SelectUP (5' - GGA GCT GTC GTA TTC CAG TC-3'; SEQ ID NO: 37; Novagen, catalog # 70005) and T7SelectDOWN (5'-AAC CCC TCA AGA CCC GTT TA-3'; SEQ ID NO: 38; Novagen, catalog # 70006). The PCR product DNA is purified using the QIAquick 96 PCR Purification Kit (Qiagen) and subjected to DNA sequencing using T7SelectUP and T7SelectDOWN primers.

***Peptide synthesis:*** The synthetic monomer of the DPDSG motif, as well a concatemerized copy (DPDSG)₅ peptides were manufactured using solid phase synthesis methodologies and FMOC chemistry.

***NF-κB-dependent luciferase reporter assay:*** Parental 293 cells and 293.hTLR2 cells (see Example 1, above) were incubated with an aliquot of test peptide four to five hours at 37°C. NF-κB-dependent luciferase activity was measured using the Steady-Glo Luciferase Assay System by Promega (E2510), following the manufacturer's instructions. Luminescence was measured on a microplate luminometer (FARCyte, Amersham) and expressed as relative luminescence units (RLU) after subtracting the background reading obtained by exposing cells to the DMEM medium alone.

### Results and Discussion

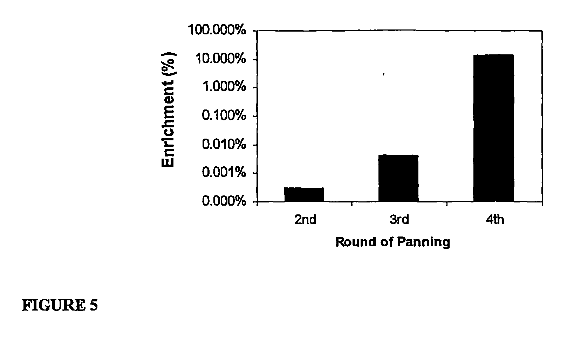

We constructed a pentameric random peptide phage display library in T7 phage. This phage library was then screened by biopanning as described in Example 3. For this screen, the TLR^{lo} cells were parental HEK293 (TLR2⁻) cells, and the TLR^{hi} cells were HEK293 cells ectopically expressing human TLR2 (293.hTLR2, see Example 1, above). Phage bound to 293.hTLR2 cells were harvested, titred, and amplified prior to initiation of the each cycle of panning. In this way, it was possible to determine the % of input phage in each cycle that was ultimately harvested from the TLR2^{hi} cells. Figure 5 shows that the biopanning assay results in considerable enrichment after each iteration.

After 4 rounds of biopanning, individual phage clones from the enriched pool were isolated via plaque formation in *E*. *coli.* 109 phage clones were randomly picked for sequencing. Of the 109 individual clones examined the motif DPDSG was predominant (see Tables 6 and 7). Homology search using tBLAST algorithm reveals that a majority (58%) of the novel sequences identified display a perfect match to various bacterial proteins of the database (See Table 6). Notable among these proteins are flagellin modification protein (FImB) of *Caulobacter crescentus,* type 4 fimbrial biogenesis protein (PilX) of *Pseudomonas*, adhesin of *Bordetella*, and OmpA-related protein of *Xantomonas*. The rest of the sequences (42%) show no obvious homology to any known protein (See Table 7).

**Table 6: Peptide TLR2 ligands which show identity to known microbial proteins. % abundance = percentage of all clones sequenced (n = 109) having given peptide sequence.**

| **PEPTIDE** | **SEQ ID NO** | **% Abundance** | **Homology** |
|---|---|---|---|
| DPDSG | 5 | 46.8 | flagellin modification protein FlmB of *Caulobacter crescentus* |
| IGRFR | 6 | 2.7 | Bacterial Type III secretion system protein |
| MGTLP | 7 | 1.8 | invasin protein of *Salmonella* |
| ADTHQ | 8 | 0.9 | Type 4 fimbrial biogenesis protein (PilX) of *Pseudomonas* |
| HLLPG | 9 | 0.9 | *Salmonella* SciJ protein |
| GPLLH | 10 | 0.9 | putative integral membrane protein of *Streptomyces* |
| NYRRW | 11 | 0.9 | membrane protein of *Pseudomonas* |
| LRQGR | 12 | 0.9 | adhesin of *Bordetella pertusis* |
| IMWFP | 13 | 0.9 | peptidase B of *Vibrio cholerae* |
| RVVAP | 14 | 0.9 | virulence sensor protein of *Bordetella* |
| IHVVP | 15 | 0.9 | putative integral membrane protein of *Neisseria meningitidis* |
| MFGVP | 16 | 0.9 | fusion of flagellar biosynthesis proteins FliR and FlhB of *Clostridium* |
| CVWLQ | 17 | 0.9 | outer membrane protein (porin) of *Acinetobacter* |
| IYKLA | 18 | 0.9 | flagellar biosynthesis protein, FlhF of *Helicobacter* |
| KGWF | 19 | 0.9 | ompA related protein of *Xanthomonas* |
| KYMPH | 20 | 0.9 | omp2a porin of *Brucella* |
| VGKND | 21 | 0.9 | putative porin/fimbrial assembly protein (LHrE) of *Salmonella* |
| THKPK | 22 | 0.9 | wbdk of *Salmonella* |
| SHIAL | 23 | 0.9 | Glycosyltransferase involved in LPS biosynthesis |
| AWAGT | 24 | 0.9 | *Salmonella* putative permease |

**Table 7: Peptide TLR2 ligands which show no homology to known proteins. % abundance = percentage of all clones sequenced (n = 109) having given peptide sequence.**

| **PEPTIDE** | **SEQ ID NO** | % **ABUNDANCE** |
|---|---|---|
| NPPTT | 54 | 0.92% |
| MRRIL | 55 | 0.92% |
| MISS | 56 | 0.92% |
| RGGSK | 57 | 3.67% |
| RGGF | 58 | 0.92% |
| NRTVF | 59 | 0.92% |
| NRFGL | 60 | 0.92% |
| SRHGR | 61 | 0.92% |
| IMRHP | 62 | 0.92% |
| EVCAP | 63 | 0.92% |
| ACGVY | 64 | 0.92% |
| CGPKL | 65 | 0.92% |
| AGCFS | 66 | 0.92% |
| SGGLF | 67 | 0.92% |
| AVRLS | 68 | 0.92% |
| GGKLS | 69 | 0.92% |
| VSEGV | 70 | 3.67% |
| KCQSF | 71 | 0.92% |
| FCGLG | 72 | 0.92% |
| PESGV | 73 | 0.92% |

The biological activity of the TLR2-binding peptides isolated by the screening method was confirmed using the isolated peptides in an NF-κB-dependent reporter gene assay. For this assay, a synthetic monomer of the DPDSG motif (SEQ ID NO: 5), or a concatemerized copy (DPDSG)₅, was incubated on parental HEK293 cells containing an NF-κB-dependent luciferase reporter construct (293) and on TLR2-overexpressing HEK293 cells containing an NF-κB-dependent Luciferase reporter construct (293.hTLR2, see Example 1, above). Luciferase activity was then measured. This assay showed that both the synthetic monomer of the DPDSG motif and the concatemerized copy (DPDSG)₅ activated luciferase reporter gene expression in a TLR2-dependent manner. Thus, the TLR2-binding peptides identified by the screening assay are functional peptide TLR2 ligands.

We also constructed 10, 15, and 20 amino acid random peptide phage display libraries in T7SELECT phage. These phage display libraries were pooled in equal proportion and then screened by biopanning as described in Example 3. For this screen, the TLR^{lo} cells were parental HEK293 (TLR2⁻) cells, and the TLR^{hi} cells were HEK293 cells ectopically expressing human TLR2 and human CD14 (293.hTLR2.hCD14, see Example 1, above). After 4 rounds of biopanning, the enriched phage population was cloned by plaque formation in *E*. *coli,* and 96 clones were randomly picked for sequencing. Of the 96 clones analyzed three peptide sequences were particularly abundant (see Table 8). Homology search using tBLAST algorithm revealed that these peptide sequences show no obvious homology to any known protein. These novel peptide sequences share a common feature, in that all contain a high percentage (≥30%) of positively charged amino acids.

**Table 8: Peptide TLR2 ligands which show no homology to known proteins. % abundance = percentage of all clones sequenced having given peptide sequence.**

| **PEPTIDE** | **SEQ ID NO** | **% Abundance** | **POSITIVELY CHARGED AA (%)** |
|---|---|---|---|
| KGGVGPVRRSSRLRRTTQPG | 25 | 33.3 | 6/20 (30%) |
| GRRGLCRGCRTRGRIKQLQSAHK | 26 | 16.1 | 9/23 (39%) |
| RWGYHLRDRKYKGVRSHKGVPR | 27 | 19.4 | 10/22 (45%) |

### EXAMPLE 6: IN VITRO TRANSCRIPTION AND TRANSLATION OF THE NOVEL TLR2 POLYPEPTIDE LIGANDS

### Materials and Methods

***Generation of DNA inserts by PCR:*** Individual T7SELECT phage clones from the enriched pool are isolated via plaque formation in *E*. *coli.* The individual T7SELECT phage clones are dispensed in a 96-well plate, which serves as a master plate. Duplicate samples are subjected to PCR using phage specific primers, T7FOR (5'-GAA TTG TAA TAC GAC TCA CTA TAG GGA GGT GAT GAA GAT ACC CCA CC-3'; SEQ ID NO: 41), and T7REV (5'-TAA TAC GAC TCA CTA TAG GGC GAA GTG TAT CAA CAA GCT GG-3'; SEQ ID NO: 42) that flank the phage inserts. The forward primer is about 600 bp away from the insert and is designed to incorporate the T7 promoter upstream of the Kozak sequence (KZ), which is critical for optimal translation of eukaryotic genes, and a 6X HIS-tag sequence. The reverse primer includes the myc sequence at the c-terminus of the peptide. Therefore, the PCR product will contain all the signals necessary for optimal transcription and translation (T7 promoter, Kozak sequence and the ATG initiation codon), as well as and sequences encoding an N-terminal 6X HIS tag and a C-terminal myc tag for capture, detection and quantitation of the translated protein. The PCR products are purified using the QIAquick 96 PCR Purification Kit (Qiagen).

**In vitro. *TNT:*** Rabbit reticulocyte lysate is programmed with the PCR DNA using TNT T7 Quick for PCR DNA kit (Promega), which couples transcription to translation. To initiate a TNT reaction, the DNA template is incubated at 30°C for 60-90 min in the presence of rabbit reticulocyte lysate, RNA polymerase, amino acid mixture and RNAsin ribonuclease inhibitor.

***Immunoanalysis of the* in vitro *translated protein:*** Immunoanalysis is used to confirm translation of the polypeptide TLR ligand. In these assays, an aliquot of the TNT reaction is analyzed by western blot using antibodies specific for one of the engineered tags, or by ELISA to allow normalization for protein levels across multiple samples. For a sandwich ELISA, 6X HIS-tagged protein is captured on Ni-NTA microplates and detected with an antibody to one of the heterologous tags (*i*.*e*., anti-c-myc).

***NF-κB-dependent luciferase reporter assay:*** An aliquot of the *in vitro* synthesized peptide is monitored for the ability to activate an NF-κB-dependent luciferase reporter gene in cell lines expressing the target TLR. Cells stably transfected with an NF-κB luciferase reporter construct may constitutively express the appropriate TLR, or may be engineered to overexpress the TLR of choice. Cells seeded in a 96-well microplate are exposed to test peptide for four to five hours at 37°C. NF-κB-dependent luciferase activity is measured using the Steady-Glo Luciferase Assay System by Promega (E2510), following the manufacturer's instructions. Luminescence is measured on a microplate luminometer (FARCyte, Amersham). Specific activity of test compound is expressed as the EC₅₀, *i*.*e*., the concentration which yields a response that is 50% of the maximal response obtained with the appropriate control reagent, such as LPS. The EC₅₀ values are normalized to protein concentration as determined in the ELISA described above.

***Dendritic cell activation assay:*** For this assay murine or human dendritic cell cultures are obtained. Murine DCs are generated *in vitro* as previously described (Lutz et al. J Immun Meth. 1999;223:77-92). In brief, bone marrow cells from 6-8 week old C57BU6 mice are isolated and cultured for 6 days in medium supplemented with 100 U/ml GMCSF (Granulocyte Macrophage Colony Stimulating Factor), replenishing half the medium every two days. On day 6, nonadherant cells are harvested and resuspended in medium without GMSCF and used in the DC activation assay. Human DCs are obtained commercially (Cambrex, Walkersville, MD) or generated *in vitro* from peripheral blood obtained from healthy donors as previously described (Sallusto & Lanzavecchia. J Exp Med 1994; 179:1109-1118). In brief, peripheral blood mononuclear cells (PBMC) are isolated by Ficoll gradient centrifugation. Cells from the 42.5-50% interface are harvested and further purified following magnetic bead depletion of B- and T- cells using antibodies to CD19 and CD2, respectively. The resulting DC enriched suspension is cultured for 6 days in medium supplemented with 100 U/ml GMCSF and 1000 U/ml IL-4 (interleukin-4). On day 6, nonadherant cells are harvested and resuspended in medium without cytokines and used in the DC activation assay. An aliquot of the *in vitro* synthesized fusion protein is added to DC culture and the cultures are incubated for 16 hours. Supernatants are harvested, and cytokine (IFNγ, TNFα, IL-12 p70, IL-10 and IL-6) concentrations are determined by sandwich enzyme-linked immunosorbent assay (ELISA) using matched antibody pairs from BD Pharmingen or R&D Systems, following the manufacturer's instructions. Cells are harvested, and costimulatory molecule expression (*e*.*g*., B7-2) is determined by flow cytometry using antibodies from BD Pharmingen or Southern Biotechnology Associates following the manufacturer's instructions. Analysis is performed on a Becton Dickinson FACScan running Cellquest software.

***Sequencing inserts* of *active phage:*** Those samples which test positive in the *in vitro* TNT cellular assays are traced back to the original master plate containing individual phage clones. The DNA inserts of positive clones are amplified in PCR using the primers T7FOR (5'-GAA TTG TAA TAC GAC TCA CTA TAG GGA GGT GAT GAA GAT ACC CCA CC-3'; SEQ ID NO: 43) and T7REV (5'-TAA TAC GAC TCA CTA TAG GGC GAA GTG TAT CAA CAA GCT GG-3'; SEQ ID NO: 44), or the commercially available primers T7SelectUP (5'- GGA GCT GTC GTA TTC CAG TC-3'; SEQ ID NO: 45; Novagen, catalog # 70005) and T7SelectDOWN (5'-AAC CCC TCA AGA CCC GTT TA-3'; SEQ ID NO: 46; Novagen, catalog # 70006). The DNA is purified and subjected to DNA sequencing using T7FOR and T7REV primers or T7SelectUP and T7SelectDOWN primers.

### Results and Discussion

We have performed *in vitro* TNT reactions on isolated T7SELECT phage expressing a novel polypeptide TLR2 ligand. The amount of protein produced by this method proved to be insufficient for detection in the TLR bioassays described above. Therefore, an alternate strategy, based upon ligase-independent cloning coupled with PCR from isolated phage expressing a polypeptide TLR2 ligand, was performed.

### EXAMPLE 7: LIGASE INDEPENDENT CLONING FOR IN VITRO ANALYSIS OF POLYPEPTIDE TLR2 LIGAND ACTIVITY

### Materials and Methods

***Ligase independent cloning:*** Clones of T7Select 10-3 bacteriophage vector (Novagen) containing nucleic acid inserts encoding the polypeptide TLR2 ligands were subjected to PCR to isolate the nucleotide sequences encoding the TLR2-binding peptides. PCR was performed using the primers T7-LICf (5'-GAC GAC GAC AAG ATT GAG ACC ACT CAG AAC AAG GCC GCA CTT ACC GAC C-3'; SEQ ID NO: 74) and T7-LICr (5'-GAG GAG AAG CCC GGT CTA TTA CTC GAG TGC GGC CGC AAG-3'; SEQ ID NO: 75) at 10 pmol each with phage lysate at 1:20 dilution using the Taq polymerase master mix (Invitrogen) at 1:2 dilution. PCR cycling conditions were as follows: denaturation at 95°C for 5min; 30 cycles of denaturation step at 95°C for 30 sec, annealing step at 58°C for 30 sec, and extension at 72°C for 30 sec; and a final extension at 72°C for 10 min.

These sequences were then cloned into the pET-LIC24 and pMTBip-LIC vectors via ligase independent cloning (LIC). For LIC, an -800 bp PCR fragment, which includes a portion of the the phage coat protein encoding sequence to facilitate expression and purification, was treated with T7 DNA polymerase in the presence of dATP and cloned into the linearized pET-LIC24 vector.

To construct the pET-LIC24 vector, an unique *Bse*RI site was introduced into pET24a (Novagen). In order to introduce the *Bse*RI site the 5'-phosphorylated primers pET24a-LICf (5'-TAT GCA TCA TCA CCA TCA CCA TGA TGA CGA CGA CAA GAG CCC GGG CTT CTC CTC AGC-3'; SEQ ID NO: 76) and pET24a-LIC-r (5'-TCA GCT GAG GAG AAG CCC GGG CTC TTG TCG TCG TCA TCA TGG TGA TGG TGA TGA TGC A-3'; SEQ ID NO: 77) were annealed and cloned into *Nde*I and *Bpu*1102I digested pET24a via cohesive end ligation. The resulting construct was then digested with *Bse*RI and treated with T4 DNA polymerase in the presence of dTTP to generate pET-LIC24 vector.

pMT-Bip-LIC was constructed in the same way as pET-LIC24 by inserting an annealed oligo into *Bgl*II and *Mlu*I digested vector pMTBip/V5-HisA. (Invitrogen). The annealed oligo was made using the 5'-phosphorylated primers pMTBip-LICf (5'-GAT CTC ATC ATC ACC ATC ACC ATG ATG ACG ACG ACA AGA GCC CGG GCT TCT CCT CAA-3'; SEQ ID NO: 78) and pMTBip-LICr (5'-CGC GTT GAG GAG AAG CCC GGG CTC TTG TCG TCG TCA TCA TGG TGA TGG TGA TGA TGA-3'; SEQ ID NO: 79).

***Protein expression in* E. coli:** *E. coli* strain BLR (DE3) pLysS strain (Invitrogen) is transformed with pET-LIC plasmid DNA using a commercially available kit (Qiagen). A colony is inoculated into 2 mL LB containing 100 µg/ml carbenicillin, 34 µg/ml chloramphenicol, and 0.5% glucose and grown overnight at 37°C with shaking. A fresh 2 mL culture is inoculated with a 1:20 dilution of the overnight culture and grown at 37°C for several hours until OD₆₀₀ = 0.5-0.8. Protein expression is induced by the addition of IPTG to 1 mM for 3 hours.

***Ni-NTA protein purification:** E. coli* cells transformed with the construct of interest were grown and induced as described above. The cells were harvested by centrifugation (7000 rpm x 7 minutes in a Sorvall RC5C centrifuge) and the pellet re-suspended in lysis Buffer B (100 mM NaH2P04, 10 mM Tris-HCl, 8 M urea, pH 8 adjusted with NaoH) and 10 mM imidazol. The suspension was freeze-thawed 4 times in a dry ice bath. The cell lysate was centrifuged (40,000 g for one hour in a Beckman Optima L ultracentrifuge) to separate the soluble fraction from inclusion bodies. The supernatant was mixed with 1ml Ni-NTA resin (Qiagen Ni-NTA) that had been equilibrated with buffer B and binding of the proteins was allowed to proceed at 4°C for 2-3 hours on a roller. The material was then loaded unto a 1cm-diameter column. The bound material was then washed 2 times with 30 mL wash buffer (Buffer B + 20mM imidazol). The proteins were eluted in two rounds with 3 mL elution buffer twice (Buffer B+250mM imidazol). The eluates were combined and the pools were used to perform a serial dialysis starting with 1 L of buffer (Buffer B + 250 mM imidazol:2x PBS in a ratio of 1:1) with change in buffer every 4-8 hours. The final dialysis step was performed with two changes of PBS overnight. The integrity of the proteins was verified by SDS-PAGE and immunoblot.

Greater than 95% purity can be achieved. Optionally, to further reduce endotoxin contamination, the protein is chromatographed through Superdex 200 gel filtration in the presence of 1% deoxycholate to separate protein and endotoxin. A second round of Superdex 200 gel filtration in the absence of deoxycholate removes the detergent from the protein sample. Purified protein is concentrated and dialyzed against 1x PBS, 1% glycerol. The protein is aliquoted and stored at -80°C.

***Protein expression in* Drosophila *S-2 cells:*** The pMTBip-LIC vectors are used to direct recombinant peptide expression in *Drosophila* S-2 cells. Conditioned medium from S-2 cells expressing the recombinant peptide may be directly used in bioassays to confirm the activity of the TLR-binding peptide. Drosophila S-2 cells and the Drosphila Expression System (DES) complete kit is obtained from Invitrogen (catalog#: K5120-01, K4120-01, K5130-1 and K4130-01). The growth and passaging of the S-2 cells, transfection and harvesting of the conditioned medium are performed according to manufacturer's protocol.

In **vitro** *IL-8 assay:* Parental 293 cells and 293.hTLR2.hCD14 cells (see Example 1, above) are seeded in 96-well microplates (50,000 cells/well), and aliquots of either purified recombinant peptide expressed in *E*. *coli* or conditioned medium from S-2 cells expressing recombinant peptide are added. As a positive control, parental 293 cells and 293.hTLR2.hCD14 cells are incubated with the PAMP tripalmitoyl-cystein-seryl-(lysyl)3-lysine (Pam3Cys; *e*.*g*. Sigma-Aldrich). The microplates are then incubated overnight. The next day, the conditioned medium is harvested, transferred to a clean 96-well microplate, and frozen at -20°C. After thawing, the conditioned medium is assayed for the presence of IL-8 in a sandwich ELISA using an anti-human IL-8 matched antibody pair (Pierce, catalog # M801E and # M802B) following the manufacturer's instructions. Optical density is measured using a microplate spectrophotometer (FARCyte, Amersham).

### Results and Discussion

Clones of T7SELECT phage containing nucleic acid inserts encoding the peptide sequences of Table 9 were subjected to ligase independent cloning into a pET-LIC expression vector.

**Table 9: Peptide TLR2 ligand sequences subjected to ligase independent cloning (LIC) and recombinantly expressed in E. coli.**

| **PEPTIDE** | **SEQ ID NO** | **Recombinant protein ID#** |
|---|---|---|
| KGGVGPVRRSSRLRRTTQPG | 25 | m#1 |
| GRRGLCRGCRTRGRIKQLQSAHK | 26 | ID#2 |
| RWGYHLRDRKYKGVRSHKGVPR | 27 | ID#3 |

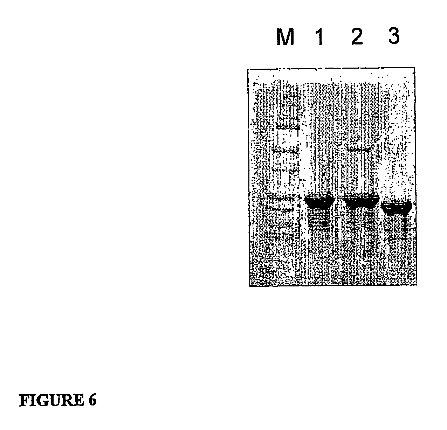

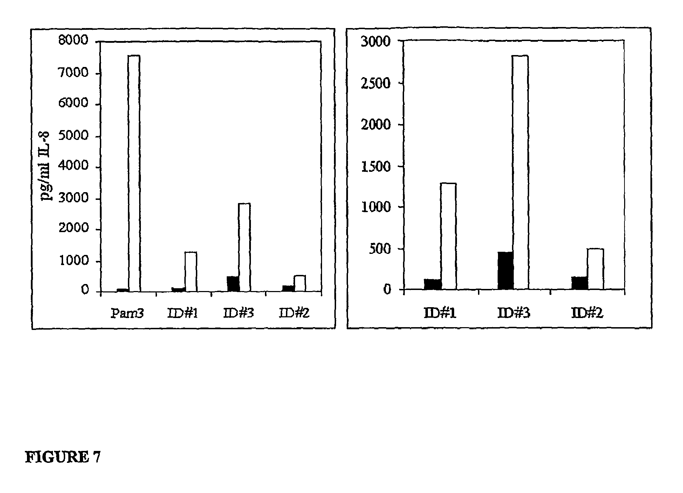

The pET-LIC vector was then used to direct recombinant peptide expression in *E*. *coli* host cells. The expressed peptides, which contain a His tag, were then purified on a Ni-NTA resin (see Figure 6). These purified peptides were used in an IL-8 induction assay (see Figure 7). The results of this assay clearly show that the novel polypeptides induce IL-8 production in a TLR2-dependent manner. Thus the polypeptides are functional peptide TLR2 ligands.

### EXAMPLE 8: A POLYPEPTIDE TLR2-LIGANII:LISTERL4 LLO-p60 ANTIGEN FUSION PROTEIN VACCINE

### Materials and Methods

***Cloning of novel TLR ligands into* E. coli:** Double stranded DNA encoding the polypeptide TLR2 ligands is ligated upstream of sequences encoding a fusion protein of antigenic MHC class I and II epitopes of *L. monocytogenes* proteins LLO and p60. The amino acid sequence of the LLO-p60 fusion protein is given in SEQ ID NO: 39. These ligated sequences encoding a polypeptide TLR2 ligand:*Listeria* LLO-p60 antigen fusion protein are inserted into a plasmid expression vector. The expression construct is engineered by using convenient restriction enzyme sites or by PCR.

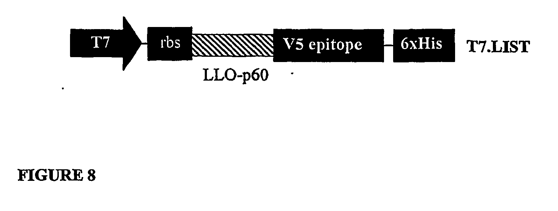

For example, sequences encoding the polypeptide TLR2 ligands are inserted upstream of the LLO-p60 encoding sequence in the expression construct T7.LIST (Figure 8), where T7.LIST is assembled as described below. In this case, the expressed fusion protein will contain both a V5 epitope and a 6xHis tag.

***Generation of the T7.LIST plasmid:*** Sequences encoding the *Listeria* LLO-p60 antigen fusion protein are isolated as follows: First primers LLOF7 (5'-CTT AAA GAA TTC CCA ATC GAA AAG AAA CAC GCG GAT G-3'; SEQ ID NO: 47) and LLOR3 (5'-TTC TAC TAA TTC CGA GTT CGC TTT TAC GAG-3'; SEQ ID NO: 48) are used to amplify a 5' portion of the LLO sequences. Next primers LLOF6 (5'-CTC GTA AAA GCG AAC TCG GAA TTA GTA GAA-3'; SEQ ID NO: 49) and P60R7 (5' AGA GGT CTC GAG TGT ATT TGT TTT ATT AGC ATT TGT G-3'; SEQ ID NO: 50) are used to amplify the remaining fused 3' portion LLO sequences and the p60 sequences. These two PCR fragments are then joined by a third PCR using the primers LLOF7 and P60R7. This PCR serves to mutate the LLO sequence spanned by LLOR3 and LLOF6 so as to remove the EcoRI site. This product is then ligated into the pCRT7CT-TOPO cloning vector (Invitrogen) to generate the T7.LIST plasmid. In this vector, the chimeric DNA insert is driven by the strong T7 promoter, and the insert is fused in frame to the V5 epitope (GKPIPNPLLGLDST; SEQ ID NO: 40) and polyhistidine (6x His) is located at the 3' end of the gene (see Figure 8).

***Protein expression and immunoblot assay:*** In general, the following protocol is used to produce recombinant polypeptide TLR2 ligand:*Listeria* LLO-p60 antigen: fusion protein. *E. coli* strain BL (DE3) pLysS strain (Invitrogen) is transformed with the desired plasmid DNA using a commercially available kit (Qiagen). A colony is inoculated into 2 mL LB containing 100 µg/ml carbenicillin, 34 µg/ml chloramphenicol, and 0.5% glucose and grown overnight at 37°C with shaking. A fresh 2 mL culture is inoculated with a 1:20 dilution of the overnight culture and grown at 37°C for several hours until OD₆₀₀ = 0.5-0.8. Protein expression is induced by the addition of IPTG to 1 mM for 3 hours. The bacteria are harvested by centrifugation and the pellet is re-suspended in 100 µl of 1x SDS-PAGE sample buffer in the presence of β-mercaptoethanol. The samples are boiled for 5 minutes and 1/10 volume of each sample is loaded onto 10% SDS-PAGE gel and electrophoresed. The samples are transferred to PVDF membrane and probed with α-His antibody (Tetra His, Qiagen) at 1:1000 dilution followed by rabbit anti-mouse IgG/AP conjugate (Pierce) at 1:25,000. The immunoblot is developed using BCIP/NBT colometric assay kit (Promega).

***Protein purification:*** Polypeptide TLR2 ligand:*Listeria* LLO-p60 antigen fusion proteins are expressed with a 6X Histidine tag to facilitate purification. *E. coli* cells transformed with the construct of interest are grown and induced as described above. Cells are harvested by centrifugation at 7,000 rpm for 7 minutes at 4°C in a Sorvall RC5C centrifuge. The cell pellet is resuspended in Buffer A (6 M guanidine HCl, 100 mM NaH₂PO₄, 10 mM Tris-HCl, pH 8.0). The suspension can be frozen at -80°C if necessary. Cells are disrupted by passing through a microfluidizer at 16,000 psi. The lysate is centrifuged at 30,000 rpm in a Beckman Coulter Optima LE-80K Ultracentrifuge for 1 hour. The supernatant is decanted and applied to Nickel-NTA resin at a ratio of 1ml resin/1L cell culture. The clarified supernatant is incubated with equilibrated resin for 2-4 hours by rotating. The resin is washed with 200 volumes of Buffer A. Non-specific protein binding is eliminated by subsequent washing with 200 volumes of Buffer B (8 M urea, 100 mM NaH₂PO₄, 10 mM Tris-HCl, pH 6.3). An additional 200 volume wash with buffer C (10 mM Tris-HCl, pH 8.0, 60% iso-propanol) reduces endotoxin to acceptable level (< 0.1 EU/µg). Protein is eluted with Buffer D (8 M Urea, 100 mM NaH₂PO₄, 10 mM Tris-HCl, pH 4.5). Protein elution is monitored by SDS-PAGE or Western Blot (anti-His, anti-LLO and anti-p60). Greater than 95% purity can be achieved. Endotoxin level may be further reduced by chromatography through Superdex 200 gel filtration in the presence of 1% deoxycholate to separate protein and endotoxin. A second round of Superdex 200 gel filtration in the absence of deoxycholate removes the detergent from the protein sample. Purified protein is concentrated and dialyzed against 1x PBS, 1% glycerol. The protein is aliquoted and stored at -80°C.

***Endotoxin** assay:* Endotoxin levels in recombinant fusion proteins are measured using the QCL-1000 Quantitative Chromogenic LAL test kit (Bio Whittaker #50-648U), following the manufacturer's instructions for the microplate method.

***Confirmation of TLR activity in NF-κB luciferase reporter assays:*** Purified recombinant polypeptide TLR2 ligand:*Listeria* LLO-p60 antigen fusion proteins are assayed for TLR activity and selectively in the NF-κB-dependent luciferase assay as described above.

***Immunization:*** Recombinant polypeptide TLR2 ligand:*Listeria* LLO-p60 antigen fusion protein is suspended in phosphate-buffered saline (PBS), without exogenous adjuvant. BALB/c mice (n = 10-20 per group) are immunized by s.c. injection at the base of the tail or in the hind footpad. Initial dosages tested range from 0.5 µg to 100 µg/animal. Positive control animals are immunized with 10³ CFU of live *L. monocytogenes*, while negative control animals receive mock-immunization with PBS alone.

***Sublethal* L. monocytogenes *challenge:*** Seven days after immunization, BALB/c mice are infected by i.v. injection of 10³ CFU *L. monocytogenes* in 0.1 ml of PBS. Spleens and livers are removed 72 hours after infection and homogenized in 5 ml of sterile PBS + 0.05% NP-40. Serial dilutions of the homogenates are plated on BHI agar. Colonies are enumerated after 48 hours of incubation. These experiments are performed a minimum of 3 times utilizing 10-20 animals per group. Mean bacterial burden per spleen or liver are compared between treatment groups by Student's t-Test.

***Lethal* L. monocytogenes *challenge:*** Seven days after immunization, BALB/c mice are infected i.v. (10⁵ CFU) or p.o. (10⁹ CFU) with *L. monocytogenes* in 0.1 ml of PBS, and monitored daily until all animals have died or been sacrificed for humane reasons. Experiments are performed 3 times utilizing 10-20 animals per group. Mean survival times of different treatment groups are compared by Student's t-Test.

***Induction of antigen-specific T-cell responses:*** CD8 T-cell responses are monitored at specific time points following vaccination (*i*.*e*. day 7, 14, 30, and 120) by quantitating the number of antigen-specific γ-interferon (IFNγ) secreting cells using ELISPOT (R&D Systems). At varying time points post-vaccination, T-cells are isolated from the draining lymph nodes and spleens of immunized animals and cultured in microtiter plates coated with capture antibody specific for the cytokine of interest. Synthetic peptides corresponding to the K^{d}-restricted epitopes p60₂₁₇₋₂₂₅ and LLO₉₁₋₉₉ are added to cultures for 16 hours. Plates are washed and incubated with anti-IFNγ detecting antibodies as directed by the manufacturer. Similarly, CD4 responses are quantified by IL-4 ELISPOT following stimulation with the I-A^{d} restricted CD4 epitopes LLO₁₈₉₋₂₀₀, LLO₂₁₆₋₂₂₇, and p60₃₀₀₋₃₁₁. Antigen specific responses are quantified using a dissection microscope with statistical analysis by Student's t-Test. For quantitation of CD8 responses, it is also possible to utilize flow cytometric analysis of T-cell populations following staining with recombinant MHC Class I tetramer (Beckman Coulter) loaded with the H-2^{d} restricted epitopes noted above.

***Cytotoxic T-lymphocyte** (CTL) **responses:*** At specific time points following vaccination (*i*.*e*. day 7, 14, 30, and 120), induction of antigen-specific CTL activity is measured following *in vitro* restimulation of lymphoid cells from immune and control animals, using a modification of the protocol described by Bouwer and Hinrichs (see, for example, Bouwer and Hinrichs. Inf. Imm. 1996;64:2515-2522). Briefly, erythrocyte-depleted spleen cells are cultured with Concanavalin A or peptide-pulsed, mitomycin C-treated syngeneic stimulator cells for 72 hours. Effector lymphoblasts are harvested and adjusted to an appropriate concentration for the effector assay. Effector cells are dispensed into round bottom black microtiter plates. Target cells expressing the appropriate antigen (*e*.*g*., cells infected with live *L. monocytogenes* or pulsed with p60 or LLO epitope peptides) are added to the effector cells to yield a final effector:target ratio of at least 40:1. After a four hour incubation, target cell lysis is determined by measuring the release of LDH using the CytoTox ONE fluorescent kit from Promega, following the manufacturer's instructions.

*Antibody **responses:*** Antigen-specific antibody titers are measured by ELISA according to standard protocols (see, *e*.*g*., Cote-Sierra et al. Infect Immun 2002;70:240-248). For example, immunoglobulin isotype titers in the preimmune and immune sera are measured by using ELISA (Southern Biotechnology Associates, Inc., Birmingham, Ala.). Briefly, 96-well Nunc-Immuno plates (Nalge Nunc International, Roskilde, Denmark) are coated with 0.5 µg of COOHgp63 per well, and after exposure to diluted preimmune or immune sera, bound antibodies are detected with horseradish peroxidase-labeled goat anti-mouse IgGl and IgG2a. ELISA titers are specified as the last dilution of the sample whose absorbance was greater than threefold the preimmune serum value. Alternatively, antigen-specific antibodies of different isotypes can be detected by Western blot analysis of sera against lysates of whole *L*. *monocytogenes*, using isotype-specific secondary reagents.

### Results and Discussion

*L. monocytogenes* is a highly virulent and prevalent food-borne gram-positive bacillus that causes gastroenteritis in otherwise healthy patients (Wing et al. J Infect Dis 2002;185 Suppl I:S18-S24), and more severe complications in immunocompromised patients, including meningitis, encephalitis, bacteremia and morbidity (Crum. Curr Gastroenterol Rep 2002;4:287-296 and Frye et al. Clin Infect Dis 2002;35:943-949). *In vivo* models have identified roles for both T- and B-cells in response to *L. monocytogenes,* with protective immunity attributed primarily to CD8 cytotoxic T cells (CTL) (Kersiek and Pamer. Curr Op Immunol 1999;11:400-405). Studies during the past several years have led to the identification of several immunodominant *L. monocytogenes* epitopes recognized by CD4 and CD8 T-cells. In BALB/c mice, several peptides have been identified including the H-2K^{d} restricted epitopes LLO₉₁₋₉₉ and p60₂₁₇₋₂₂₅ (Pamer et al. Nature 1991;353:852-854 and Pamer. J Immunol 1994;152:686). The vaccine potential for such peptides is supported by studies demonstrating that the transfer of LLO₉₁₋₉₉-specific CTL into naïve hosts conveys protection to a lethal challenge with *L. monocytogenes* when the bacterial challenge is administered within a week of CTL transfer (Harty. J Exp Med 1992;175:1531-1538). The mouse model of listeriosis (Geginat et al. J Immunol 1998;160:6046-6055) has provided invaluable insights into the mechanisms of disease and the immunological response to infection with *L. monocytogenes*. This model allows the investigator to study both short-term and memory responses. This mouse model, with modifications, may be employed to confirm the *in vivo* efficacy and mechanism of action of novel polypeptide TLR ligands in fusion protein vaccines.

The polypeptide TLR2 ligands of the invention may be used to generate a fusion protein vaccine for *Listeria* infection. This vaccine comprises a fusion protein of a polypeptide TLR2 ligand and antigenic MHC class I and II epitopes of the *L. monocytogenes* proteins LLO and p60 (LLO-p60 fusion protein, SEQ ID NO: 39). The amino acid sequences of exemplary polypeptide TLR2 ligand:*Listeria* LLO-p60 antigen fusion proteins are set forth in SEQ ID NOs: 51, 52, and 53. For such vaccines, sequences encoding a polypeptide TLR2 ligand:*Listeria* LLO-p60 antigen fusion protein are inserted into a plasmid expression vector. The expression construct is then expressed in *E. coli* and the recombinant fusion protein purified based upon the included His tag.

The purified protein is then used to vaccinate mice. At specific time points following vaccination (*i*.*e*. day 7, 14, 30, and 120), animals are examined for antigen-specific humoral and cellular responses, including serum antibody titers, cytokine expression, CTL frequency and cytotoxicity activity, and antigen-specific proliferative responses. Protection versus *Listeria* infection is confirmed in the vaccinated animals using sublethal and lethal *Listeria* challenge assays. The polypeptide TLR2 ligand:*Listeria* LLO-p60 antigen fusion protein vaccine provides strong antigen-specific humoral and cellular immune responses, and provides protective immunity versus *Listeria* infection.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

It is further to be understood that all values are approximate, and are provided for description.

Patents, patent applications, publications, product descriptions, and protocols are cited throughout this application, the disclosures of which are incorporated herein by reference in their entireties for all purposes.

## Claims

1. A polypeptide TLR2 ligand comprising the amino acid sequence of DPDSG (SEQ ID NO: 5), with the proviso that the polypeptide TLR2 ligand is not a polypeptide selected from:
flagellin modification protein FlmB of *Caulobacter crescentus,*
Bacterial Type III secretion system protein,
invasin protein of *Salmonella,*
Type 4 fimbrial biogenesis protein (PiIX) of *Pseudomonas,*
*Salmonella* SciJ protein,
putative integral membrane protein of *Streptomyces,*
membrane protein of *Pseudomonas,*
adhesin of *Bordetella pertusis,*
peptidase B of *Vibrio cholerae,*
virulence sensor protein of *Bordetella,*
putative integral membrane protein of *Neisseria meningitides,*
fusion of flagellar biosynthesis proteins FliR and FlhB or *Clostridium,*
outer membrane protein (porin) of *Acinetobacter,*
flagellar biosynthesis protein, FlhF of *Helicobacter,*
ompA related protein of *Xanthomonas,*
omp2a porin of *Brucella,*
putative porin/fimbrial assembly protein (LhrE) of *Salmonella,*
wbdk of *Salmonella,*
Glycosyltransferase involved in LPS biosynthesis, and
*Salmonella* putative permease.

2. The polypeptide of claim 1 further comprising at least one antigen.

3. A polypeptide comprising:
i) a polypeptide TLR2 ligand comprising the amino acid sequence of DPDSG (SEQ ID NO: 5); and
ii) at least one antigen, wherein if the at least one antigen is a polypeptide antigen, the polypeptide antigen is heterologous to the polypeptide TLR2 ligand.

4. The polypeptide of claim 2 or 3, wherein the antigen is a polypeptide antigen.

5. The polypeptide of any one of claims 2 to 4, wherein the antigen is a tumor-associated antigen, an allergen-related antigen or a pathogen-related antigen.

6. The polypeptide of claim 5, wherein the pathogen-related antigen is an *Influenza* antigen, a *Listeria monocytogenes* antigen, a West Nile Virus antigen or a Dengue virus antigen.

7. A pharmaceutical composition comprising the polypeptide of any one of claims 1 to 6 and a pharmaceutically acceptable carrier.

8. A vaccine comprising the polypeptide of any of claims 1 to 6 and a pharmaceutically acceptable carrier.

9. The vaccine of claim 8, wherein the polypeptide TLR2 ligand and the antigen are covalently linked.

10. The polypeptide of any one of claims 1 to 6, the pharmaceutical composition of claim 7 or the vaccine of claim 8 or 9 for use in a method of modulating TLR2 signaling in a subject.

11. The polypeptide, pharmaceutical composition or vaccine of claim 10, wherein the subject is a mammal.

12. A method of modulating TLR2 signaling in a cell *in vitro* comprising contacting a cell, wherein the cell comprises TLR2, with the polypeptide of any of claims 1 to 6.

13. A method of modulating TLR2 signaling in a cell *in vitro* comprising contacting a cell, wherein the cell comprises TLR2, with a polypeptide TLR2 ligand comprising the amino acid sequence of DPDSG (SEQ ID NO: 5).

14. The method of claim 12 or 13, wherein the cell is a mammalian cell.
